# EUROPEAN PATENT SPECIFICATION

(11) **EP 1 339 684 B1**
(45) Date of publication and mention of the grant of the patent: **04.11.2009**
(21) Application number: 01988218.2
(22) Date of filing: 29.11.2001
(51) Int. Cl.: C07D 211/26, A61K 31/4465, C07D 211/58, C07D 207/12, C07D 207/14, C07D 295/125, C07D 241/04, C07C 275/28, A61P 3/04

(54) **MCH ANTAGONISTS AND THEIR USE IN THE TREATMENT OF OBESITY**
MCH ANTAGONISTEN UND DEREN VERWENDUNG ZUR BEHANDLUNG VON FETTLEIBIGKEIT
ANTAGONISTES DE L'HORMONE DE CONCENTRATION DE LA MELANINE (MCH) ET LEUR UTILISATION DANS LE TRAITEMENT DE L'OBESITE

(30) Priority: 01.12.2000 US 250502 P
(43) Date of publication of application: 03.09.2003
(73) Proprietor: Schering Corporation, Kenilworth, NJ 07033-0530 (US)
(72) Inventor: MC BRIAR, Mark, D., Clinton, New Jersey 08809 (US); PALANI, Anandan, Bridgewater, NJ 08807 (US); SHAPIRO, Sherry, A., Belford, NJ 07718 (US); XU, Ruo, Watchung, NJ 07060 (US); CLADER, John, Cranford, NJ 07016 (US)
(74) Representative: Adams, Harvey Vaughan John
(86) International application number: PCT/US2001/045242
(87) International publication number: WO 2002/057233

(56) References cited:
- WO-A-99/64394
- US-A- 5 908 830
- US-A- 6 043 246

## Description

### BACKGROUND OF THE INVENTION

This invention relates to antagonists of melanin-concentrating hormone (MCH) and their use in the treatment of eating disorders, metabolic disorders and diabetes.

MCH, a cyclic peptide, was first identified over a decade ago in teleost fish where it appears to regulate color change. More recently, MCH has been the subject of investigation for its possible role as a regulator of eating behavior in mammals. As reported by Shimada et al., Nature, Vol. 396 (17 Dec. 1998), pp. 670-673, MCH-deficient mice have reduced body weight and leanness due to hypophagia (reduced feeding). In view of their findings, the authors have suggested that antagonists of MCH action may be effective for the treatment of obesity. U.S. Patent No. 5,908,830 discloses a combination therapy for the treatment of diabetes or obesity involving the administration of a metabolic rate increasing agent and a feeding behavior modifying agent, an example of the latter being an MCH antagonist. U.S. Patent No. 6,043,246 discloses urea derivatives said to be useful as neuropeptide Y receptor antagonists and as agents for the treatment of, *inter alia*, diseases of the metabolic system including obesity and diabetes. Published PCT patent application WO 00/27845 describes a class of compounds, characterized therein as spiro-indolines, said to be selective neuropeptide Y Y5 antagonists and useful for the treatment of obesity and the complications associated therewith. Commonly assigned, copending U.S. patent application Serial No. 09/950,908 discloses and claims aryl-substituted urea neuropeptide Y Y5 antagonists and their use in the treatment of obesity, hyperphagia (increased feeding) and diabetes.

### SUMMARY OF THE INVENTION

The present invention relates to compounds of the general formula 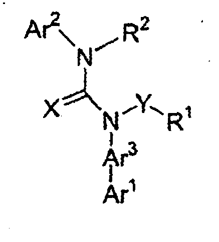 or a pharmaceutically acceptable addition salt and/or hydrate thereof, or where applicable, a geometric or optical isomer or racemic mixture thereof;
wherein
Ar¹ is an aryl or heteroaryl group,
Ar² is an aryl, heteroaryl or aralkyl group;
Ar³ is an arylene or heteroarylene group or Ar¹ and Ar³ together form a fluorene, substituted fluorene or fluorenone group with the proviso that Ar³ must be arylene;
said Ar¹, Ar² and Ar³ groups possessing 0 to 3 substituents independently selected from the group consisting of -(C₁-C₆)alkyl, -(C₃-C₇)cycloalkyl, halo, -CN, -(C₁-C₆)alkoxy, -CF₃, -OCF₃, -CONH₂, -CONH(C₁-C₆)alkyl, -CON(C₁-C₆)alkyl (C₁-C₆)alkyl, - NH₂, -NH C(O)(C₁-C₆)alkyl, -NHSO₂(C₁-C₆)alkyl, -S(C₁-C₆)alkyl, -SO(C₁-C₆)alkyl, - SO₂(C₁-C₆)alkyl, methylenedioxy and NO₂;
X is O, S or N-CN;
Y is a single bond or a -(C₁-C₄)alkylene- group;
R¹ is thiazole, aryl or heteroaryl; or 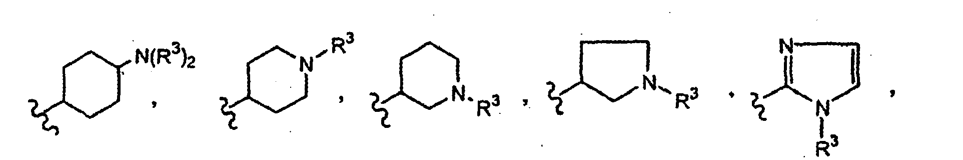 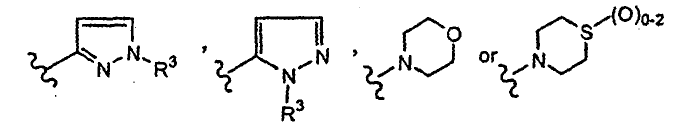 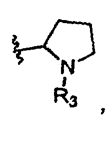 or 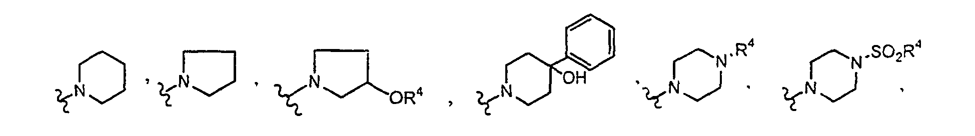 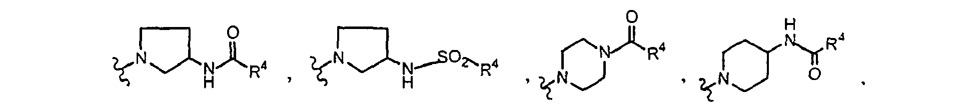 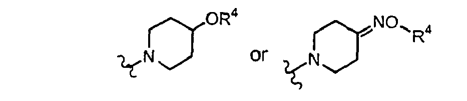 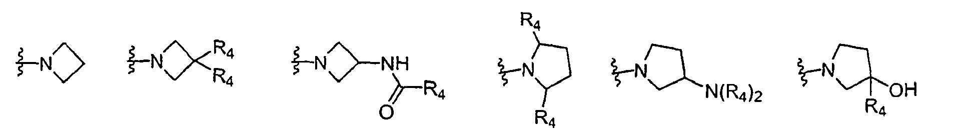 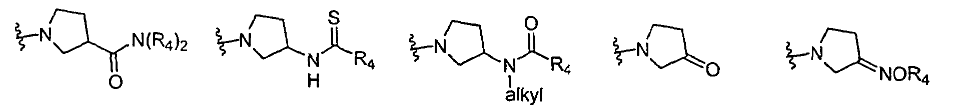 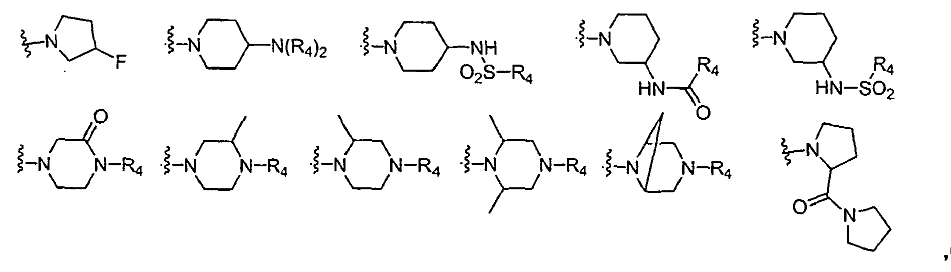 or

R¹ is -N(R⁵)₂, -NHC(O)(C₂-C₃)alkylene N(R⁵)₂; -C(O)NH(C₂-C₃)alkylene N(R⁵)_{2;} C(O)N(Me)(C₂-C₃)alkyleneN(R⁵)_{2,} -C(OH)(C₁-C₂)alkyleneN(R⁵)₂, -N(Me)(C₂-C₃)alkyleneN(R⁵)₂, -NH(C₂-C₃)alkyleneC(O)R⁵, -N(Me)(C₂-C₃)alkyleneN(Me)SO₂(R⁵) or -N(Me)(C₂-C₃)alkyleneC(O)N(R⁵)₂;
R² is H or -(C₁-C₆)alkyl.
R³ is independently H, or nonsubstituted or halosubstituted -(C₁-C₆)alkyl, -(C₃-C₇)cycloalkyl, -(C₃-C₇)cycloalkyl(C₁-C₆)alkyl, -(C₁-C₆)alkoxy, -(C₁-C₆)alkoxy (C₁-C₆)alkylene, aryl, -aralkyl or -heteroaralkyl; or
R⁴ is H, nonsubstituted or halosubstituted -(C₁-C₆)alkyl, -NH(C₁-C₆)alkyl, -NHaryl, aryl; or alkoxy or hydroxy substituted alkyl, and
R⁵ is independently H, or nonsubstituted or halosubstituted -(C₁-C₆)alkyl, -(C₃-C₇)cycloalkyl, -(C₃-C₇)cycloalkyl(C₁-C₆)alkyl, aryl, -aralkyl, -heteroaralkyl, -(C₁-C₆)alkoxy or (C₁-C₆)alkylene(C₁-C₆)alkoxy.

This invention also relates to compositions containing the compounds of the invention as well as methods of using the compounds for the treatment of metabolic disorders, eating disorders or diabetes. The compounds of the invention may be used along or in combination with other appropriate therapeutic agents.

### DETAILED DESCRIPTION OF THE INVENTION

The present invention relates to compounds of the general formula 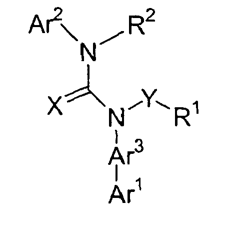 or a pharmaceutically acceptable addition salt and/or hydrate thereof, or where applicable, a geometric or optical isomer or racemic mixture thereof;
wherein
Ar¹ is an aryl or heteroaryl group,
Ar² is an aryl, heteroaryl or aralkyl group or Ar¹ and Ar² together form a fluorene, substituted fluorene or fluorenone group with the proviso that Ar³ must be arylene;
Ar³ is an arylene or heteroarylene group;
said Ar¹, Ar² and Ar³ groups possessing 0 to 3 substituents independently selected from the group consisting of -(C₁-C₆)alkyl, -(C₃-C₇)cycloalkyl, halo, -CN, -(C₁-C₆)alkoxy, -CF₃, -OCF₃, -CONH₂, -CONH(C₁-C₆)alkyl, -CON(C₁-C₆)alkyl (C₁-C₆)alkyl, - NH₂, -NH C(O)(C₁-C₆)alkyl, -NHSO₂(C₁-C₆)alkyl, -S(C₁-C₆)alkyl, -SO(C₁-C₆)alkyl, - SO₂(C₁-C₆)alkyl, methylenedioxy and NO₂;
X is O, S or N-CN;
Y is a single bond or a -(C₁-C₄)alkylene- group;
R¹ is thiazole, aryl or heteroaryl; or 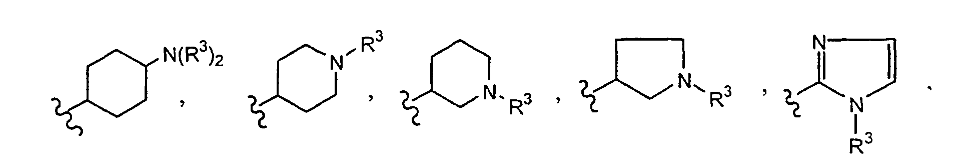 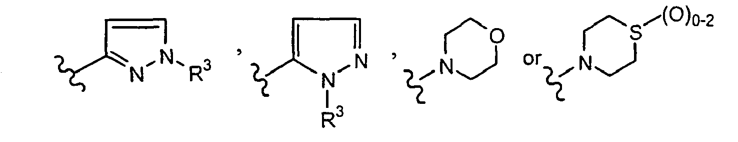 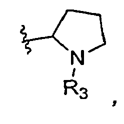 or 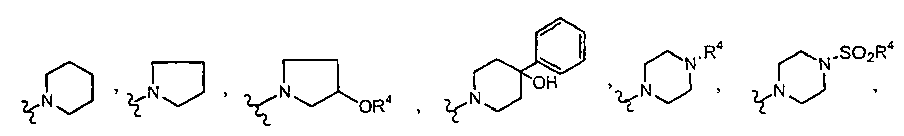 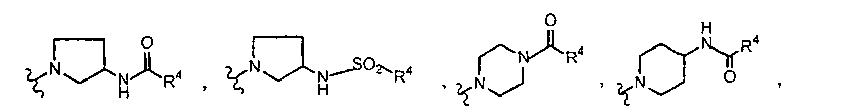 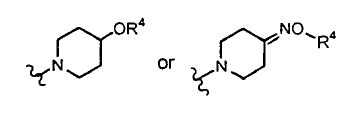 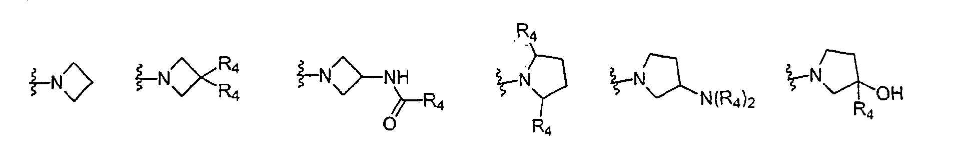 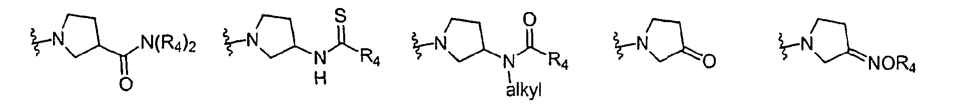 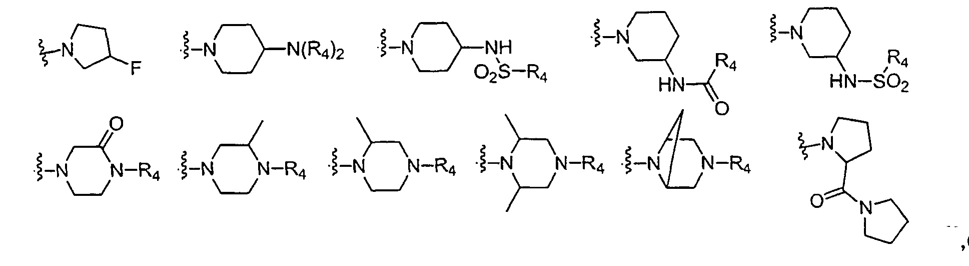 or

R¹ is -N(R⁵)₂, -NHC(O)(C₂-C₃)alkylene N(R⁵)₂; -C(O)NH(C₂-C₃)alkylene N(R⁵)₂; C(O)N(Me)(C₂-C₃)alkyleneN(R⁵)₂, -C(OH)(C₁-C₂)alkyleneN(R⁵)₂, -N(Me)(C₂-C₃)alkyleneN(R⁵)₂, -NH(C₂-C₃)alkyleneC(O)R⁵, -N(Me)(C₂-C₃)alkyleneN(Me)SO₂(R⁵) or -N(Me)(C₂-C₃)alkyleneC(O)N(R⁵)₂;
R² is H or -(C₁-C₆)alkyl.
R³ is independently H, or nonsubstituted or halosubstituted -(C₁-C₆)alkyl, -(C₃-C₇)cycloalkyl, -(C₃-C₇)cycloalkyl(C₁-C₆)alkyl, -(C₁-C₆)alkoxy, -(C₁-C₆)alkoxy (C₁-C₆)alkylene, aryl, -aralkyl or -heteroaralkyl; or
R⁴ is H, nonsubstituted or halosubstituted -(C₁-C₆)alkyl, -NH(C₁-C₆)alkyl, -NHaryl, aryl; or alkoxy or hydroxy substituted alkyl, and
R⁵ is independently H, or nonsubstituted or halosubstituted -(C₁-C₆)alkyl, -(C₃-C₇)cycloalkyl, -(C₃-C₇)cycloalkyl(C₁-C₆)alkyl, aryl, -aralkyl, -heteroaralkyl, -(C₁-C₆)alkoxy or (C₁-C₆)alkylene(C₁-C₆)alkoxy.

This invention also relates to pharmaceutical compositions which comprise an amount of a compound of the invention, a pro-drug thereof, or a pharmaceutically acceptable salt of said compound or of said pro-drug and a pharmaceutically acceptable carrier therefor.

The invention also relates to a method of treating a patient having a disease or condition mediated by MCH by administering a therapeutically effective amount of a compound of the invention, a pro-drug thereof, or a pharmaceutically acceptable salt of said compound or of said pro-drug to the mammal.

Further, this invention relates to a method of treating obesity comprising administering to a mammal in need of such treatment a therapeutically effective amount of a compound of the invention or a pro-drug thereof, or a pharmaceutically acceptable salt of said compound or of said pro-drug.

Another aspect of this invention relates to a method for treating metabolic disorders such as obesity and eating disorders such as bulimia and anorexia comprising administering to a mammal a therapeutically effective amount of a compound of the invention, a pro-drug thereof, or a pharmaceutically acceptable salt of said compound or of said pro-drug.

Another aspect of this invention relates to a method for treating hyperlipidemia comprising administering to a mammal a therapeutically effective amount of a compound of the invention, a pro-drug thereof, or a pharmaceutically acceptable salt of said compound or of said pro-drug.

Another aspect of this invention is directed to a method for treating cellulite and fat accumulation comprising administering to a mammal a therapeutically effective amount of a compound of the invention, a pro-drug thereof, or a pharmaceutically acceptable salt of said compound or of said pro-drug.

Another aspect of this invention is directed to a method for treating type II diabetes comprising administering to a mammal a therapeutically effective amount of a compound of the invention, a pro-drug thereof, or a pharmaceutically acceptable salt of said compound or of said pro-drug.

In addition to the "direct" effect of the compounds of this invention on the MCH subtype, there are diseases and conditions that will benefit from the weight loss such as insulin resistance, impaired glucose tolerance, Type II Diabetes, hypertension, hyperlipidemia, cardiovascular disease, gall stones, certain cancers, and sleep apnea.

This invention is also directed to pharmaceutical compositions for the treatment of obesity which comprise an obesity treating amount of a compound of the invention, a pro-drug thereof, or a pharmaceutically acceptable salt of said compound or of said pro-drug and a pharmaceutically acceptable carrier therefor.

In addition to monotherapies including the compounds of the invention, another aspect of this invention is combinations (such as, for example, dual combination therapy, three combination therapy and the like,) of therapeutically effective amounts of a compound of the invention, or a prodrug thereof, or a pharmaceutically acceptable salt of said compound or a pharmaceutically acceptable salt of said prodrug, and therapeutically effective amounts of one or more antiobesity/anorectic agent such as, for example, a β₃ agonist, a thyromimetic agent, or an NPY antagonist.

Still another aspect of this invention is a method for treating obesity comprising administering to a mammal (which term includes humans) in need of such treatment:
a. therapeutically effective amounts of a first compound, said first compound being a compound of the invention, a prodrug thereof, or a pharmaceutically acceptable salt of said compound or a pharmaceutically acceptable salt of said prodrug; and
b. therapeutically effective amounts of a second compound, said second compound being an antiobesity and/or anorectic agent such as, for example, a β₃ agonist, a thyromimetic agent, or an NPY antagonist, wherein the amounts of the first and second compounds result in the desired therapeutic effect of treating obesity.

This invention is also directed to a pharmaceutical composition comprising a combination of therapeutically effective amounts of a first compound, said first compound being a compound of the invention, a prodrug thereof, or a pharmaceutically acceptable salt of said compound or a pharmaceutically acceptable salt of said prodrug; and therapeutically effective amounts of a second compound, said second compound being an antiobesity and/or anorectic agent such as, for example, a β₃ agonist, a thyromimetic agent, or an NPY antagonist; and/or optionally a pharmaceutical acceptable carrier, vehicle or diluent.

Another aspect of this invention is a kit comprising:
a. therapeutically effective amounts of a first compound, said first compound being a compound of the invention, a prodrug thereof, or a pharmaceutically acceptable salt of said compound or a pharmaceutically acceptable salt of said prodrug; and a pharmaceutically acceptable carrier, vehicle or diluent in a first unit dosage form;
b. therapeutically effective amounts of a second compound, said second compound being an antiobesity and/or anorectic agent such as, for example, a β₃ agonist, a thyromimetic agent, or an NPY antagonist; and a pharmaceutically acceptable carrier, vehicle or diluent in a second unit dosage form; and
c. means for containing said first unit dosage form and said second unit dosage form, wherein the amounts of the first compound and of the second compound result in the desired therapeutic effect of treating obesity.

Illustrative non-limiting examples of preferred antiobesity and/or anorectic agents in the above combination methods, combination compositions and combination kits include: phenylpropanolamine, ephedrine, pseudoephedrine, phentermine, a cholecystokinin-A (hereinafter referred to as CCK-A) agonist, a monoamine reuptake inhibitor (such as, for example, sibutramine), a sympathomimetic agent, a serotonergic agent (such as, for example, dexfenfluramine or fenfluramine), a dopamine agonist (such as, for example, bromocriptine), a melanocyte-stimulating hormone receptor agonist or mimetic, a melanocyte-stimulating hormone analog, a cannabinoid receptor antagonist, a melanin concentrating hormone antagonist, the OB protein (hereinafter referred to as "leptin"), a leptin analog, a leptin receptor agonist, a galanin antagonist or a GI lipase inhibitor or decreaser (such as orlistat). Other anorectic agents include bombesin agonists, dehydroepiandrosterone or analogs thereof, glucocorticoid receptor agonists and antagonists, orexin receptor antagonists, urocortin binding protein antagonists, agonists of the glucagon-like peptide-1 receptor such as, for example, Exendin and ciliary neurotrophic factors such as, for example, Axokine.

Another aspect of this invention is a method for treating diabetes comprising administering to a mammal:
a. therapeutically effective amounts of a first compound, said first compound being a Formula I compound (or a Formula II compound or a Formula III compound), a prodrug thereof, or a pharmaceutically acceptable salt of said compound or a pharmaceutically acceptable salt of said prodrug; and
b. therapeutically effective amounts of a second compound, said second compound being an aldose reductase inhibitor, a glycogen phosphorylase inhibitor, a sorbitol dehydrogenase inhibitor, a protein tyrosine phosphatase 1B inhibitor, a dipeptidyl protease inhibitor, insulin (including orally bioavailable insulin preparations), an insulin mimetic, metformin, acarbose, a PPAR-gamma ligand such as troglitazone, rosaglitazone, pioglitazone or GW-1929, a sulfonylurea, glipazide, glyburide, or chlorpropamide wherein the amounts of the first and second compounds result in the therapeutic effect of treating diabetes.

Unless otherwise stated or indicated, the following definitions apply throughout the specification and claims. These definitions apply regardless of whether a term is used by itself or in combination with other terms. Hence the definition of "alkyl" applies to "alkyl" as well as to the "alkyl" portions of "alkoxy", etc.

"Alkyl" represents a straight or branched saturated hydrocarbon chain having the designated number of carbon atoms. Where the number of carbon atoms is not specified, 1 to 6 carbons are intended.

"Halo" represents fluoro, chloro, bromo or iodo.

"Aryl" refers to a monoaromatic ring or a bicyclic nonfused or fused ring system possessing one or two aromatic rings including, but not limited to, phenyl, naphthyl, tetrahydronaphthyl, indanyl, and the like. The aryl group can be unsubstituted or substituted with one, two, or three substituents independently selected from lower alkyl, halo, cyano, nitro, haloalkyl, hydroxy, alkoxy, carboxy, carboxamide, mercapto, sulfhydryl, amino, alkylamino and dialkylamino.

The term "heteroaryl" and the heteroaryl portion of the term "-heteroaralkyl" independently refer to 5- to 10-membered single, bicyclic or fused ring systems having at least one heteroaryl ring possessing from 1 to 3 heteroatoms independently selected from the group consisting of -O-, -S-, -N-, and -N=. Individual heteroaryl rings can be unsubstituted or substituted with one, two or three substituents independently selected from lower alkyl, halo, cyano, nitro, haloalkyl, hydroxy, alkoxy, carboxy, carboxamide, mercapto, sulfhydryl, amino, alkylamino, dialkylamino.

When a variable appears more than once in the structural formula, for example R³ or R⁵, the identity of each variable appearing more than once may be independently selected from the definition for that variable.

N-oxides can form on a tertiary nitrogen present in an R substituent, or on =N-in a heteroaryl ring substituent and are included in the compounds of the invention.

As used herein, the term "composition" is intended to encompass a product comprising the specified ingredients in the specified amounts, as well as any product which results, directly or indirectly, from combination of the specified ingredients in the specified amounts.

In addition, it is well know to those skilled in the art that many of the foregoing heterocyclic groups can exist in more than one tautomeric form. It is intended that all such tautomers be included within the scope of this invention.

For compounds of the invention having at least one asymmetrical carbon atom, all isomers, including diastereomers, enantiomers and rotational isomers are contemplated as being part of this invention. These individual enantiomers are commonly designated according tot he optical rotation they effect by the symbols (+) and (-), (L) and (D), (I) and (d) or combinations thereof. These isomers may also be designated according to their absolute spatial configuration by (R) and (S), which stands for sinister and rectus, respectively.

The individual isomers can be prepared using conventional resolution procedures, e.g., treatment with an appropriate optically active acid, separating the diastereomers and then recovering the desired isomer. In addition, the individual optical isomers may be prepared by asymmetric synthesis.

Compounds of the invention can exist in unsolvated and solvated forms, including hydrated forms. In general, the solvated forms, with pharmaceutically acceptable solvents such as water, ethanol and the like, are equivalent to the unsolvated forms for purposes of this invention.

The compounds of the presents invention, while effective themselves, may be formulated and administered in the form of their pharmaceutically acceptable addition salts for purposes of stability, convenience of crystallization, increased solubility and other desirable pharmaceutical properties.

A compound of the invention may form pharmaceutically acceptable salts with organic and inorganic acids. The term "pharmaceutically acceptable salt" is intended to include all acceptable salts. Examples of acid salts are hydrochloric, nitric, sulfuric, phosphoric, formic, acetic, trifluoroacetic, propionic, citric, malonic, salicylic, maleic, fumaric, succinic, ascorbic, maleic, methanesulfonic and other mineral and carboxylic acids well known to those skilled in the art. Depending on the particular functionality of the compound, pharmaceutically acceptable salts of the compounds of the invention include those formed from cations such as sodium, potassium, aluminum, calcium, lithium, magnesium, zinc, and from bases such as ammonia, ethylenediamine, N-methyl-glutamine, lysine, arginine, ornithine, choline, N,N'-dibenzylethylendiamine, chlorprocaine, diethanolamine, procaine, N-benzylphenethylamine, diethylamine, piperazine, tris (hydroxymethyl)aminomethane, and tetramethylammonium hydroxide. These salts may be prepared by standard procedures such as reacting a free acid with a suitable organic or inorganic base, or alternatively by reacting a free base with a suitable organic or inorganic acid.

Also, in the case of an acid (-COOH) or alcohol group being present, pharmaceutically acceptable esters can be employed such as methyl, ethyl, butyl, acetate, maleate, pivaloyloxymethyl, and the like, and those esters know in the art for modifying solubility or hydrolysis characteristics for use as sustained release or prodrug formulations.

In a preferred group of compounds of the invention,
Ar¹ and Ar² are independently phenyl or pyridyl,
Ar³ is 1, 4-arylene,
R¹ is 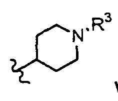 wherein R³ is -(C₁-C₆)alkyl, -(C₃-C₇)cycloalkylmethyl, (C₁-C₆)alkoxy- or (C₁-C₆)alkoxy(C₁-C₆)alkylene-,
R² is H,
X is O; and
Y is a single bond or -(C₁-C₂)alkylene.

In another preferred group of compounds of the invention,
Ar¹ and Ar² are independently phenyl or pyridyl,
Ar³ is 1,4-arylene,
R¹ is -N(R⁵)₂ or -C(O)NH(C₂-C₃)alkylene N(R⁵)₂ in which each R⁵ is independently H, -(C₁-C₆)alkyl, -ar(C₁-C₆)alkyl, heteroaryl, heteroarylalkyl, halo-substituted -(C₁-C₆)alkyl, -(C₃-C₇)cycloalkyl,
X is O; and
Y is -(C₂-C₃)alkylene.

In still another preferred group of compounds of the invention,
Ar¹and Ar² are independently phenyl or pyridyl,
Ar³ is 1,4-arylene,
R¹ is one of the groups 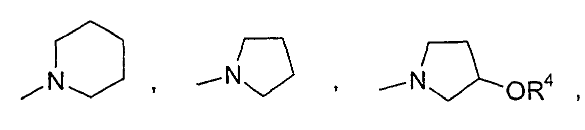 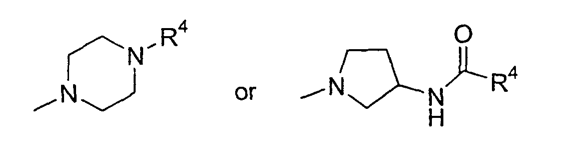
X is O; and
Y is -(C₂-C₃)alkylene.

In the above preferred groups, especially preferred compounds are those in which Ar¹ is 3-substituted phenyl, most preferably those in which the 3-substitution is -CN, -OCF₃ or chloro, or Ar¹ is pyridyl, Ar² is halo-substituted or CF₃-substituted phenyl or pyridyl and any of R³, R⁴ and R⁵, where present, is methyl, ethyl, propyl, - CH₂CH₂CF₃, cyclopentyl, cyclopropylmethyl or 3-methoxyethyl.

Preferred compounds of the invention include those shown in the following table:

| | | |
|---|---|---|
| 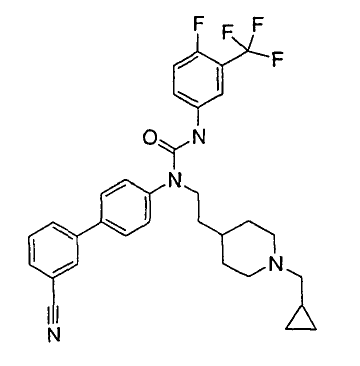 | N-[3'-CYANO[1,1'-BIPHENYL]-4-YL]-N-[2-[1-(CYCLOPROPYLMETHYL)-4-PIPERIDINYL]ETHYL]-N'-[4-FLUORO-3-(TRIFLUOROMETHYL)PHENYL]UREA | |
| 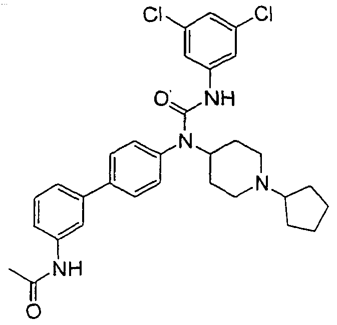 | N-[4'-[(1-CYCLOPENTYL-4-PIPERIDINYL)[[(3,5-DICHLOROPHENYL)AMINO]CARBONY L]AMINO][1,1'-BIPHENYL]-3-YL]ACETAMIDE | |
| 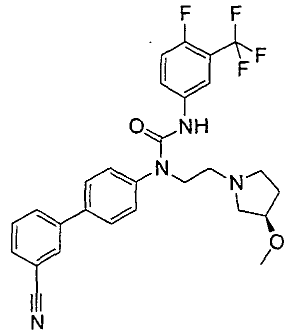 | N-[3'-CYANO[1,1'-BIPHENYL]-4-YL]-N'-[4-FLUORO-3-(TRIFLUOROMETHYL)PHENYL]-N-[2-(3(R)-METHOXY-1-PYRROLIDINYL)ETHYL]UREA | |
| 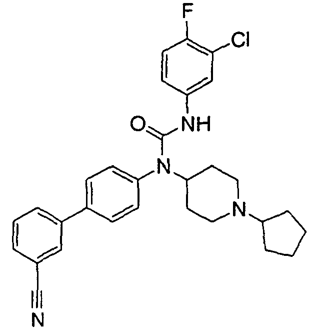 | N'-(3-CHLORO-4-FLUOROPHENYL)-N-[3'-CYANO[1,1'-BIPHENYL]-4-YL]-N-(1-CYCLOPENTYL-4-PIPERIDINYL)UREA | |
| 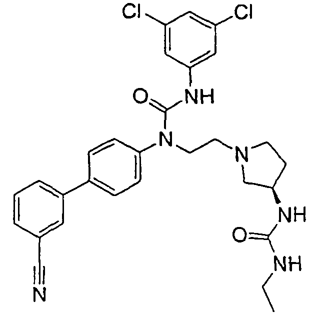 | N-[1-[2-[[3'-CYANO[1,1'-BIPHENYL]-4-YL][[(3,5-DICHLOROPHENYL)AMINO]CARBONY L]AMINO]ETHYL]-3(R)-PYRROLIDINYL]-N'-ETHYLUREA | |
| 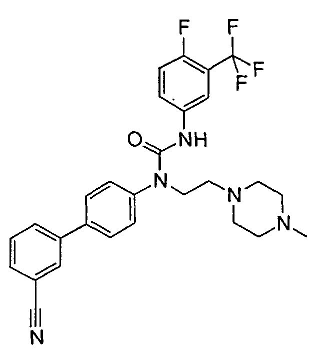 | N-[3'-CYANO[1,1'-BIPHENYL]-4-YL]-N'-[4-FLUORO-3-(TRIFLUOROMETHYL)PHENYL]-N-[2-(4-METHYL-1-PIPERAZINYL)ETHYL]UREA | |
| 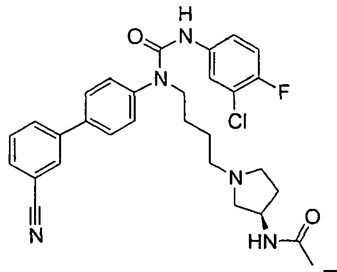 | N-[1-[4-[[[(3-CHLORO-4-FLUOROPHENYL)AMINO]CARBONYL][3'-CYANO[1,1'-BIPHENYL]-4-YL]AMINO]BUTYL]-3(R)-PYRROLIDINYL]ACETAMIDE | |
| 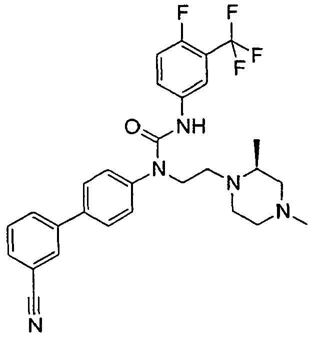 | N-[3'-CYANO[1,1'-BIPHENYL]-4-YL]-N-[2-(2(S),4-DIMETHYL-1-PIPERAZINYL)ETHYL]-N'-[4-FLUORO-3-(TRIFLUOROMETHYL)PHENYL]UREA | |
| 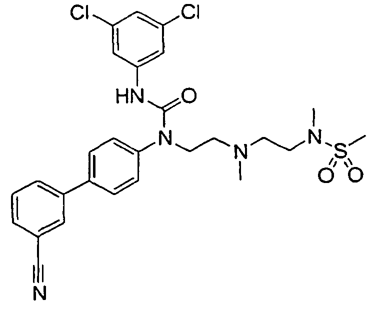 | N-[2-[[2-[[3'-CYANO[1,1'-BIPHENYL]-4-YL][[(3,5-DICHLOROPHENYL)AMINO]CARBONY L]AMINO]ETHYL]METHYLAMINO]ETHY L]-N-METHYLMETHANESULFONAMIDE | |
| 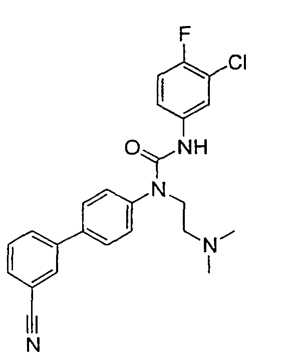 | N'-(3-CHLORO-4-FLUOROPHENYL)-N-[3'-CYANO[1,1'-BIPHENYL]-4-YL]-N-[2-(DIMETHYLAMINO)ETHYL]UREA DIMETHYLAMINO)ETHYL]UREA | |
| 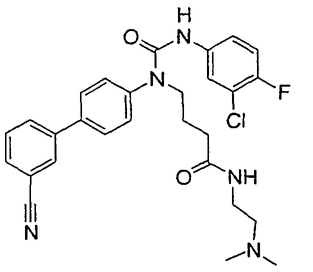 | 4-[[[(3-CHLORO-4-FLUOROPHENYL)AM I NO]CARBONYL][3'-CYANO[1,1'-BIPHENYL]-4-YL]AMINO]-N-[2-(DIMETHYLAMINO)ETHYL]BUTANAMID E | |
| 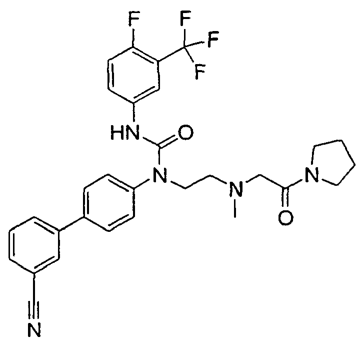 | 1-[[[2-[[3'-CYANO[1,1'-BIPHENYL]-4-YL][[[4-FLUORO-3-(TRIFLUOROMETHYL)PHENYL]AMINO] CARBONYL]AMINO]ETHYL]METHYLAM INO]ACETYL]PYRROLIDINE | |
| 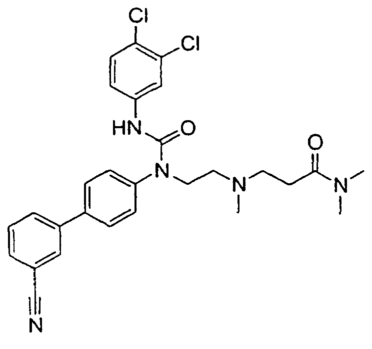 | 3-[[2-[[3'-CYANO[1,1'-BIPHENYL]-4-YL][[(3,46DICHLOROPHENYL)AMINO]CARBONY L]AMINO]ETHYL]METHYLAMINO]-N,N-DIMETHYLPROPANAMIDE | |
| 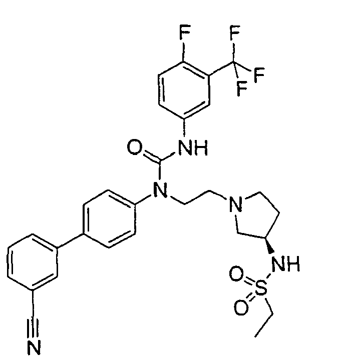 | N-[1-[2-[[3'-CYANO[1,1'-BIPHENYL]-4-YL][[[4-FLUORO-3-(TRIFLUOROMETHYL)PHENYL]AMINO] CARBONYL]AMINO]ETHYL]-3(R)-PIPERIDINYL]ETHANESULFONAMIDE | |
| 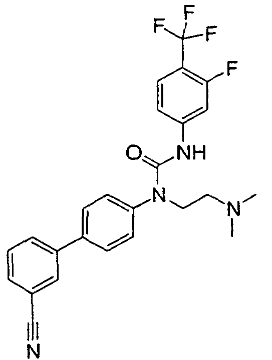 | N-[3'-CYANO[1,1'-BIPHENYL]-4-YL]-N'-[3-FLUORO-4-(TRIFLUOROMETHYL)PHENYL]-N-[2-(DIMETHYLAMINO)ETHYL]UREA | |
| 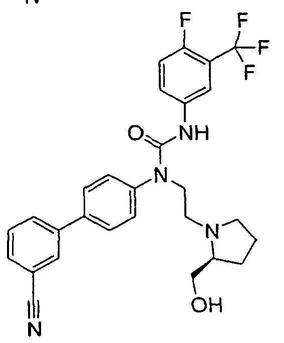 | N-[3'-CYANO[1,1'-BIPHENYL]-4-YL]-N'-[4-FLUORO-3-(TRIFLUOROMETHYL)PHENYL]-N-[2-[2(S)-(HYDROXYMETHYL)-1-PYRROLIDINYL]ETHYL]UREA | |
| 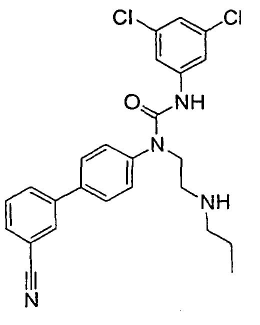 | N-[3'-CYANO[1,1'-BIPHENYL]-4-YL]-N'-(3,5-DICHLOROPHENYL)-N-[2-(PROPYLAMINO)ETHYL]UREA | |
| 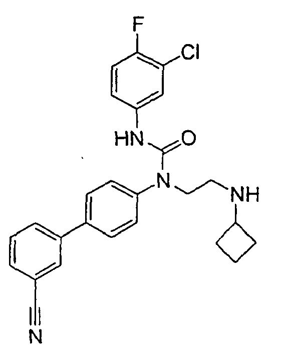 | N'-(3-CHLORO-4-FLUOROPHENYL)-N-[3'-CYANO[1,1'-BIPHENYL]-4-YL]-N-[2-(CYCLOBUTYLAMINO)ETHYL]UREA | |
| 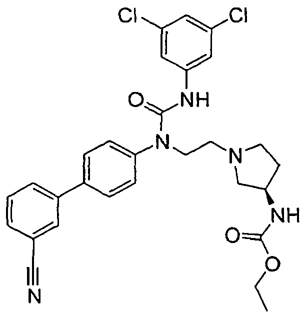 | ETHYL[1-[2-[[3'-CYANO[1,1'-BIPHENYL]-4-YL][[(3,56DICHLOROPHENYL)AMINO]CARBONY L]AMINO]ETHYL]-3(R)-PYRROLIDINYL]CARBAMATE | |
| 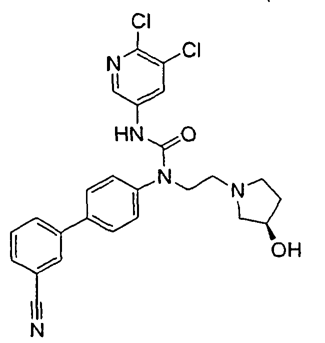 | N-[3'-CYANO[1,1'-BIPHENYL]-4-YL]-N'-(5,6-DICHLORO-3-PYRIDINYL)-N-[2-(3(R)-HYDROXY-1-PYRROLIDINYL)ETHYL]UREA | |
| 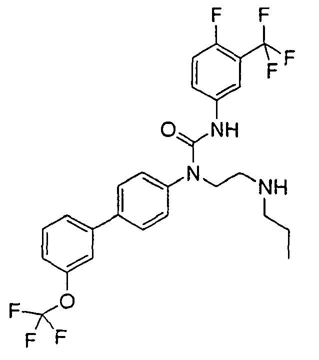 | N'-[4-FLUORO-3-(TRIFLUOROMETHYL)PHENYL]-N-[2-(PROPYLAMINO)ETHYL]-N-[3'-(TRIFLUOROMETHOXY)[1,1'-BIPHENYL]-4-YL]UREA | |
| 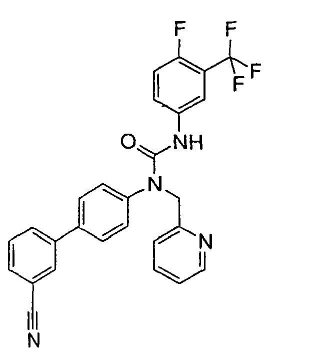 | N-[3'-CYANO[1,1'-BIPHENYL]-4-YL]-N'-[4-FLUORO-3-(TRIFLUOROMETHYL)PHENYL]-N-(2-PYRIDINYLMETHYL)UREA | |
| 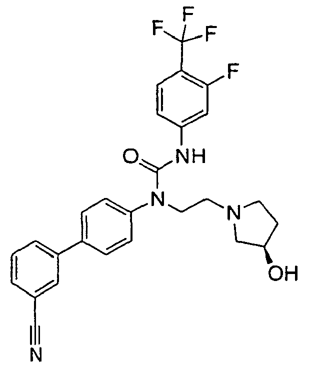 | N-[3'-CYANO[1,1'-BIPHENYL]-4-YL]-N'-[3-FLUORO-4-(TRIFLUOROMETHYL)PHENYL]-N-[2-(3(R)-HYDROXY-1-PYRROLIDINYL)ETHYL]UREA | |
| 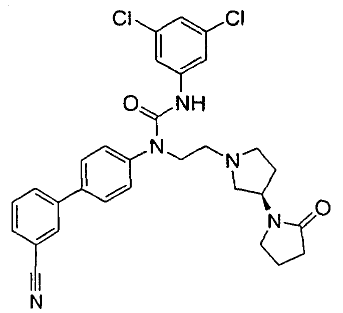 | N-[3'-CYANO[1,1'-BIPHENYL]-4-YL]-N'-(3,5-DICHLOROPHENYL)-N-[2-[2-OXO[1,3'(R)-BIPYRROLIDIN]-1'-YL]ETHYL]UREA | |
| 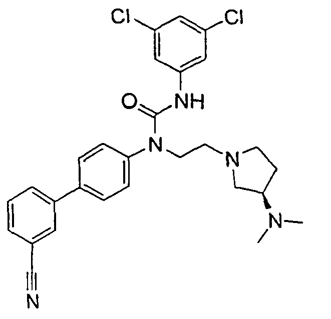 | N-[3'-CYANO[1,1'-BIPHENYL]-4-YL]-N'-(3,5-DICHLOROPHENYL)-N-[2-[3(R)-(DIMETHYLAMINO)-1-PYRROLIDINYL]ETHYL]UREA | |
| 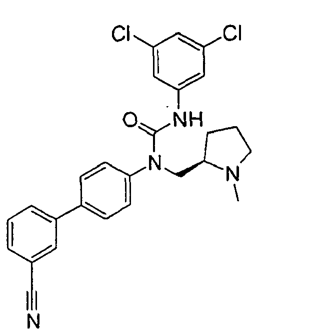 | N-[3'-CYANO[1,1'-BIPHENYL]-4-YL]-N'-(3,5-DICHLOROPHENYL)-N-[(1-METHYL-2(R)-PYRROLIDINYL)METHYL]UREA | |
| 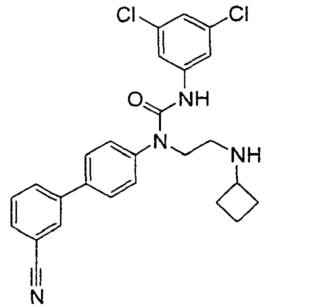 | N-[3'-CYANO[1,1'-BIPHENYL]-4-YL]-N-[2-(CYCLOBUTYLAMINO)ETHYL]-N'-(3,5-DICHLOROPHENYL)UREA | |
| 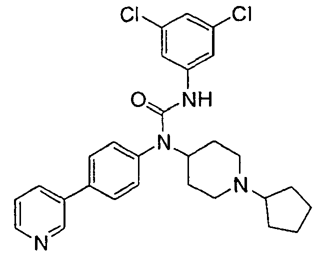 | N-(1-CYCLOPENTYL-4-PIPERIDINYL)-N'-(3,5-DICHLOROPHENYL)-N-[4-(3-PYRIDINYL)PHENYL]UREA | |
| 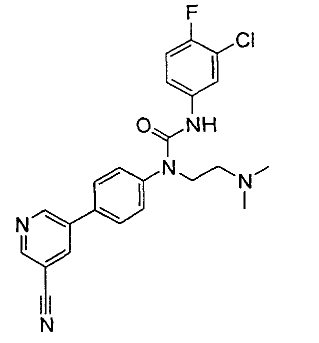 | N'-(3-CHLORO-4-FLUOROPHENYL)-N-[4-(5-CYANO-3-PYRIDINYL)PHENYL]-N-[2-(DIMETHYLAMINO)ETHYL]UREA | |
| 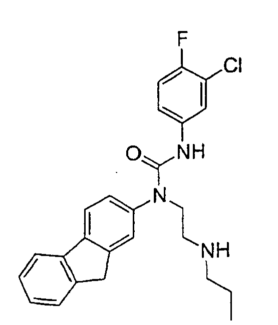 | N'-(3-CHLORO-4-FLUOROPHENYL)-N-(9H-FLUOREN-2-YL)-N-[2-(PROPYLAMINO)ETHYL]UREA | |
| 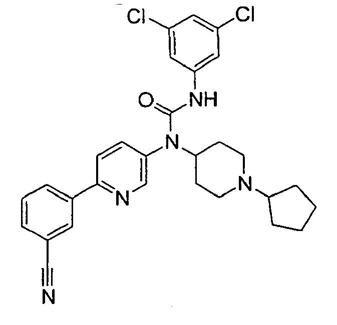 | N-[6-(3-CYANOPHENYL)-3-PYRIDINYL]-N-(1-CYCLOPENTYL-4-PIPERIDINYL)-N'-(3,5-DICHLOROPHENYL)UREA | |

Compounds of the invention can be produced by processes known to those skilled in the art and as shown in the following reaction schemes and in the preparations and examples below.

### Preparations:

Biaryl ureas of type 1 a are prepared via Method 1 as shown in Scheme 1a and Scheme 1 b.

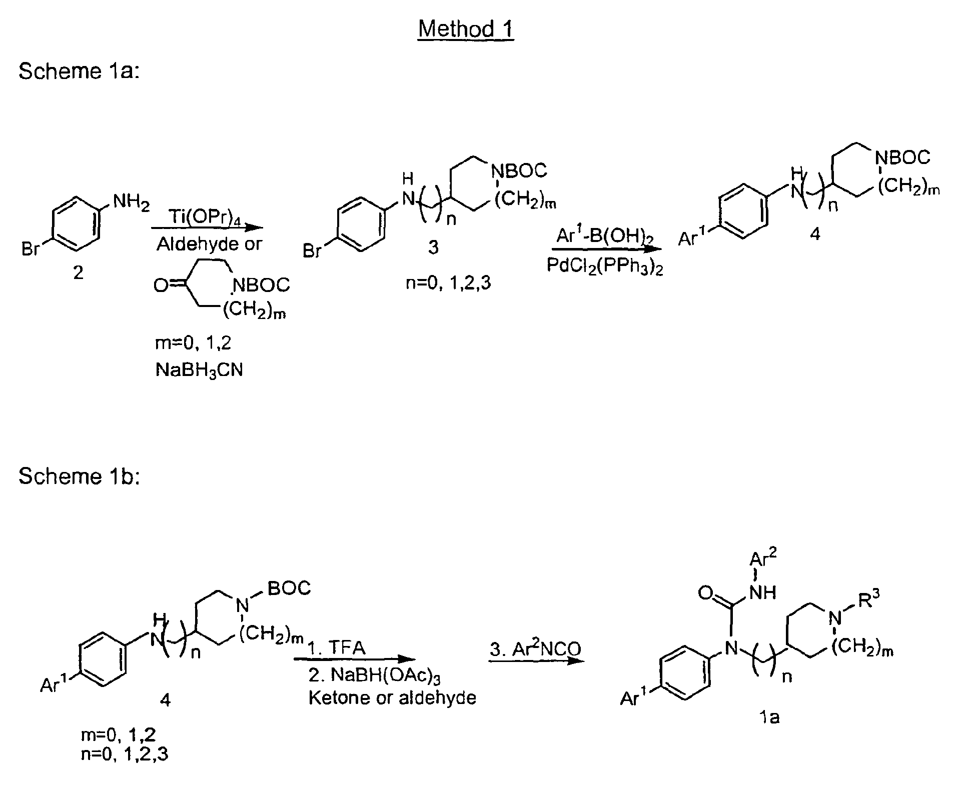

Reductive alkylation of bromoaniline 2 with an aldehyde or ketone corresponding to group R¹ (as exemplified by n-BOC piperidone, m=1) affords amine 3 (Scheme 1 a). Suzuki coupling of amine 3 with an aryl boronic acid gives biaryl amine 4. The BOC deprotection of biaryl amine of 4 is carried out using trifluoroacetic acid (Scheme 1 b) and the resulting amine is reacted with a ketone or aldehyde under reductive alkylation conditions or with alkyl bromide to introduce group R³. Treatment of the resulting amine with isocyanate Ar²NCO affords biaryl urea 1a.

Alternatively, the sequence of steps in scheme 1 a can be reversed so that the reductive alkylation step is carried out after Suzuki coupling. The sequence of steps in scheme 1 b can be reversed so that the urea formation is carried out before the deprotection with TFA.

### Preparation of biaryl open chain ureas:

Biaryl ureas of type 1 b are prepared via Methods 2 to 6.

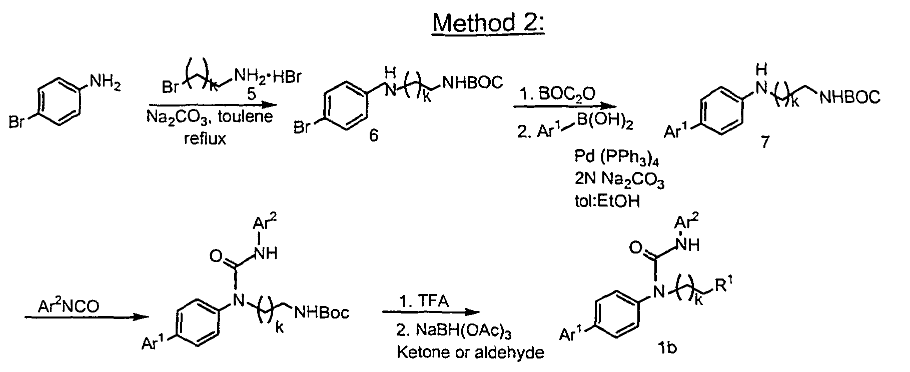

Alkylation of bromide 5 in which k is 1 or 2 with 4-bromoaniline in the presence of a base such as Na₂CO₃ affords the diamine 6. Selective protection of the primary amine group with BOC₂O followed by Suzuki coupling with an aryl boronic acid provides biarylamine 7. Treatment of biarylamine 7 with isocyanate Ar²NCO provides the urea functionality. Deprotection of BOC with TFA affords compounds 1 b where R¹ is -NH₂. Reductive alkylation under standard conditions with R¹H gives with R¹H biaryl urea 1b wherein R¹ can be -NHR⁵ or -N(R⁵)₂ in which R⁵ is -(C₁-C₆)alkyl, -(C₃-C₇)cycloalkyl or -(C₃-C₇)cycloalkyl(C₁-C₆)alkyl.

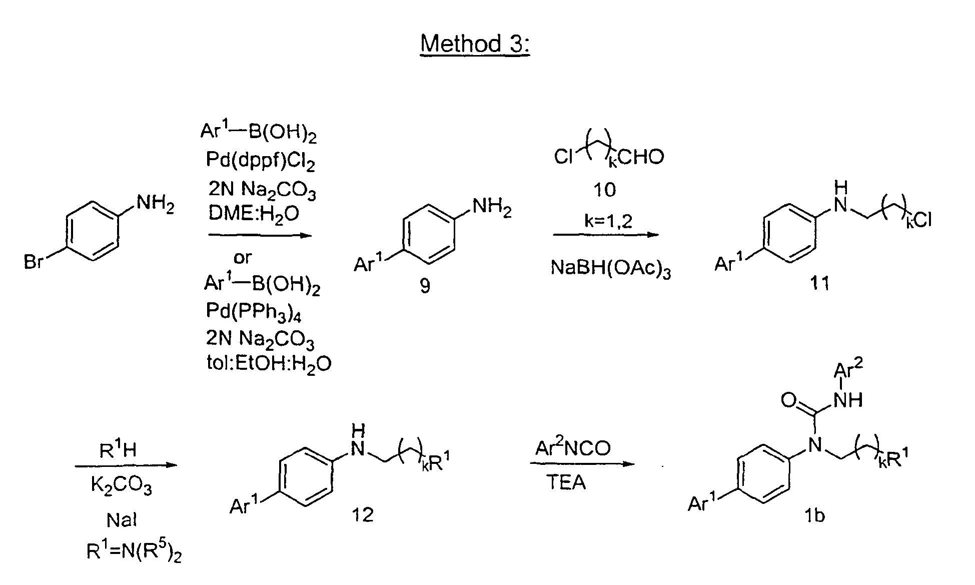

Suzuki coupling between 4-bromoaniline and an aryl boronic acid affords the biaryl amine 9. Reductive alkylation of 9 with chloroaldehyde 10 under standard conditions gives chloride 11 in which k is 1 or 2. Nucleophilic displacement of chloride 11 with the appropriate amine followed by treatment with isocyanate Ar²NCO affords the desired urea 1 b in which R¹ is -N(R⁵)₂, where R⁵ is as previously defined or where N(R⁵)₂ forms a cyclic amine as previously defined for R¹.

Ureas 1b wherein R¹ contains a BOC protected amine group can be further modified by TFA deprotection of BOC followed by alkylation, reductive alkylation, acylation, sulfonylation, carbamate formation, urea formation, thiourea formation or sulfamide formation to form other R¹ groups as defined previously.

Ureas 1 b wherein R¹ contains a ketal group can be further modified by HCl hydrolysis to the ketone followed by addition of Grignard reagent, oxime formation or reduction to alcohol to form other R¹ groups as defined previously.

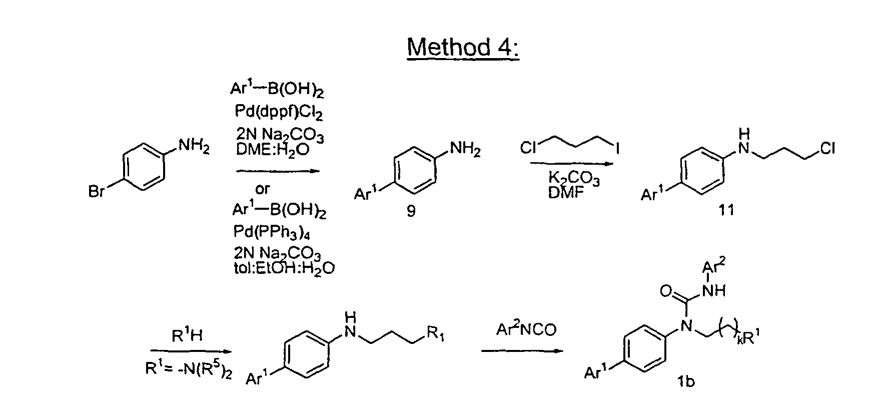

Suzuki coupling between 4-bromoaniline and an aryl boronic acid affords biaryl amine 9. Nucleophilic displacement of 3-chloro-iodopropane affords the chloride 11. Nucleophilic displacement of chloride 11 with the appropriate amine followed by treatment with isocyanate Ar²NCO affords the desired urea 1 b in which k = 2 and R¹ is - N(R⁵)₂ as defined in Method 3.

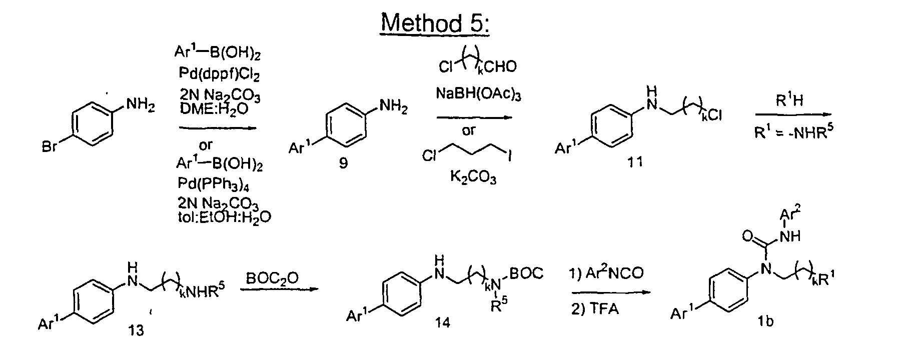

Suzuki coupling between 4-bromoaniline and an aryl boronic acid affords biaryl amine 9. Reductive alkylation or nucleophilic displacement of iodine gives chloride 11. Nucleophilic displacement of chloride 11 with the appropriate amine affords the secondary amine 13. Selective protection of the right-hand side alkyl amine group with BOC₂O gives intermediate 14. Treatment with isocyanate Ar²NCO followed by deprotection of BOC with trifluoroacetic acid affords 1 b in which k is 1 or 2 and R¹ is - NHR⁵ as defined in Method 2.

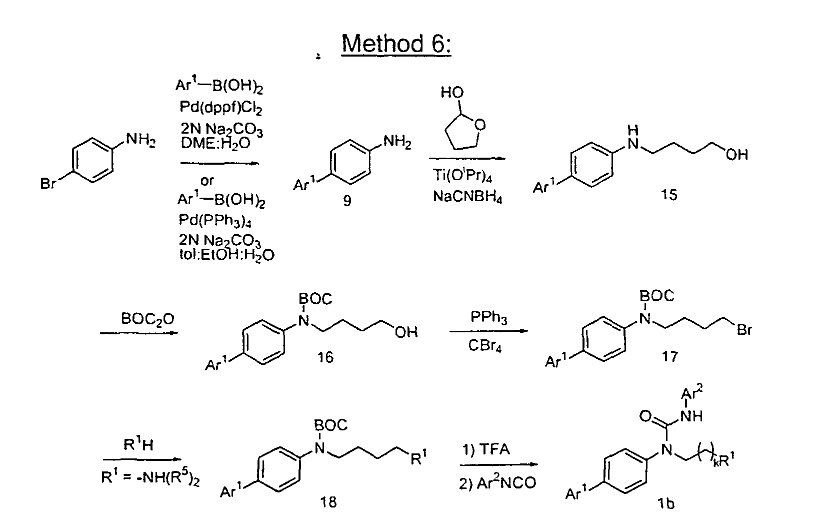

Suzuki coupling between 4-bromoaniline and an aryl boronic acid affords biaryl amine 9. Reductive alkylation with 2-hydroxy-tetrahydrofuran under standard conditions gives alcohol 15. Protection of the amine group with BOC₂O gives intermediate 16, followed by bromination gives bromide 17. Nucleophilic displacement of bromide 17 with the appropriate amine affords amine 18. The deprotection of BOC is carried out with trifluoroacetic acid followed by treatment with isocyanate Ar²NCO affords 1 b in which k is 3 and R¹ is -N(R⁵)₂ as defined in Method 3.

Biaryl ureas of type 1 c are prepared via Method 7.

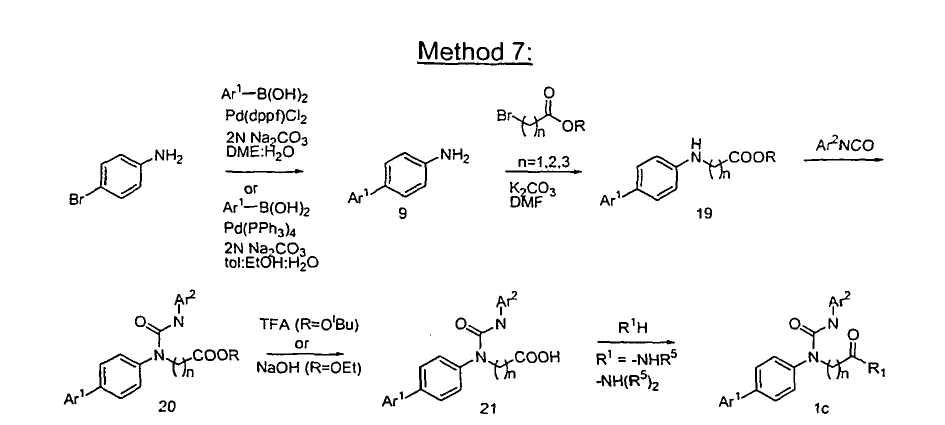

Suzuki coupling between 4-bromoaniline and an aryl boronic acid affords biaryl amine 9. Nucleophilic displacement of bromine gives ester 19 in which n is 1, 2 or 3. Treatment with isocyanate Ar²NCO affords urea 20. Hydrolysis of the ester with either trifluoroacetic acid or NaOH affords carboxylic acid 21. Coupling of the acid with the appropriate amine affords 1 c in which n is 1, 2 or 3 and R¹ is -NHR⁵ as defined in Method 2 or - N(R⁵)₂ as defined in Method 3.

Biaryl ureas of type 1 d are prepared via Method 8.

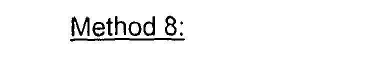

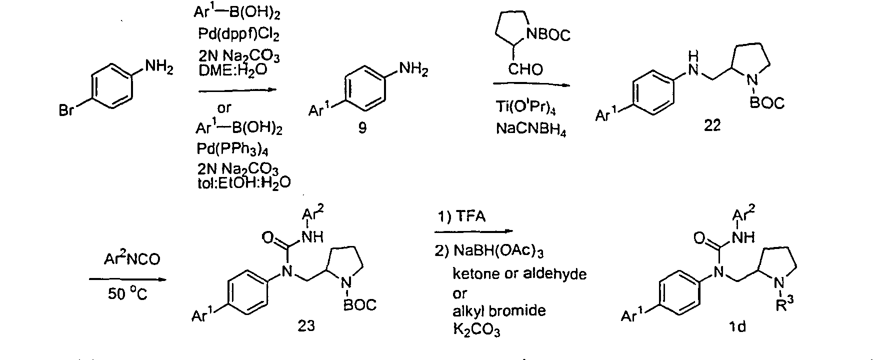

Suzuki coupling between 4-bromoaniline and an aryl boronic acid affords biaryl amine 9. Reductive alkylation of 9 with prolinal under standard conditions followed by treatment with isocyanate Ar²NCO affords urea 23. The BOC deprotection is carried out with trifluoroacetic acid and the resulting amine is reacted with ketone or aldehyde under reductive alkylation conditions or with alkyl bromide to introduce group R³, in which R³ is as previously defined.

The compounds of the present invention exhibit MCH receptor antagonizing activity, which has been correlated with pharmaceutical activity for treating eating disorders, metabolic disorders and for the treatment of diabetes.

The compounds of the invention display pharmacological activity in the following assay designed to demonstrate MCH receptor antagonist activity. The compounds are non-toxic at pharmaceutically therapeutic doses.

### MCH receptor binding assay

Membranes from CHO cells expressing the MCH receptor were prepared by lysing cells with 5 mM HEPES for 15 min at 4°C. Cell lysates were centrifuged (12.5000 x g, 15 min) and the pellet was resuspended in 5 mM HEPES. For each 96-well plate (Microlite, Dynex Technologies), 1 mg of cell membranes were incubated with 10 mg of wheat germ agglutinin SPA beads (Amersham) for 5 min at 4°C in a volume of 10 ml of binding buffer (25 mM HEPES, 10 mM MgCl₂, 10 mM NaCl, 5 mM MnCl₂, 0.1% BSA). The membrane/bead mixture was centrifuged (1500 x g, 3.5 min), the supernatant was aspirated, and the pellet was resuspended in 10 ml binding buffer. The centrifugation, aspiration and resuspension were then repeated. The membrane/bead mixture (100 l) was then added to 96-well plates containing 50 µl of 500 pM [¹²⁵I]-MCH (NEN) and 50 ml of the appropriate concentration of compound (4X the desired final concentration). Nonspecific binding was determined by including 1 µM MCH in the binding reaction. The binding reaction was incubated at room temperature for 2 h. Plates were then analyzed in a TOPCOUNT microplate scintillation counter (Packard). Data was analyzed and Ki values were determined using GraphPad Prim.

For the compounds of this invention, a range of MCH receptor binding activity (Ki values) of from about 0.5 nM to about 100 nM was observed. Compounds of this invention preferably have a binding activity in the range of from about 0.5 nM to about 50 nM, and more preferably from about 0.5 to about 10 nM.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, com starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the technique described in the U.S. Pat. Nos. 4,256,108; 4,166,452; and 4,265,874 to form osmotic therapeutic tablets for controlled release.

Formulations for oral use may also be presented as hard gelatin capsules wherein the active ingredients is mixed with an inert solid diluent, for example, calcium carbonate, calcium phosphate or kaolin, or a soft gelatin capsules where in the active ingredient is mixed with water or an oil medium, for example peanut oil, liquid paraffin or olive oil.

Aqueous suspensions contain the active material in admixture with excipients suitable for the manufacture of aqueous suspensions. Such excipients are suspending agents, for example, sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example, heptadecaethylene-oxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example, polyethylene sorbitan monooleate. The aqueous suspensions may also contain one or more preservatives, for example, ethyl or n-propyl, p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example, arachis oil, olive oil, sesame oil or coconut oil, or in mineral oil such as liquid paraffin. The oily suspensions may contain a thickening agent, for example, beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, e.g., sweetening, flavoring and coloring agents, may also be present.

The pharmaceutical compositions of the invention may also be in the form of an oil-in-water emulsions. The oily phase may be a vegetable oil, e.g., olive oil or arachis oil, or a mineral oil, e.g., liquid paraffin or mixtures of these. Suitable emulsifying agents may be naturally-occurring phosphatides, e.g., soy beans, lecithin, and esters or partial esters derived from fatty acids and hexitol anhydrides, for example, sorbitan monooleate, and condensation products of the said partial esters with ethylene oxide, e.g.,polyoxyethylene sorbitan monooleate. The emulsions may also contain sweetening and flavouring agents.

Syrups and elixirs may be formulated with sweetening agents, for example, glycerol, propylene glycol, sorbitol or sucrose. Such formulations may also contain a demulcent, a preservative and flavoring and coloring agents.

The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, e.g.,as a solution in 1,3-butane diol. Among the acceptable vehicles and solvents that may be employed are water, Ringer's solution and isotonic sodium chloride solution. In addition, sterile fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of the invention may also be administered in the form of suppositories for rectal administration of the drug. The compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compound of the invention are employed. (For purposes of this application, topical application shall include mouthwashes and gargles.)

The compounds for the present invention can be administered in the intranasal form via topical use of suitable intranasal vehicles, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethyleme glycols of various molecular weights and fatty acid esters of polyethylene glycol.

The dosage regimen utilizing the compounds of the present invention is selected in accordance with a variety of factors including type, species, weight, sex and medical condition of the patient; the severity of the condition to be treated; the route of administration; the renal and hepatic function of the patient; and the particular compound thereof employed. A physician or veterinarian of ordinary skill can readily determine and prescribe the effective amount of the drug required to prevent, counter, arrest or reverse the progress of the condition. Optimal precision in achieving concentration of drug within the range that yields efficacy without toxicity requires a regimen based on the kinetics of the drug's availability to target sites. This involves a consideration of the distribution, equilibrium, and elimination of a drug. Preferably, doses of the compound of structural The invention useful in the method of the present invention range from 0.01 to 1000 mg per adult human per day. Most preferably, dosages range from 0.1 to 500 mg/day. For oral administration, the compositions are preferably provided in the form of tablets containing 0.01 to 1000 milligrams of the active ingredient, particularly 0.01, 0.05, 0.1, 0.5, 1.0, 2.5, 5.0, 10.0, 15.0, 25.0, 50.0, 100 and 500 milligrams of the active ingredient for the symptomatic adjustment of the dosage to the patient to be treated. An effective amount of the drug is ordinarily supplied at a dosage level of from about 0.0002 mg/kg to about 50 mg/kg of body weight per day. The range is more particularly from about 0.001 mg/kg to 1 mg/kg of body weight per day.

Advantageously, the active agent of the present invention may be administered in a single daily dose, or the total daily dosage may be administered in dividend doses of two, three or four time daily.

The amount of active ingredient that may be combined with the carrier materials to produce single dosage form will vary depending upon the host treated and the particular mode of administration.

It will be understood, however, that the specific dose level for any particular patient will depend upon a variety of factors including the age, body weight, general health, sex, diet, time of administration, route or administration, rate of excretion, drug combination and the severity of the particular disease undergoing therapy.

The following examples illustrate the preparation of some of the compounds of the invention and are not to be construed as limiting the invention disclosed herein.

### EXAMPLE 1

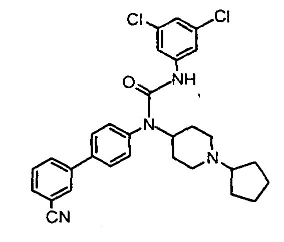

### N-{3'-cyano[1,1'-biphenyl]-4-yl]-N-(-cyclopentyl-4-piperidinyl) -N'-(,5-dichlorophenyl)urea

To a stirred solution of 4-bromoaniline (10.0 g, 58.13 mmol) and N-Boc-piperidone (11.6 g, 58.13 mmol) was added titanium isopropoxide (20.78 mL, 69.72 mmol) and stirred at rt for 24 h. The reaction mixture was then cooled to 0°C and sodium cyanoborohydride (10.95 g, 174.3 mmol) was added in methanol (30 mL) drop-wise. The reaction was then stirred at 0°C for 20 mins and at rt for 5 h. The reaction mixture was then diluted with EtOAc:water (3:1), filtered through Celite. The resulting filtrate was then washed over brine, dried over Na₂SO₄, concentrated and chromatographed over silica gel (eluting EtOAc/hexanes) to yield 20.9 g (49%) of amine as a solid.

To a stirred solution of amine (2.55 g, 7.18 mmol) in 30 mL of DME:H₂O (4:1) was added 3-cyanophenylboronic acid (2.11 g, 14.4 mmol), PdCl₂(PPh₃)₂ (0.500g, 0.718 mmol), Na₂CO₃ (2.28 g, 21.55 mmol) and heated to 80°C for 24 h. The reaction mixture was then cooled to rt, poured into aqueous NaOH and extracted with ether. The combined extracts were then dried, concentrated and chromatographed to yield 1.46 g (54%) of biaryl amine as a solid.

To a stirred solution of biaryl amine (0.546 g, 1.45 mmol) in methylene chloride (3 mL) was added trifluoroacetic acid (2.2 mL) and stirred at rt for 2 h. The reaction mixture was then concentrated and poured into 10% NaOH and extracted with methylene chloride. The combined extracts were dried and concentrated to afford 0.357 g (89%) of amine.

To a stirred solution of amine (0.357 g, 1.28 mmol) and cyclopentanone (0.15 mL, 1.67 mmol) in methylene chloride (5 mL) was added sodium triacetoxyborohydride (0.546 g, 2.57 mmol), acetic acid (0.15 mL, 2.57 mmol) and stirred at rt for 24 h. The reaction mixture was quenched with aqueous NaOH and extracted with methylene chloride. The combined extracts were dried, concentrated and chromatographed to yield 0.376 g (85%) of amine as a solid.

To a stirred solution of amine (0.300 g, 0.869 mmol) and triethyl amine (0.600 mL, 4.34 mmol) in methylene chloride was added 3,5-dichlorophenyl isocyanate (0.817 g, 4.34 mmol) and stirred at rt for 24 h. The reaction mixture was then diluted with methylene chloride, washed with aqueous NaOH, dried, concentrated and chromatographed to yield 0.24 g (52%) of urea as a solid.

300 MHz- ¹H NMR (CDCl₃) δ 7.86 (s, 1 H), 7.79-7.82 (m, 1 H), 7.59-7.69 (m, 4H), 7.31 (d, J = 8.4 Hz, 2H), 7.23 (m, 2 H), 7.20 (s, 1 H), 5.99 (s, 1 H), 4.52-4.60 (m, 1 H), 3.07 (m, 2H), 2.46 (m, 1 H), 2.09 (m, 2H), 1.89 (m, 4 H), 1.27-1.63 (m, 9 H). HRMS (M+H⁺) 533.1867

### EXAMPLES 2-43

Employing preparative procedures similar to those described in Example 1, the following compounds shown below in Table 1 were prepared.

**TABLE I**

| Ex. | Structure/Name | HRMS | 300MHZ-¹H NMR CDCl₃, δ |
|---|---|---|---|
| 2 | 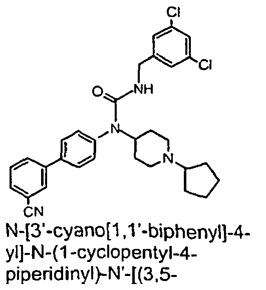 | 547.2036 | |
| 3 | 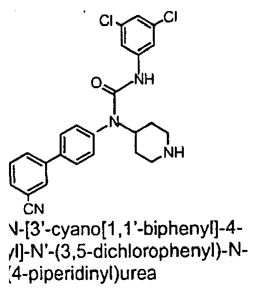 | 465.1255 | |
| 4 | 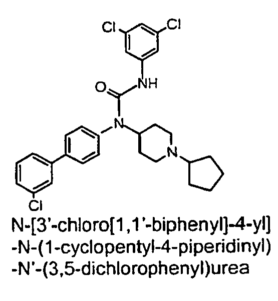 | 542.1529 | |
| 5 | 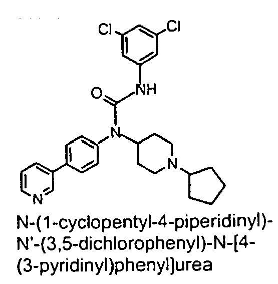 | 509.1869 | |
| 6 | 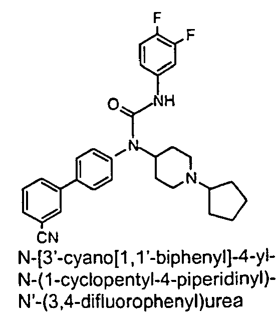 | 501.2473 | |
| 7 | 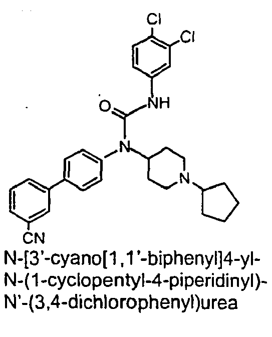 | 533.1867 | 7.80-7.87 (m, 3H), 7.58-7.71 (m, 4H), 7.49 (d, J=3 Hz, 1H), 7.46 (d, J=9.0 Hz, 2H), 7.26 (s, 1H), 7.06-7.10 (m, 1 H), 5.92 (s, 1H), 4.59 (m, 1H), 3.08(m, 2H), 2.46 (m, 1 H), 2.09 (m, 2 H), 1.89 (m, 4 H), 1.27-1.63 (m, 8 H). |
| 8 | 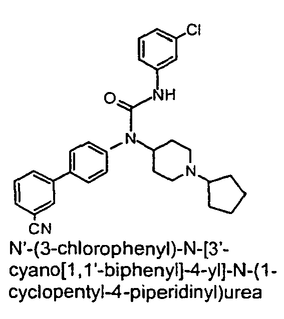 | 499.2269 | |
| 9 | 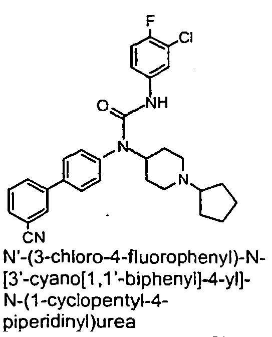 | 517.2165 | |
| 10 | 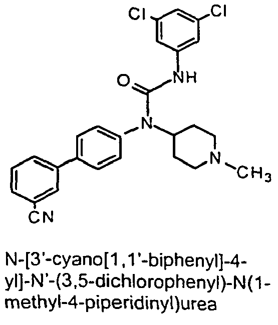 | 479.1400 | |
| 11 | 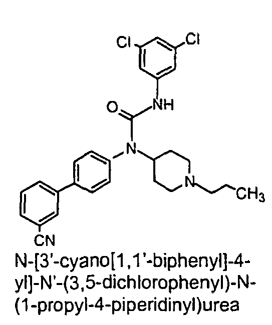 | 507.1709 | |
| 12 | 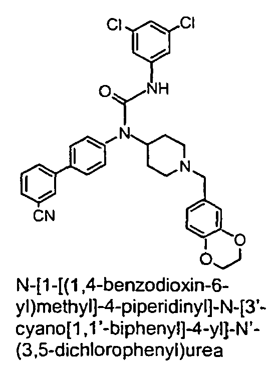 | 613.1779 | |
| 13 | 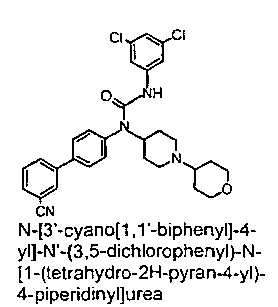 | 549.1832 | |
| 14 | 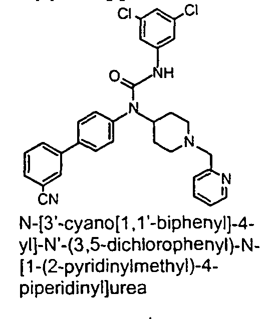 | 556.1672 | |
| 15 | 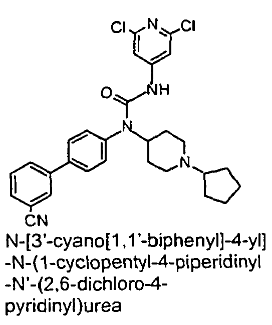 | 534.1823 | |
| 16 | 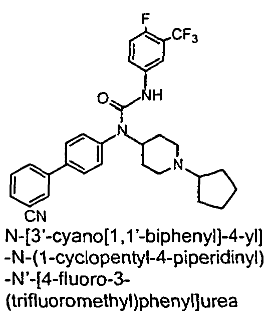 | 551.2443 | 7.79-7.86(m, 2H), 7.52-7.70 (m, 5H), 7.40(m, 1H), 7.35 (d, J=9.0 Hz, 2H), 7.04 (t, J=9.0 Hz, 1 H), 5.97 (s, 1H), 4.59(m, 1 H), 3.09 (m, 2H), 2.45 (m, 1H), 2.11 (m, 2H). 1.87(m, 4H), 1.24-1.65(m, 8H) |
| 17 | 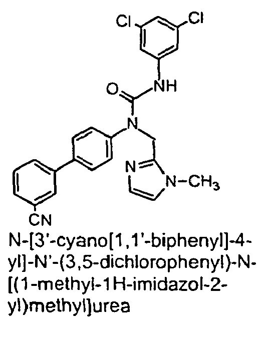 | 476.1043 | |
| 18 | 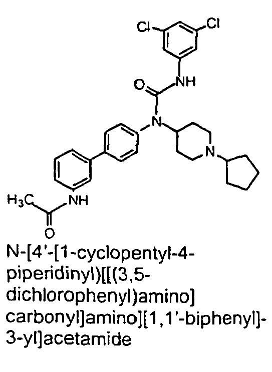 | 565.2145 | 7.83 (s, 1 H), 7.62 (d, J=9.0 Hz, 2 H), 7.38-7.47 (m, 2 H), 7.28 (m, 6 H), 7.15 (d, J = 9.0 Hz, 2 H), 6.95 (s, 1 H), 6.11 (s, 1 H), 4.56 (m, 1 H), 3.09 (m, 2 H), 2.45 (m, 1 H), 2.21 (s, 3 H), 2.13 (m, 2 H), 1.87 (m, 4 H), 1.36-1.66 (8 H) |
| 19 | 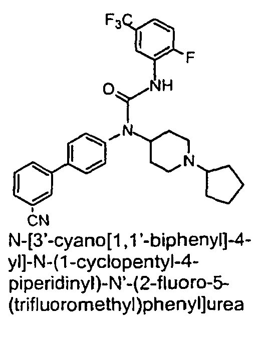 | 551.2427 | |
| 20 | 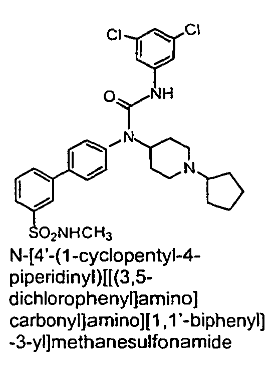 | 601.1798 | |
| 21 | 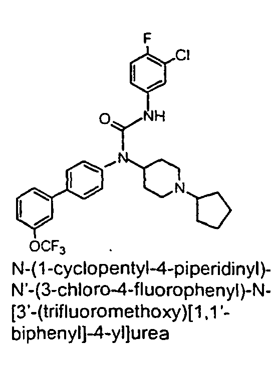 | 576.2035 | |
| 22 | 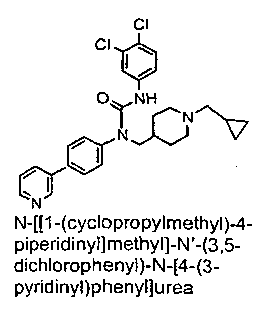 | 509.1879 | 8.90 (s, 1 H); 8.66(d, J = 3.6 Hz, 1 H); 7.92(d, J=8.2 Hz, 2 Hz, 2 H); 7.72 (d, J=8.2 Hz; 2 H); 7.45, 7.40 (m, 2 H); 7.27 (s, 2 H); 6.98 (d, J= 1.7 Hz, 1 H); 6.28 (s, 1 H); 3.70 (d, J= 6.9 Hz, 2 H); 3.13-3.08(m,2H);2.29(d, J =5.5 Hz, 2H);2.06-2.02(m,2H);1.99-1.70 (m, 3 H); 1.50-1.41 (m, 2 H); 0.90 (m, 1 H); 0.55-0.49 (m, 2 H); 0.12-0.10 (m, 2 H). |
| 23 | 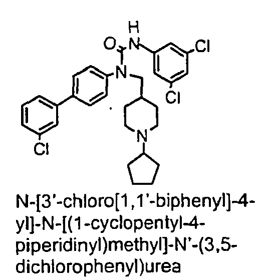 | 558.1667 | 7.68 (d, J = 8.1 Hz, 2 H); 7.62 (s, 1 H); 7.52-7.45 (m, 5 H); 7.38 (s, 2 H);6.97 (d, J = 1.7 Hz, 1 H); 6.24 (s, 1 H); 3.67 (d, J = 6.6 Hz, 2 H); 3.03 (d, J = 11.0 Hz, 2 H); 2.49 (m, 1 H); 2.01-1.26 (m, 15 H). |
| 24 | 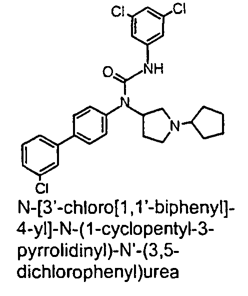 | 530.1354 | 7.60-7.27 (m, 11 H); 6.97 (s, 1 H); 4.48 (m, 2 H); 3.33-3.30 (m, 2 H); 2.63-2.58 (m, 2 H); 2.50 (m, 1 H); 2.48-2.25 (m, 2 H); 2.00-1.26 (m, 8 H). |
| 25 | 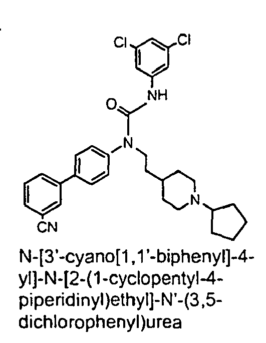 | 561.2125 | 7.91 (t, J= 1.4 Hz, 1 H); 7.86 (m, 1 H); 7.70 (d, J = 8.5 Hz, 2 H); 7.69 (m, 1 H); 7.61 (t, J = 7.6 Hz, 1 H); 7.39 (d, J =8.5 Hz, 2 H); 7.26 (s, 2 H); 6.98 (d, J = 1.8 Hz, 1 H); 6.18 (s, 1 H): 3.80 (t, J = 7.1 Hz, 2 H); 3.28-3.10 (m, 2 H); 2.68 (m, 1 H); 2.20-2.11 (m, 2 H); 1.94-1.38 (m, 15 H). |
| 26 | 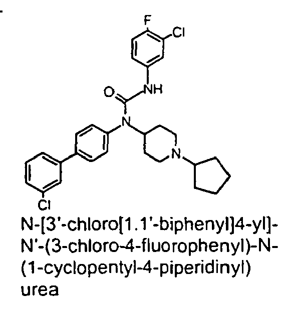 | 526.1824 | |
| 27 | 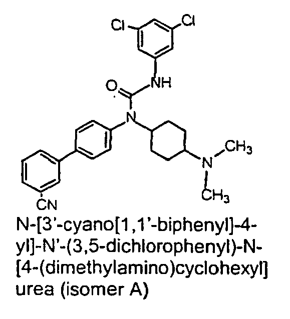 | 507.1724 | |
| 28 | 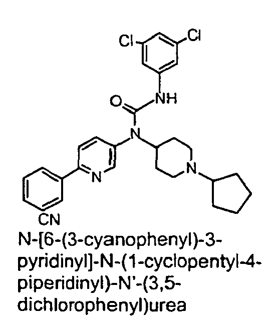 | 534.1831 | |
| 29 | 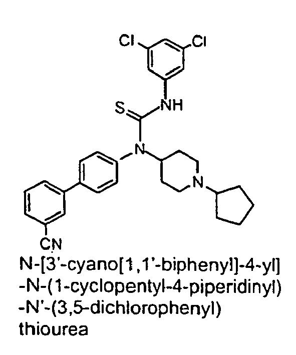 | 549.1633 | |
| 30 | 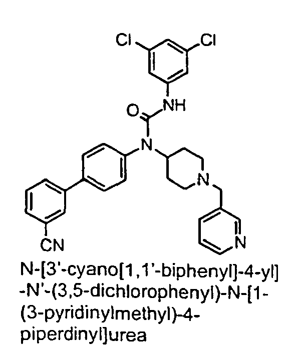 | 556.1678 | |
| 31 | 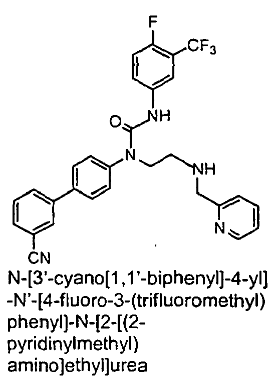 | 534.1917 | |
| 32 | 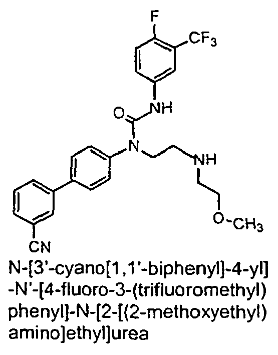 | 501.1921 | |

| Ex. | Structure/Name | HRMS | 300MHZ-¹H NMR CDCl₃, |
|---|---|---|---|
| 33 | 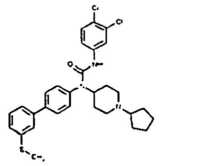 | 554.1809 | |
| 34 | 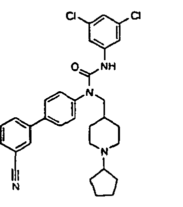 | 547.2036 | |
| 35 | 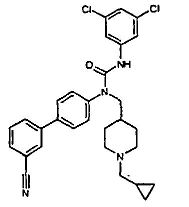 | 533.1867 | |
| 36 | 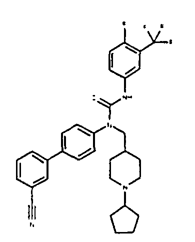 | 565.2595 | 7.86 (s, 1 H), 7.85 (d, *J* = 7.8 Hz, 1 H), 7.71 (m, 1 H). 7.70 (d, *J* = 8.4 Hz, 2 H), 7.60 (t, *J* = 7.5 Hz. 1 H). 7.52 (d, *J* = 3.0 Hz, 1 H), 7.50 (d, *J* = 3.0 Hz, 1 H), 7.43 (d, *J* = 8.4 Hz, 2 H), 7.07 (t, *J* = 9.9 Hz, 1 H), 6.27 (s, 1 H), 3.69 (d, *J* = 6.3 Hz, 2 H), 3.04 (d, *J* = 11.1 Hz, 2 H), 2.50 (m, 1 H), 2.25 (s, 1 H), 1.98 (t, *J* = 11.1 Hz, 2 H), 1.85 - 1.68 (m, 6 H), 1.57 - 1.38 (m, 6 H) |
| 37 | 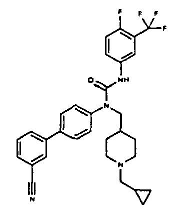 | 551.2443 | |
| 38 | 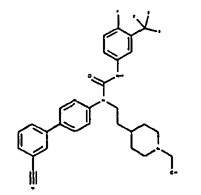 | 539.2435 | |
| 39 | 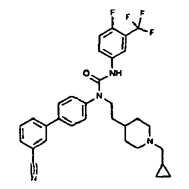 | 565.2592 | 7.90 (s, 1 H), 7.85 (dt, *J* = 8.1, 1.5, 1.2 Hz, 1H), 7.72 (m, 1 H), 7.71 (d, *J* = 8.4 Hz, 2 H), 7.61 (t, J = 7.8 Hz, 1 H), 7.52 (d, *J* = 3.6 Hz, 1 H), 7.50 (d, *J* = 2.7 Hz, 1 H), 7.43 (d, *J* = 8.4 Hz, 2 H), 7.08 (t, *J* = 9.6 Hz, 1 H), 6.20 (s, 1 H), 3.82 (t, *J* = 7.5 Hz, 2 H), 3.08 (d, *J* = 11.1 Hz, 2 H), 2.27 (d, *J* = 6.6 Hz, 2 H), 1.98 (t, *J* = 10.5 Hz, 2 H), 1.74 - 1.71 (m, 2 H), 1.56 - 1.53 (m, 2 H), 1.35 - 1.25 (m, 3 H), 0.88 (m, 1 H), 0.54 - 0.48 (m, 2 H), 0.10 (q, *J* = 5.1 Hz, 2 H) |
| 40 | 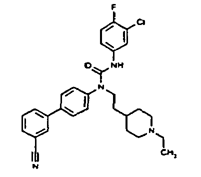 | 505.2172 | |
| 41 | 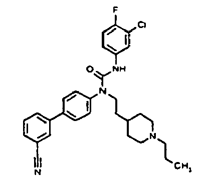 | 519.2350 | |
| 42 | 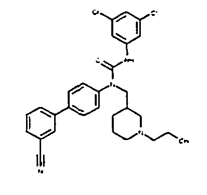 | 521.1872 | |
| 43 | 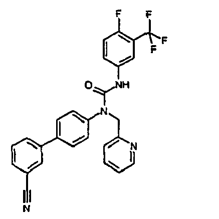 | 491.1493 | |

### EXAMPLE 44

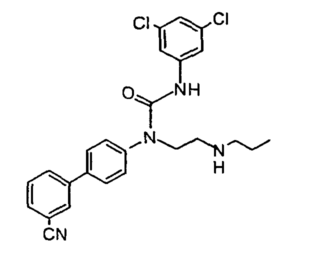

### N-3'-cyano[1,1'-biphenyl]-4-yl]-N'-(3,5-dichlorophenyl)-N-[2-(propylamino)ethyl]urea

To a solution of 4-bromoaniline (16.86 g, 98.0 mmol) in toluene (40 ml) was added 2-bromoethylamine hydrobromide (10.0 g, 49.0 mmol) and heated to 110°C for 4 h. The reaction mixture was then cooled to rt and poured into water (60 mL) and 50% KOH (20 mL). The layers were separated and the aqueous layer is then extracted with toluene. The combined organic layer was then dried, concentrated and chromatographed over silica gel (eluting with 90% MeOH/methylene chloride and 1 % NH₄OH) to afford 8.41 g (40%) of diamine.

To a solution of amine (8.41 g, 39.11 mmol) in methylene chloride (200 mL) was added DMAP (5 mg) followed by drop-wise addition of BOC₂O (8.53 g, 39.11 mmol in methylene chloride) over 45 min and stirred for 1 h at rt. The reaction mixture was then poured into 10% NaOH and extracted with methylene chloride, dried, concentrated to yield 12.44 g (100%) of mono BOC-protected amine as a solid.

To a stirred solution of amine (5.36 g, 17.01 mmol) in 40 mL of DME:H₂O (4:1) was added 3-cyanophenylboronic acid (5.00g, 34.03 mmol), PdCl₂(PPh₃)₂ (1.19 g, 1.70 mmol), Na₂CO₃ (5.41 g, 51.05 mmol) and heated to 80°C for 24h. The reaction mixture was then cooled to rt, poured into aqueous NaOH and extracted with ether. The combined extracts were then dried, concentrated and chromatographed to yield 1.1 g (19%) of biaryl amine as a solid.

To a stirred solution of amine (0.300 g, 0.890 mmol) and triethyl amine (0.620 mL, 4.45 mmol) in methylene chloride (5 ml) was added 3,5-dichlorophenyl isocyanate (0.837 g, 4.45 mmol) and stirred at rt for 24 h. The reaction mixture was then diluted with methylene chloride, washed with aqueous NaOH, dried, concentrated and chromatographed to yield 0.49 g (100%) of urea as an oil. To a stirred solution of biaryl amine (0.490 g, 0.89 mmol) in methylene chloride (5 mL) was added trifluoroacetic acid (1.3 mL) and stirred at rt for 2 h. The reaction mixture was concentrated and poured into 10% NaOH and extracted with methylene chloride. The combined extracts were dried and concentrated to afford 0.35 g (92%) of amine.

To stirred solution of amine (0.075 g, 0.23 mmol) and propionaldehyde (0.016 mL, 0.23 mmol) in methylene chloride (2 mL) was added sodium triacetoxyborohydride (0.054 g, 0.254 mmol), acetic acid (0.015 mL, 0.254 mmol) and stirred at rt for 24 h. The reaction mixture was quenched with aqueous NaOH and extracted with methylene chloride. The combined extracts were dried, concentrated and chromatographed to yield 0.026 g (25%) of amine as a solid.

300 MHz - ¹H NMR (CDCl₃) δ 7.86 (s, 1 H), 7.79-7.82 (m, 1 H), 7.54-7.68 (m, 4H), 7.41 (d, J = 8.4 Hz, 2 H), 7.35 (m, 2 H), 6.96 (m, 1 H), 3.88 (t, J= 5.4 Hz, 2 H), 2.92 (t, J = 5.7 Hz, 2 H), 2.68 (t, J = 7.5 Hz, 2 H), 1.52- 1.63 (m, 3 H), 0.95 (t, J = 7.5 Hz, 3 H).
HRMS (M+H⁺) 467.1400

### EXAMPLES 45-73

Employing preparative procedures similar to those described in Example 44, the following compounds shown below in Table II were prepared.

**TABLE II**

| Ex. | Structure/Name | HRMS | 300MHZ-¹H NMR CDCl₃, δ |
|---|---|---|---|
| 45 | 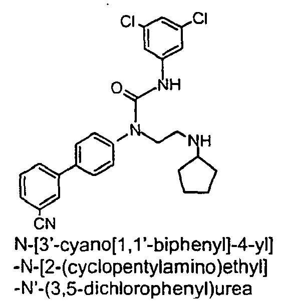 | 493.1562 | |
| 46 | 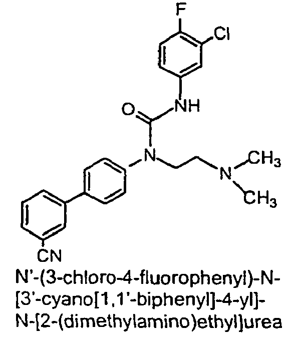 | 437.1548 | 7.80-7.86 (m, 3 H), 7.60-7.66 (m, 5 H), 7.41 (d, J= 9.0 Hz, 2 H), 7.11 (m, 1 H), 7.01 (t, J = 8.7 Hz, 1 H), 3.89 (t, J = 5.4 Hz, 2 H), 2.63 (t, J = 5.4 Hz, 2 H), 2.40 (s, 6 H) |
| 47 | 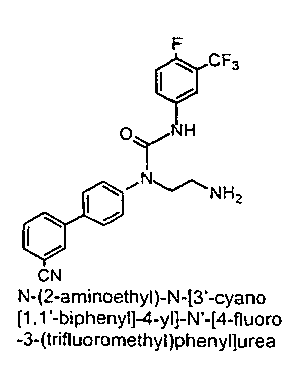 | 443.1490 | |
| 48 | 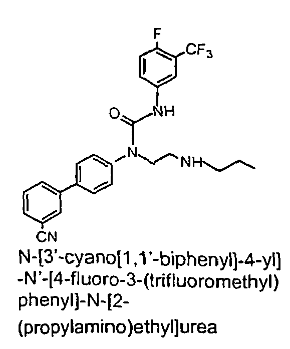 | 485.1960 | 7.87-7.80 (m, 3 H), 7.55-7.66 (m, 6 H), 7.47 (d, J = 9.0 Hz, 2 H), 7.09 (t, J = 10.0 Hz, 1 H), 3.96 (t, J = 5.4 Hz, 2 H), 2.98 (t, J = 5.7 Hz, 2 H), 2.75 (t, J = 7.2 Hz, 2 H), 2.50 (m, 1 H), 1.59-1.66 (m, 3 H), 0.96 (t, J = 7.5 Hz, 3 H). |
| 49 | 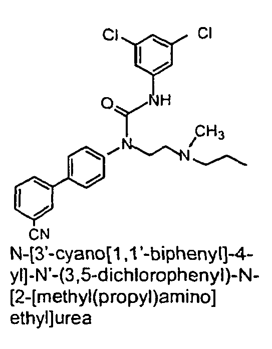 | 481.1553 | |
| 50 | 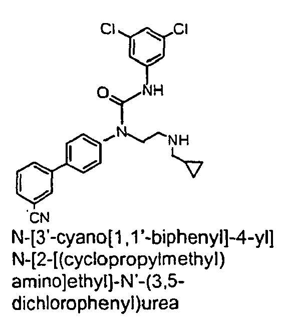 | 479.1400 | |
| 51 | 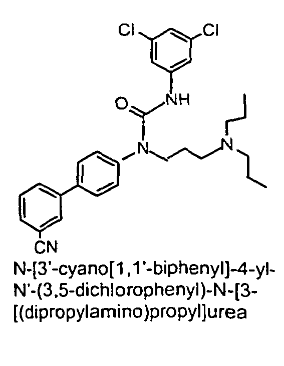 | 523.2023 | |
| 52 | 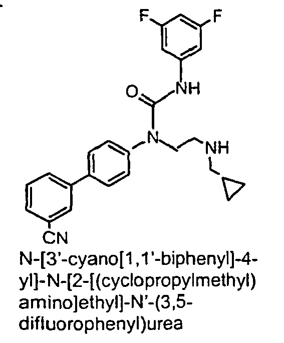 | 447.1992 | |
| 53 | 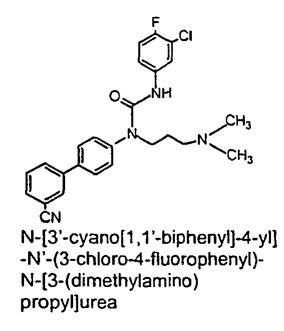 | 451.1706 | 9.40 (s, 1 H), 7.79-7.86 (m, 2 H), 7.56-7.63 (m, 5 H), 7.39 (d, J = 9.0 Hz, 2 H), 7.17 (m, 1 H), 7.03 (t, J = 8.7 Hz, 1 H), 3.90 (t, J = 6.0 Hz, 2 H), 2.48 (t, J = 6.6 Hz, 2 H), 2.34 (s, 6 H), 1.75 (t, J = 6.0 Hz, 2 H) |
| 54 | 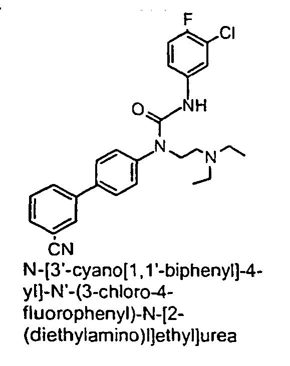 | 465.1853 | |
| 55 | 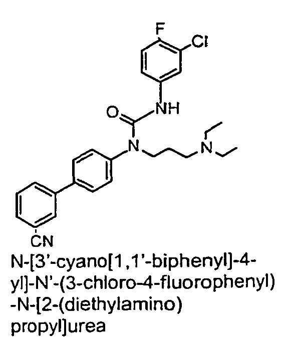 | 479.2007 | |
| 56 | 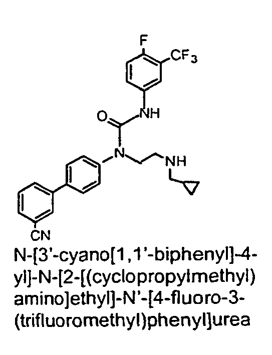 | 497.1971 | 8.90 (s, 1 H), 7.86-7.80 (m, 2H), 7.55-7.66 (m, 6 H), 7.58 (d, J = 8.1 Hz, 2 H), 7.08 (t, J = 9.3 Hz, 1 H), 3.93 (t, J = 5.4 Hz, 2 H), 2.98 (t, J = 5.7 Hz, 2 H), 2.60 (t, J = 6.9 Hz, 2 H), 2.20 (m, 1 H), 1.00 (m, 1 H), 0.53 (m, 2 H), 0.18 (m, 2 H) |
| 57 | 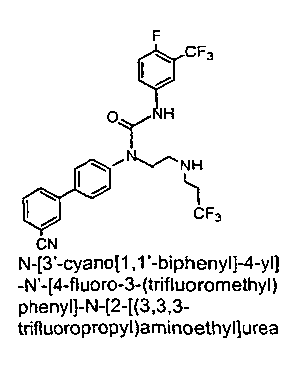 | 539.1691 | |
| 58 | 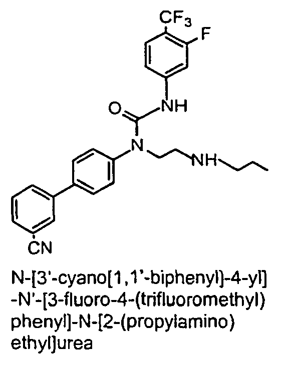 | 485.1964 | |
| 59 | 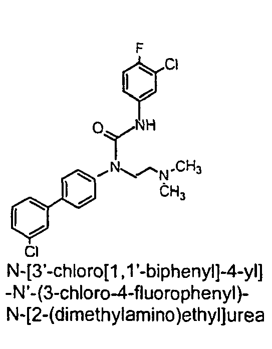 | 446.1195 | |
| 60 | 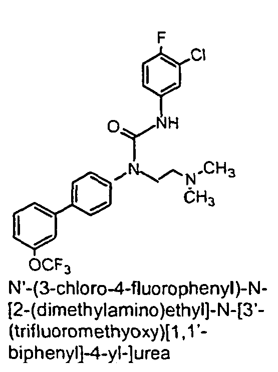 | 496.1410 | |

| Ex. | Structure/Name | HRMS | 300MHZ-¹H NMR CDCl₃, |
|---|---|---|---|
| 61 | 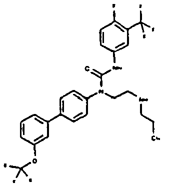 | 544.1837 | |
| 62 | 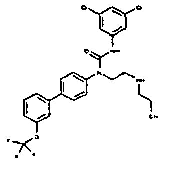 | 526.1270 | |
| 63 | 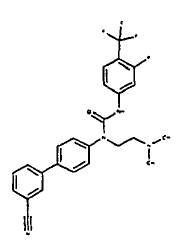 | 471.1810 | |
| 64 | 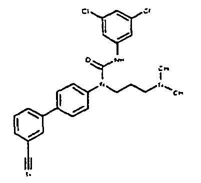 | 467.1409 | |
| 65 | 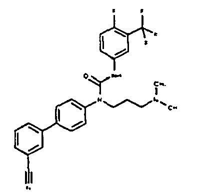 | 485.1970 | |
| 66 | 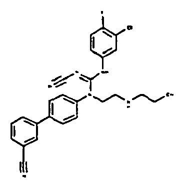 | 475.1821 | |
| 67 | 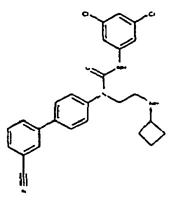 | 479.1403 | |
| 68 | 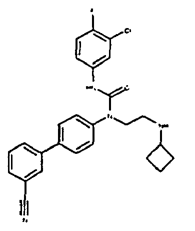 | 463.1702 | |
| 69 | 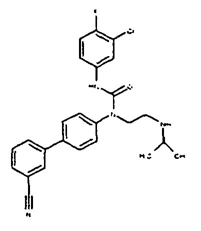 | 451.1692 | |
| 70 | 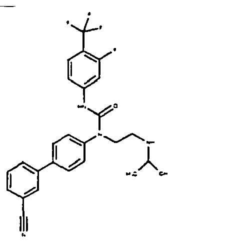 | 485.1969 | |
| 71 | 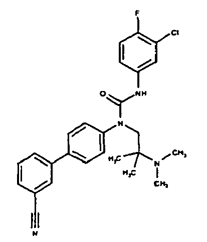 | 465.1858 | |
| 72 | 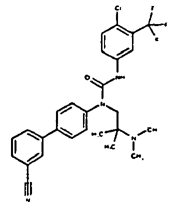 | 515.1823 | |
| 73 | 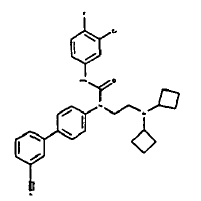 | 517.2171 | |

### EXAMPLE 74

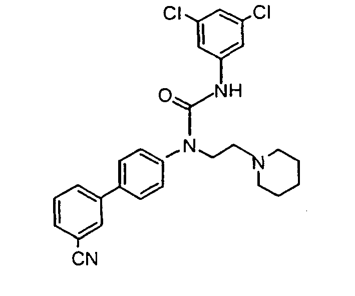

### N-[3'-cyano[1,1'-biphenyl]-4-yl]-N-(3,5-dichlorophenyl) -N-[2-(1-piperidinyl)ethyl]urea

To a stirred solution of 4-bromoaniline (10.0 g, 58.1 mmol) in 500 mL of DME:H₂O (4:1) was added 3-cyanophenylboronic acid (13.3 g, 91.2 mmol), PdCl₂dppf(4.72 g, 5.86 mmol), 2N Na₂CO₃ (100 mL) and heated to 100°C for 5 h. The reaction mixture was then cooled to rt, poured into aqueous NaOH and extracted with EtOAc. The combined extracts are then dried, concentrated and chromatographed to yield 7.4 g (66%) of biaryl amine as a solid.

To a stirring solution of biaryl aniline (4.49 g, 23.14 mmol) and chloroacetaldehyde (2.18 g, 27.77 mmol) in 4 mL of 1:1 of 6M HCI:MeOH in MeOH (60 mL) was added sodium cyanoborohydride (1.63 g, 25.92 mmol). The reaction mixture was then stirred at rt for 5 days, concentrated, diluted with methylene chloride and washed with NaHCO₃, dried, concentrated and chromatographed to yield 4.19 g (67%) of chloride as a solid.

To a stirred solution of chloride (0.350 g, 1.28 mmol) in propionitrile (10 mL) was added piperidine (0.546 mL, 6.43 mmol), Nal (0.19, 1.28 mmol), Na₂CO₃ (0.203 g, 1.92 mmol) and heated to 80°C for 12 h. The reaction mixture was then cooled to rt and diluted with EtOAc and water. The layers were separated and the organic layer is then dried, concentrated and chromatographed to yield 0.32 g (78%) as an oil.

To a stirred solution of amine (0.04 g, 0.124 mmol) and triethyl amine (0.038 mL, 0.372 mmol) in methylene chloride (2 ml) was added 3,5-dichlorophenyl isocyanate (0.069 g, 0.372 mmol) and stirred at rt for 24 h. The reaction mixture was then diluted with methylene chloride, washed with aqueous NaOH, dried, concentrated and chromatographed to yield 0.048 g (63%) of urea as an oil.

### EXAMPLES 75-200

Employing preparative procedures similar to those described in Example 74, the following compounds shown below in Table III were prepared:

**TABLE III**

| Ex. | Structure/Name | HRMS | 300MHZ-¹H NMR CDCl₃, δ |
|---|---|---|---|
| 75 | 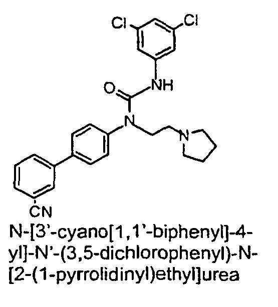 | 479.1400 | |
| 76 | 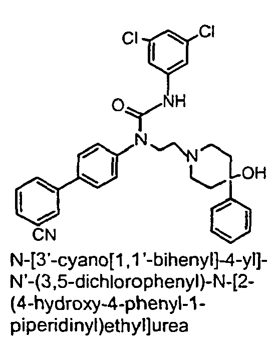 | 585.1816 | |
| 77 | 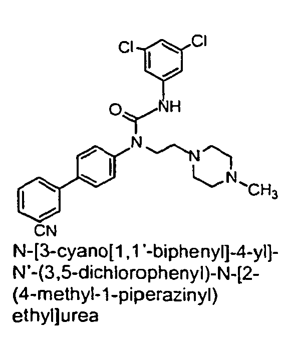 | 508.1664 | 7.81-7.86 (m, 3 H), 7.58-7.66 (m, 5 H), 7.43 (d, J = 8.7 Hz, 2 H), 7.36 (m, 1 H), 6.98 (m, 1 H), 3.89 (t, J = 5.7 Hz, 2 H), 2.63 (m, 7 H), 2.49 (s, 4 H), 2.31 (s, 3 H) |
| 78 | 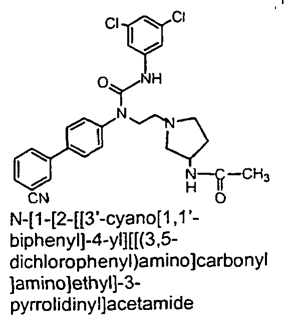 | 536.1630 | |
| 79 | 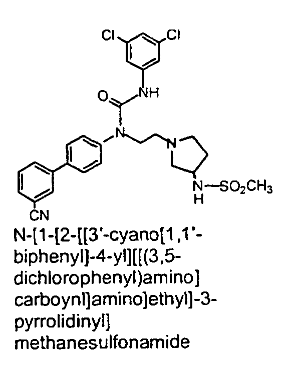 | 572.1296 | |
| 80 | 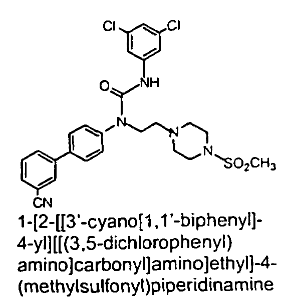 | 572.1284 | |
| 81 | 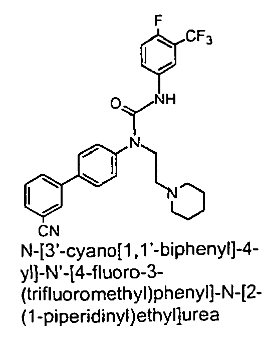 | 511.2121 | |
| 82 | 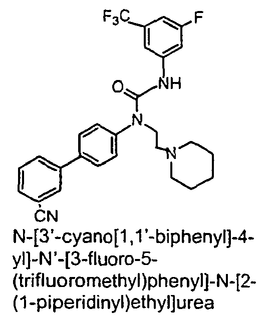 | 511.2121 | |
| 83 | 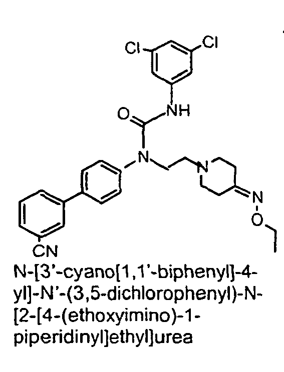 | 550.1972 | |

| Ex. | Structure/Name | HRMS | 300MHZ-¹H NMR CDCl₃, |
|---|---|---|---|
| 84 | 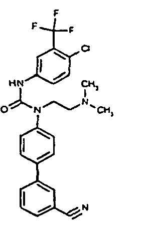 | 487.1514 | |
| 85 | 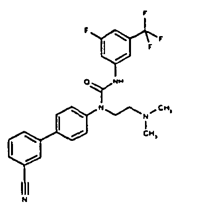 | 471.1810 | |
| 86 | 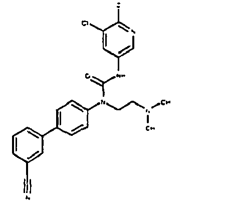 | 454.1212 | |
| 87 | 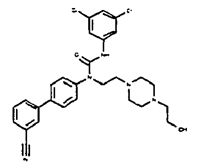 | 536.1986 | 8.07 (s, 1H), 7.87-7.80 (m, 2H), 7.68-7.58 (m, 4H), 7.43 (d, *J* = 9.3 Hz, 2H), 7.38 (d, *J* = 2.0 Hz, 2H). 6.98 (d, *J* = 2.0 Hz, 1H), 3.89 (t, *J* = 6.3 Hz, 2H), 2.66-2.62 (m, 4H), 2.53 (m, 2H), 2.36-2.31 (m, 2H) 1.53-1.50 (m, 2H), 0.90 (t, *J* = 8.3 Hz. 3H) |
| 88 | 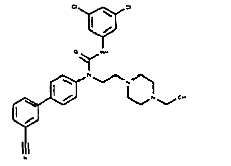 | 522.1820 | |
| 89 | 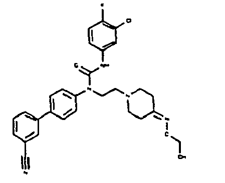 | 534.2066 | |
| 90 | 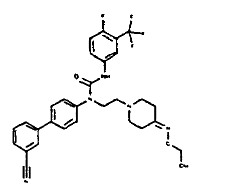 | 568.2345 | |
| 91 | 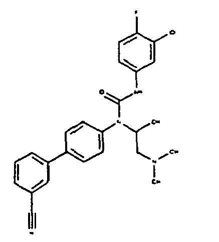 | 451.1700 | |
| 92 | 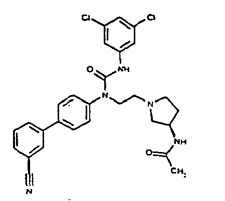 | 536.1622 | 7.86-7.80 (m, 2H), 7.66 (d, J = 9.3 Hz, 2H), 7.61-7.55 (m, 2H), 7.42 (d, J = 9.3 Hz, 2H), 7.28 (d, J = 2 Hz, 2H), 7.13 (s, 1H), 6.95 (s, 1H), 6.18 (d, J = 8.3 Hz, 1H), 4.36-4.34 (m, 1H), 4.06-3.99 (m, 1H), 3.74-3.64 (m, 1H), 2.98-2.91 (m, 1H), 2.83-2.49 (m, 4H), 2.35-2.22 (m, 2H), 1.86 (s, 3H), 1.67-1.63 (m, 1H) |
| 93 | 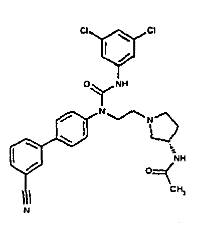 | 536.1622 | |
| 94 | 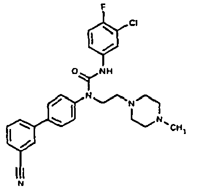 | 492.1966 | |
| 95 | 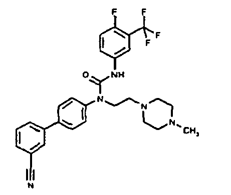 | 526.2232 | |
| 96 | 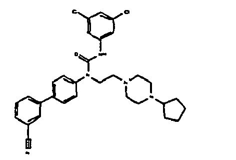 | 562.2142 | |
| 97 | 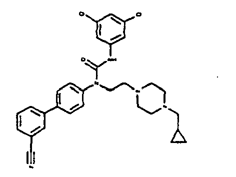 | 548.2018 | |
| 98 | 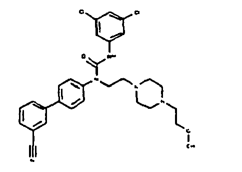 | 552.1935 | |
| 99 | 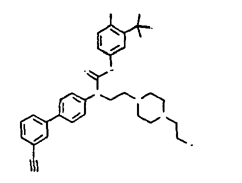 | 554.2546 | |
| 100 | 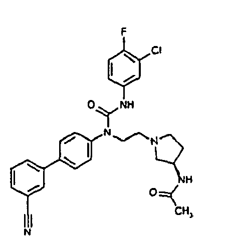 | 520.1911 | |
| 101 | 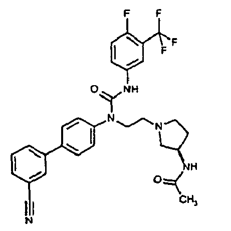 | 554.2173 | |
| 102 | 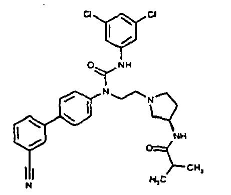 | 564.1938 | |
| 103 | 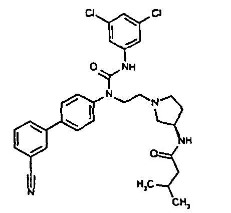 | 578.2097 | |
| 104 | 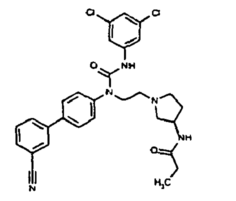 | 550.1784 | |
| 105 | 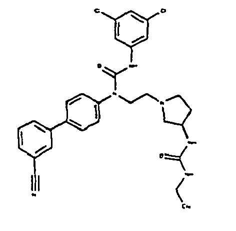 | 565.1887 | |
| 106 | 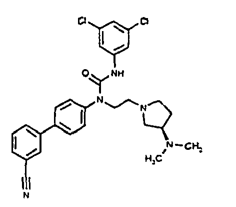 | 522.1820 | |
| 107 | 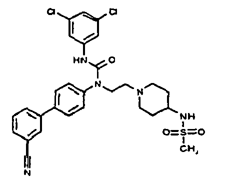 | 586.1770 | |
| 108 | 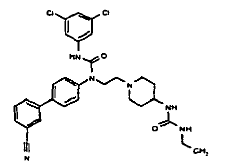 | 579.2042 | |
| 109 | 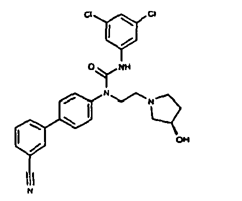 | 495.1353 | |
| 110 | 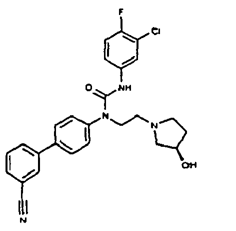 | 479.1657 | |
| 111 | 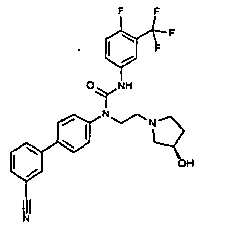 | 513.1913 | 8.22 (s, 1H), 7.86-7.80 (m, 2H), 7.67-7.54 (m, 6H), 7.44 (d, *J* = 9.3 Hz, 2H), 7.07 (t, *J* = 10.3 Hz, 1H), 4.42-4.38 (m, 1H), 3.91 (t, *J* = 6.3 Hz, 2H), 3.07-3.01 (m, 1H), 2.87-2.84 (m, 1H), 2.79 (t, *J* = 6.3 Hz, 2H), 2.65-2.60 (m, 1H), 2.46-2.38 (m, 1H), 2.29-2.14 (m, 2H), 1.81-1.77 (m, 1H) |
| 112 | 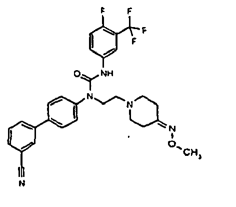 | 554.2189 | |
| 113 | 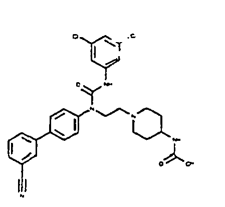 | No HRMS | |
| 114 | 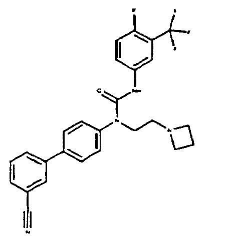 | 483.1801 | |
| 115 | 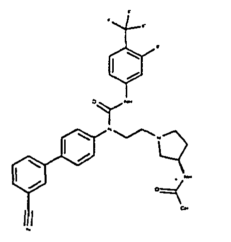 | 554.2150 | |
| 116 | 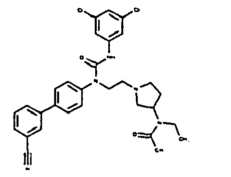 | 564.1933 | |
| 117 | 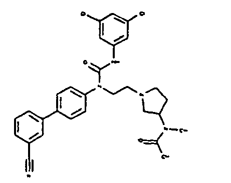 | 550.1795 | |
| 118 | 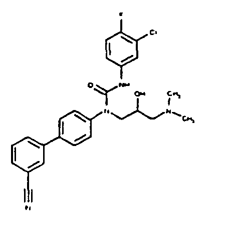 | 467.1648 | |
| 119 | 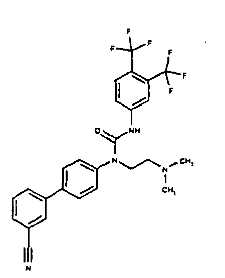 | 521.1782 | |
| 120 | 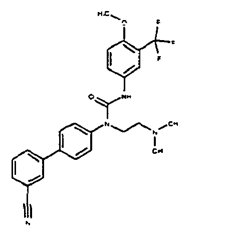 | 483.2002 | |
| 121 | 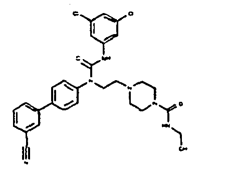 | 565.1876 | |
| 122 | 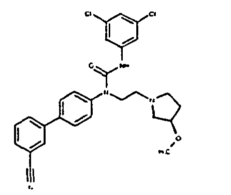 | 509.1519 | |
| 123 | 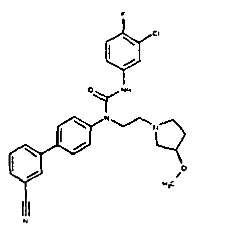 | 493.1803 | |
| 124 | 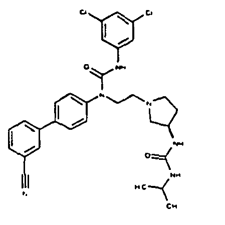 | 579.2051 | |
| 125 | 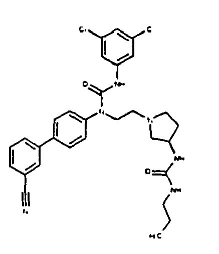 | 579.2051 | |
| 126 | 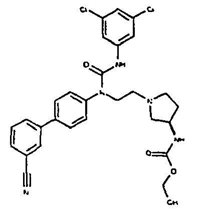 | 566.1717 | |
| 127 | 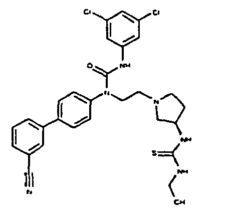 | 581.1665 | |
| 128 | 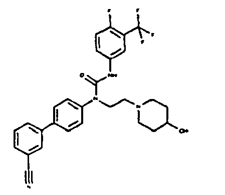 | 527.2070 | |
| 129 | 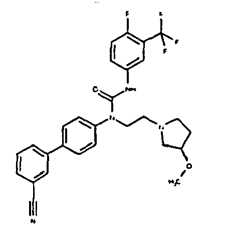 | 527.2078 | |
| 130 | 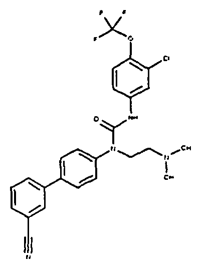 | 503.1465 | |
| 131 | 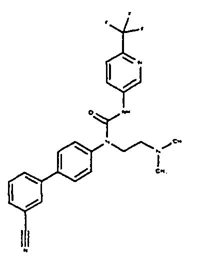 | 454.1849 | |
| 132 | 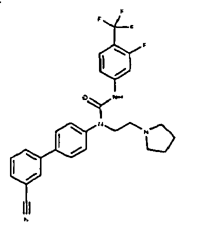 | 497.1961 | |
| 133 | 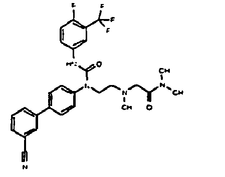 | 542.2179 | |
| 134 | 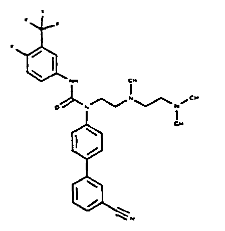 | No HRMS | |
| 135 | 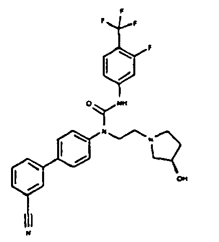 | 513.1923 | 8.55 (s, 1H), 7.87-7.80 (m, 2H), 7.68-7.53 (m, 5H), 7.46-7.38 (m, 3H), 7.11 (d, *J* = 10.0 Hz, 1H), 4.45-4.41 (m, 1H), 3.92 (t, *J* = 6.3 Hz, 2H), 3.11-3.04 (m, 1H), 2.95-2.86 (m, 1H), 2.82 (t, *J* = 6.3 Hz, 2H). 2.68-2.63 (m, 1H), 2.49-2.41 (m, 1H), 2.27-2.15 (m, 2H), 1.87-1.79 (m, 1H) |
| 136 | 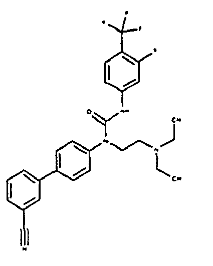 | 499.2128 | |
| 137 | 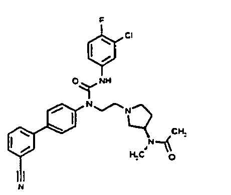 | 534.2073 | |
| 138 | 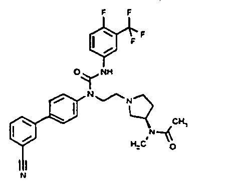 | 568.2324 | |
| 139 | 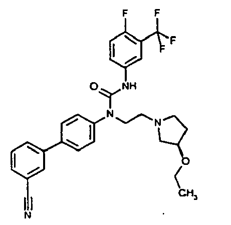 | 541.2228 | |
| 140 | 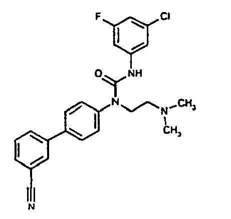 | 437.1548 | |
| 141 | 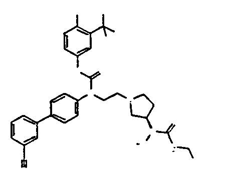 | 597.2611 | |
| 142 | 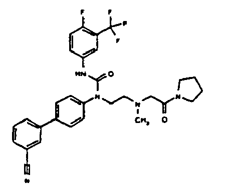 | 568.2346 | |
| 143 | 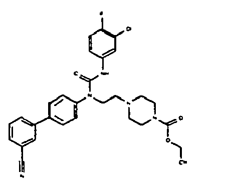 | 550.2014 | |
| 144 | 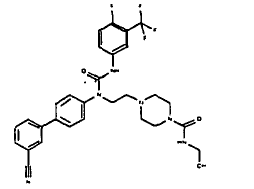 | 583.2457 | |
| 145 | 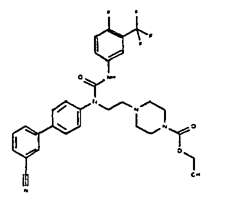 | 584.2292 | |
| 146 | 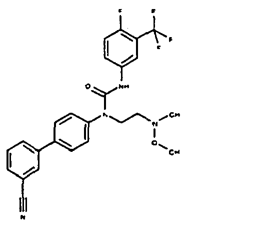 | 487.1756 | |
| 147 | 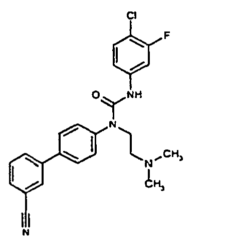 | 437.940 | |
| 148 | 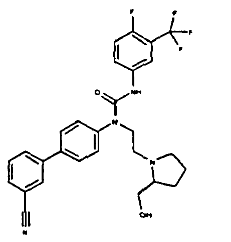 | 527.2077 | |
| 149 | 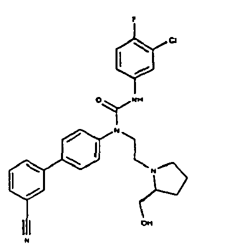 | 493.1803 | |
| 150 | 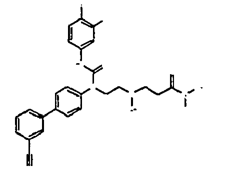 | 538.1772 | |
| 151 | 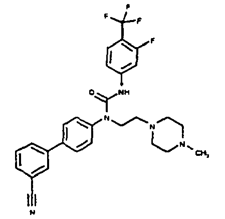 | 526.2224 | |
| 152 | 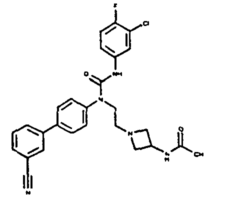 | 506.1769 | |
| 153 | 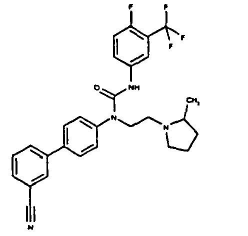 | 511.2128 | |
| 154 | 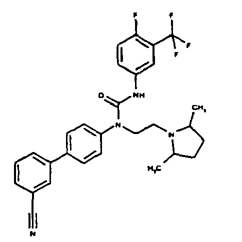 | 525.2289 | 7.85-7.78 (m, 2H), 7.67-7.53 (m, 6H), 7.42 (d, *J* = 12.3 Hz, 2H), 7.12 (t, *J* = 10.6 Hz, 1H), 4.01 (t, 2H), 2.87 (t, 2H), 2.68 (m, 2H), 2.04 (m, 2H), 1.56 (m, 2H), 1.27 (d, 6H) |
| 155 | 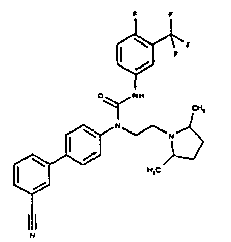 | 525.2284 | |
| 156 | 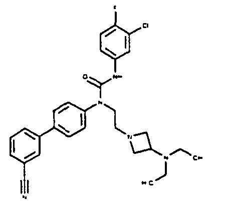 | 520.2288 | |
| 157 | 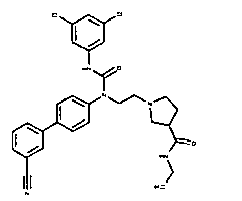 | 550.1784 | |
| 158 | 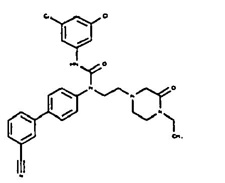 | 525.2289 | |
| 159 | 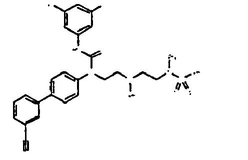 | 574.1459 | 7.84 (d, *J* = 7.7 Hz, 1 H), 7.67 (m, 3 H), 7.59(t, *J* = 7.7 Hz 1 H), 7.47 (d, *J* = 8.2 Hz, 2 H), 7.28 (d, *J* = 1.8 Hz, 2 H), 6.97 (t, *J* = 1.8 Hz, 1 H), 3.87 (t, *J* = 6.2 Hz, 2 H), 3.26 (t, *J* = 6.6 Hz, 2 H), 2.89 (s, 3 H), 2.81 (s, 3 H), 2.67 (m, 4 H), 2.35 (s, 3 H) |
| 160 | 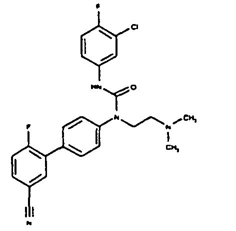 | 455.1452 | |
| 161 | 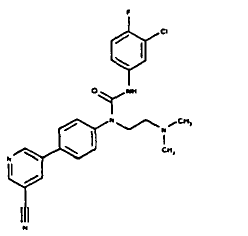 | 438.1502 | |
| 162 | 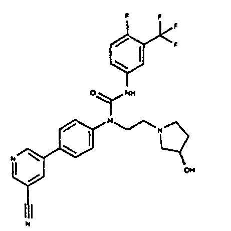 | 514.1873 | |
| 163 | 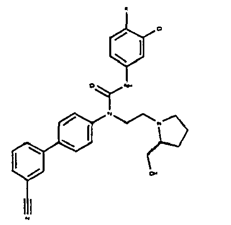 | 493.1803 | |
| 164 | 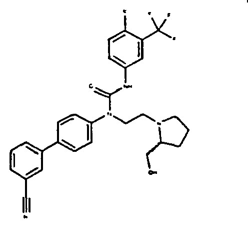 | 527.2080 | |
| 165 | 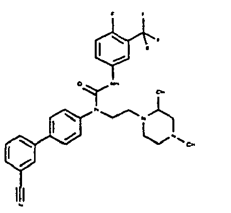 | 540.2377 | |
| 166 | 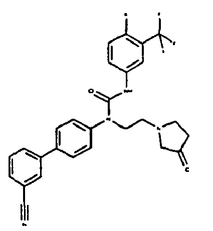 | 511.1753 | |
| 167 | 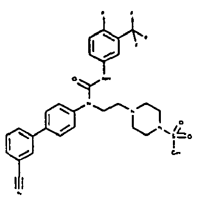 | 590.1848 | |
| 168 | 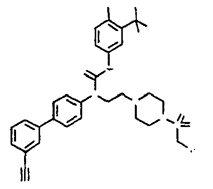 | 604.2005 | |
| 169 | 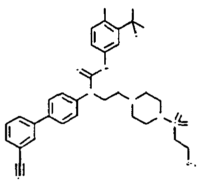 | 618.2162 | |
| 170 | 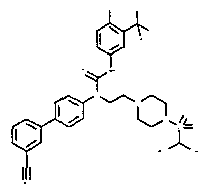 | 618.2162 | |
| 171 | 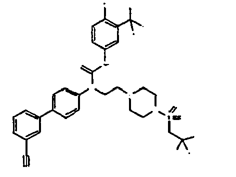 | 658.1719 | |
| 172 | 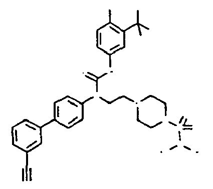 | 619.2112 | |
| 173 | 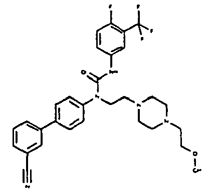 | 570.2493 | |
| 174 | 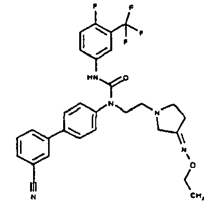 | 554.2177 | |
| 175 | 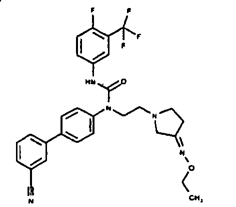 | 554.2177 | |
| 176 | 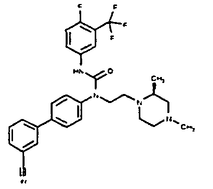 | 540.2390 | |
| 177 | 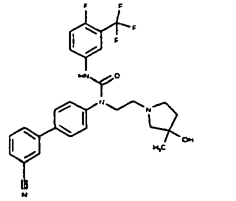 | 527.2072 | |
| 178 | 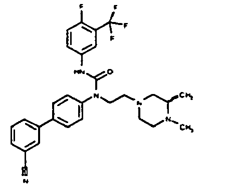 | 540.2385 | |
| 179 | 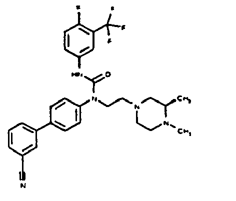 | 540.2385 | |
| 180 | 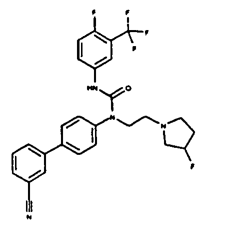 | 515.1877 | |
| 181 | 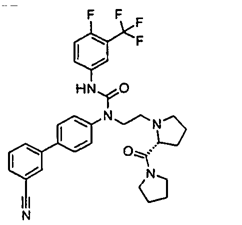 | 594.2497 | |
| 182 | 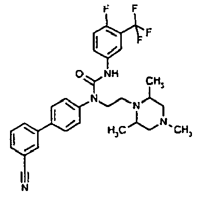 | 554.2543 | |
| 183 | 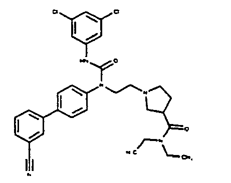 | 578.2097 | |
| 184 | 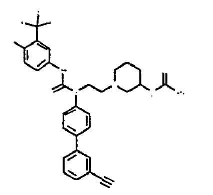 | 568.2334 | |
| 185 | 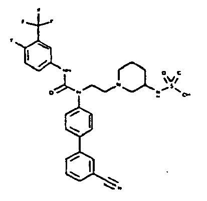 | 604.2011 | |
| 186 | 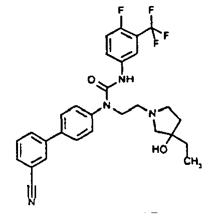 | 541.2223 | 7.87-7.81 (m, 3H), 7.69-7.56 (m, 6H), 7.46 (d, *J* = 12.3 Hz, 2H), 7.08 (t, *J* = 11.0 Hz, 1H), 3.92 (t, *J* = 6.6 Hz, 2H), 3.13-3.08 (m, 1H), 2.87-2.78 (m, 3H), 2.47-2.39 (m, 2H), 1.91-1.84 (m, 1H), 1.63 (q, *J* = 8.3 Hz, 2H), 1.29-1.21 (m, 2H), 0.97 (t, *J* = 8.3 Hz, 3H) |
| 187 | 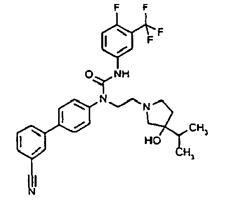 | 555.2374 | |
| 188 | 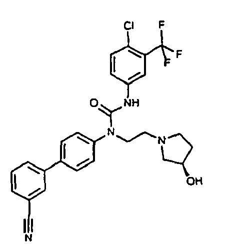 | 529.1626 | |
| 189 | 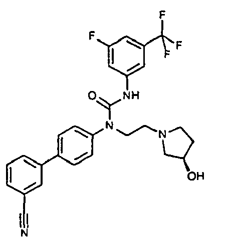 | 513.1908 | |
| 190 | 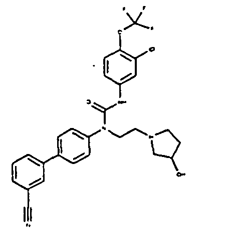 | 545.1573 | |
| 191 | 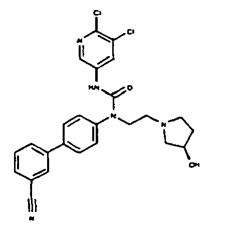 | 496.1304 | |
| 192 | 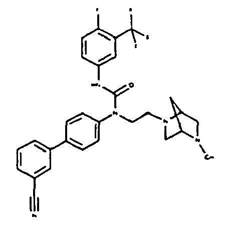 | 538.2238 | 7.86-7.80 (m, 2H), 7.67-7.54 (m, 6H), 7.44 (d, *J* = 9.3 Hz, 2H), 7.09 (t, *J* = 10.3 Hz, 1H), 3.88-3.83 (m, 2H), 3.42-3.32 (m, 2H), 2.95-2.88 (m, 3H), 2.82-2.78 (m, 2H), 2.61-2.57 (m, 1H), 2.42 (s, 3H), 1.82 (d, *J* = 11.3 Hz, 1H), 1.71 (d, *J* = 11.3 Hz, 1H) |
| 193 | 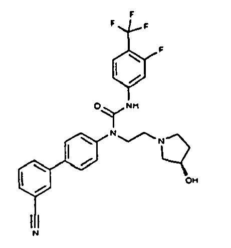 | 513.1914 | |
| 194 | 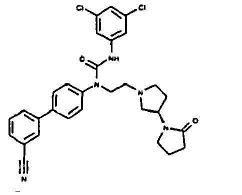 | 562.1785 | |
| 195 | 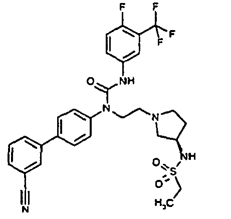 | 605.2082 | |
| 196 | 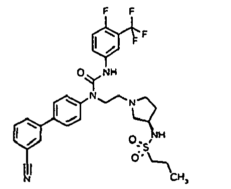 | 618.2170 | |
| 197 | 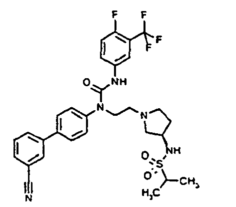 | 618.2170 | |
| 198 | 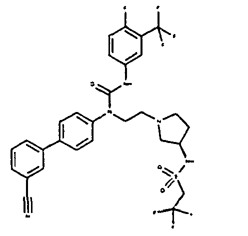 | 659.1789 | |
| 199 | 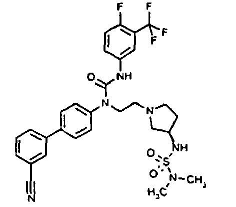 | 619.2106 | |
| 200 | 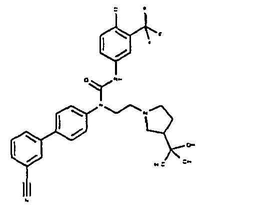 | 571.2079 | |

The following example illustrates the preparation of compounds using method 4:

### EXAMPLE 201

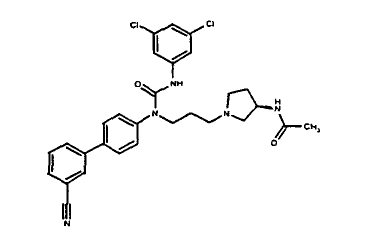

To a stirred solution of 4-bromoaniline (9.4 g, 54.82 mmol) in 250 mL toluene:ethanol:H₂O (3:1:1) was added 3-cyanophenyl boronic acid (16.11 g, 109.65 mmol), Pd(PPh₃)₄ (6.3 g, 5.48 mmol) and Na₂CO₃ (35 g, 330 mmol). The mixture was degassed with N₂, then heated to 100 °C for 24 h. The reaction mixture was concentrated then diluted with EtOAc, washed with H₂O, dried over MgSO₄, filtered, concentrated and chromatographed to yield 4.78 g (45%) of biaryl aniline.

To a stirred solution of biaryl aniline (2.37 g, 12.2 mmol) and 1-chloro-3-iodopropane (1.45 mL, 13.4 mmol) in DMF (20mL) was added K₂CO₃ (3.38 g, 24.4 mmol). The reaction mixture stirred at rt for 3 days, concentrated, diluted with methylene chloride and washed with aqueous NaOH, dried, concentrated and chromatographed to yield 0.94 g (29%) of chloride.

To a solution of the chloride (0.48 g, 1.77 mmol) in acetonitrile (3 mL) was added (3R)-(+)-3-acetamidopyrrolidine (0.68 g, 5.31 mmol), Nal (0.26 g, 1.77 mmol) and Na₂CO₃ (0.28 g, 2.65 mmol) and heated to 90°C for 24 h. The reaction mixture was cooled to rt, diluted with EtOAc, washed with water, aqueous NaOH, dried, concentrated and chromatographed to yield 0.64 g (100%) of amine as a solid.

To a stirred solution of amine (0.100 g, 0.276 mmol) and triethyl amine (0.077 mL, 0.552 mmol) in methylene chloride (1 mL) was added 3,5-dichlorophenyl isocyanate (0.104 g, 0.552 mmol) and stirred at rt for 24 h. The reaction mixture was then diluted with methylene chloride, washed with water, aqueous NaOH, dried, concentrated and chromatographed to yield 0.084 g (55%) of urea.

300 MHz- ¹H NMR (CDCl₃) δ 7.87-7.82 (m, 2H), 7.68-7.56 (m, 4H), 7.40 (d, J = 9.0 Hz, 2H), 7.31 (s, 2H), 6.98 (s, 1 H), 6.15 (d, J = 8.3 Hz, 1 H), 4.44 (m, 1 H), 3.97-3.47 (m, 3H), 2.93 (m, 1H), 2.65-2.54 (m, 4H), 2.33-2.27 (m, 2H), 1.95 (s, 3H), 1.95-1.79 (m, 2H)
HRMS (M+H⁺): 550.1795

### EXAMPLES 202-208

Employing preparative procedures similar to those described in Example 201, the following compounds shown below in Table IV were prepared:

**Table IV**

| Ex. | Structure/Name | HRMS | 300MHZ-¹H NMR CDCl₃, |
|---|---|---|---|
| 202 | 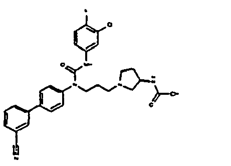 | 534.3035 | |
| 203 | 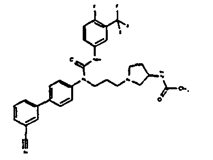 | 568.2374 | |
| 204 | 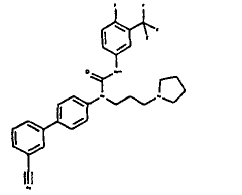 | 511.2128 | |
| 205 | 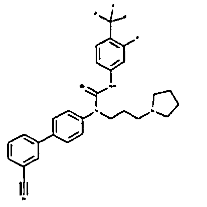 | 511.2133 | |
| 206 | 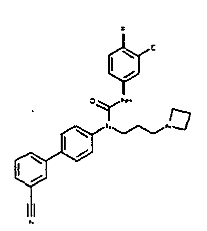 | 463.1698 | 7.84-7.78 (m, 2H), 7.71-7.68 (m, 1H), 7.65-7.55 (m, 4H), 7.38-7.34 (m, 3H), 7.05 (t, *J* = 10.0 Hz, 1H), 3.86 (t, *J* = 6.6 Hz, 2H), 3.32 (m, *J* = 8.0 Hz, 4H), 2.61 (t, *J* = 6.6 Hz, 2H), 2.21 (m. *J* = 8.0 Hz, 2H), 1.61 (m, *J* = 6.66 Hz, 2H) |
| 207 | 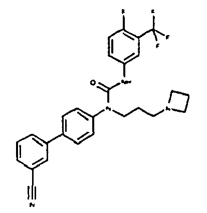 | 497.1962 | |
| 208 | 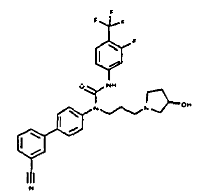 | 527.2068 | |

The following example illustrates the preparation of compounds using method 5.

### EXAMPLE 209

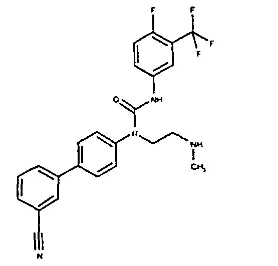

To a stirred solution of 4-bromoaniline (9.4 g, 54.82 mmol) in 250 mL toluene:ethanol:H₂O (3:1:1) was added 3-cyanophenyl boronic acid (16.11 g, 109.65 mmol), Pd(PPh₃)₄ (6.3 g, 5.48 mmol) and Na₂CO₃ (35 g, 330 mmol). The mixture was degassed with N₂, then heated to 100 °C for 24 h. The reaction mixture was concentrated then diluted with EtOAc, washed with H₂O, dried over MgSO₄, filtered, concentrated and chromatographed to yield 4.78 g (45%) of biaryl aniline.

To a stirred solution of biaryl aniline (4.49 g, 23.14 mmol) and chloroacetaldehyde (2.18 g, 27.77 mmol) in 4 mL of 1:1 of 6M HCl:MeOH in MeOH (60mL) was added sodium cyanoborohydride (1.63 g, 25.92 mmol). The reaction mixture stirred at rt for 5 days, concentrated, diluted with methylene chloride and washed with NaHCO₃, dried, concentrated and chromatographed to yield 4.19 g (67%) of chloride as a solid.

To a solution of the chloride (1.39 g, 5.35 mmol) in 2M methylamine solution in THF (50 mL) was added Nal (0.80 g, 5.35 mmol) and Na₂CO₃ (1.13 g, 10.7 mmol) and heated to 90°C for 12 h. Cooled to rt and filtered off solids and concentrated filtrate. Chromatographed to yield 1.34 g (100%) of amine as a solid.

To a stirred solution of amine (1.34 g, 5.35 mmol) in EtOAc (100 mL) was added 10% aqueous NaOH (100 mL) and di-*tert*-butyl dicarbonate and stirred at rt for 3 h. The layers were separated and the organic layer was dried over Na₂SO₄ and concentrated to yield 1.76 g (94%) BOC protected amine.

To a stirred solution of amine (0.087 g, 0.248 mmol) and triethyl amine (0.069 mL, 0.496 mmol) in methylene chloride (1 mL) was added 4-fluoro-3-trifluoromethylphenyl isocyanate (0.071 mL, 0.496 mmol) and stirred at rt for 24 h. The reaction mixture was then diluted with methylene chloride, washed with aqueous NaOH, dried, concentrated and chromatographed to yield 0.97 g (63%) of urea.

To a stirred solution of urea (0.97 g, 0.174 mmol) in methylene chloride (3 mL) was added trifluoroacetic acid (0.134 mL, 1.74 mmol) and stirred under N₂ at rt for 18 h. The reaction mixture was concentrated, then diluted with methylene chloride and washed with aqueous NaOH, dried, concentrated and chromatographed to yield 0.041 g (52%) of the secondary amine.

300 MHz- ¹H NMR (CDCl₃) δ 7.86-7.80 (m, 2H), 7.68-7.52 (m, 7H), 7.44 (d, J = 9.3 Hz, 2H), 7.08 (t, J = 10.3 Hz, 1H), 3.92 (t, J = 6.0 Hz, 2H), 2.92 (t, J = 6.0 Hz, 2H), 2.55 (s, 3H), 2.12 (s, 1H)
HRMS (M+H⁺): 457.1647

### EXAMPLES 210-221

Employing preparative procedures similar to those described in Example 209, the following compounds shown below in Table V were prepared:

**Table V**

| Ex. | Structure | HRMS | 300MHZ-¹H NMR CDCl₃, |
|---|---|---|---|
| 210 | 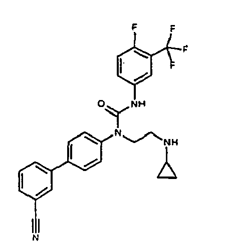 | 483.1815 | 7.88-7.81 (m, 3H), 7.69-7.51 (m, 6H), 7.44 (d, *J* = 12.0 Hz. 2H), 7.08 (t, *J* = 10.6 Hz, 1H), 3.91 (t, *J* = 6.6 Hz, 2H). 3.00 (t. *J* = 6.3 Hz, 2H), 2.26-2.22 (m, 1H), 1.97 (s, 1H), 0.52-0.37 (m. 4H) |
| 211 | 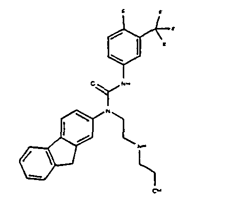 | 472.2017 | |
| 212 | 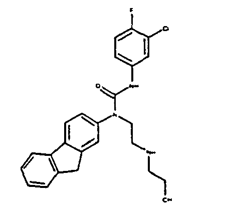 | 438.1744 | |
| 213 | 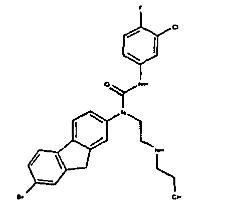 | 516.0859 | |
| 214 | 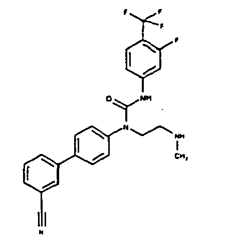 | 457.1642 | |
| 215 | 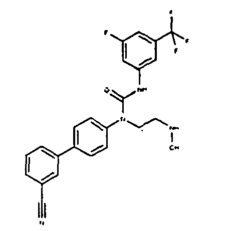 | 457.1647 | |
| 216 | 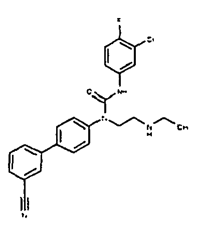 | 437.1553 | |
| 217 | 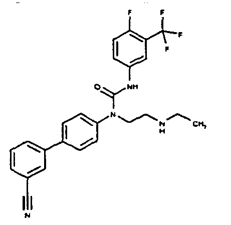 | 471.1818 | |
| 218 | 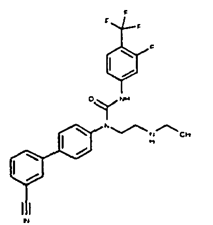 | 471.1813 | |
| 219 | 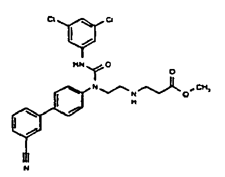 | 511.1309 | |
| 220 | 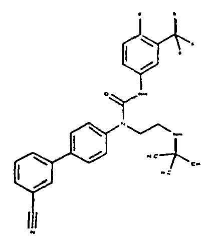 | 499.2130 | |
| 221 | 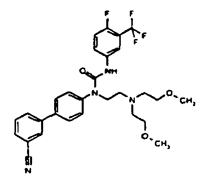 | 559.2337 | |

The following example illustrates the preparation of compounds using method 6.

### EXAMPLE 222

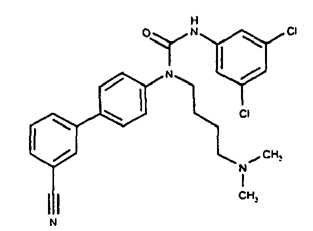

To a stirred solution of 4-bromoaniline (9.4 g, 54.82 mmol) in 250 mL toluene:ethanol:H₂O (3:1:1) was added 3-cyanophenyl boronic acid (16.11 g, 109.65 mmol), Pd(PPh₃)₄ (6.3 g, 5.48 mmol) and Na₂CO₃ (35 g, 330 mmol). The mixture was degassed with N₂, then heated to 100 °C for 24 h. The reaction mixture was concentrated then diluted with EtOAc, washed with H₂O, dried over MgSO₄, filtered, concentrated and chromatographed to yield 4.78 g (45%) of biaryl aniline.

A solution of biaryl aniline (2.2 g, 11.4 mmol) in titanium isopropoxide (4.25 mL, 14.25 mmol) was treated with γ-butyrolactol (1.0 g; 11.4 mmol) and stirred at rt for 40 h. The reaction mixture was diluted with methanol (5 mL), NaCNBH₃ (0.93 g, 14.8 mmol) was added and stirred for 24 h. H₂O (3 mL) was added and the suspension stirred for 30 min. The suspension was filtered through celite, rinsed with MeOH and concentrated. The residue was dissolved in EtOAc, dried over MgSO₄, filtered and concentrated. The crude amino-alcohol (4.0 g) was used directly for the following step.

A solution of amino-alcohol (4.0 g) in THF/MeOH/H₂O (4:1:1, 50 mL) was treated with sodium bicarbonate (1.70 g, 20.2 mmol) and BOC₂O (2.74 g, 12.5 mmol). After 18 h, the reaction mixture was concentrated, diluted with saturated aqueous NH₄Cl and extracted with EtOAc (2x). The combined organic extracts were washed with saturated aqueous NaHCO₃, brine, dried over MgSO₄, filtered, concentrated, and chromatographed to yield 2.60 g (62% over 2 steps) alcohol as an oil.

A solution of alcohol (2.60 g, 7.07 mmol) in THF (35 mL) at 0 °C was treated with carbon tetrabromide (4.64 g, 14.0 mmol) and triphenylphosphine (4.1 g, 15.6 mmol) and warmed to rt. After 30 min, the suspension was filtered through celite, concentrated and chromatographed to yield 2.76 g (91%) bromide as an oil.

A solution of bromide (300 mg, 700 mmol) in CH₃CN (7 mL) was treated with potassuim carbonate (290 mg, 2.1 mmol) and dimetyl amine (2.0 M in THF, 2.8 mL, 5.6 mmol). The reaction mixture was heated to 70 °C in a sealed tube. After 5h, the reaction mixture was cooled to rt, diluted with H₂O and extracted with CH₂Cl₂ (3x). The organic extracts were dried over MgSO₄, filtered and concentrated to provide the 250 mg (91 %) amine as an oil.

A solution of carbamate (250 mg, 630 mmol) in CH₂Cl₂ (4 mL) was treated with trifluoroacetic acid (2.0 mL). After 5 h, the reaction mixture was concentrated. The residue was diluted with saturated aqueous NaHCO₃ and extracted with CH₂Cl₂ (3x). The organic extracts were dried over MgSO₄, filtered and concentrated to yield 180 mg (97%) aniline as a solid.

A solution of aniline (77.0 mg, 0.262 mmol) in dichloroethane (2 mL) was treated with diisopropylethylamine (183 µL, 1.05 mmol) and 3,5-dichloroisocyanate (99.0 mg, 0.520 mmol). After 6 h, the reaction mixture was diluted with saturated aqueous NaHCO₃ and extracted with CH₂Cl₂ (3x). The organic extracts were dried over MgSO₄, filtered, concentrated and chromatographed to yield 66.6 mg (49%) of urea.

¹H NMR (300 MHz, CDCl₃) δ 7.89 (s, 1 H), 7.84 (d, *J* = 6.6 Hz, 1 H), 7.69 (m, 1 H), 7.68 (d, *J* = 8.7 Hz, 2 H), 7.60 (t, *J* = 7.8 Hz, 1 H), 7.41 (d, *J* = 8.7 Hz, 2 H), 7.26 (s, 2 H), 6.97 (s, 1 H), 6.30 (s, 1 H), 3.78 (t, *J* = 7.2 Hz, 2 H), 2.33 (t, *J* = 7.2 Hz, 2 H), 2.24 (s, 6 H), 1.64 - 1.54 (m, 4 H)
HRMS (M+H⁺): 481.1544

### EXAMPLES 223-229

Employing preparative procedures similar to those described in Example 222, the following compounds shown below in Table VI were prepared.

**Table VI**

| Ex. | Structure/Name | HRMS | 300MHZ-¹H NMR CDCl₃, | |
|---|---|---|---|---|
| 223 | 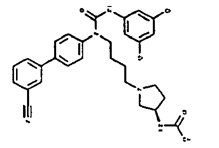 | 564.1914 | | |
| 224 | 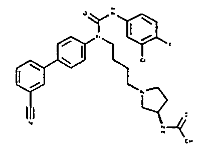 | 548.3279 | | |
| 225 | 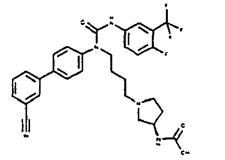 | 582.2528 | 7.90 (s, 1H), 7.86 (dt, *J* = 8.1, 1.5, 1.2 Hz, 1H), 7.71 (d, *J* = 8.4 Hz, 2 H), 7.70 (m, 1 H), 7.61 (t, *J* = 7.8 Hz, 1 H), 7.55 (d, *J* = 2.7 Hz, 1 H), 7.53 (d, *J* = 2.7 Hz, 1 H), 7.49 - 7.42 (m, 2 H). 7.07 (t, *J* = 9.3 Hz, 1 H), 6.23 (s, 1 H). 6.22 (m, 1 H), 4.45 (m. 1 H), 3.81 (t, *J* = 6.9 Hz, 2 H), 2.87 (m. 1 H). 2.71 (d, *J* = 9.9 Hz, 1 H), 2.52 - 2.45 (m, 3 H), 2.30 - 2.20 (m, 2 H), 1.93 (s, 3 H), 1.65 - 1.50 (m, 5 H) | |
| 226 | 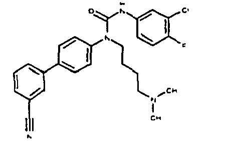 | 465.1846 | | |
| 227 | 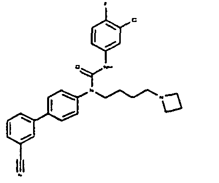 | 477.1863 | | 4 8 8 8 3 0 |
| 228 | 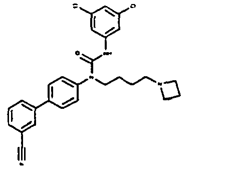 | 493.1564 | | 4 8 8 8 3 2 |
| 229 | 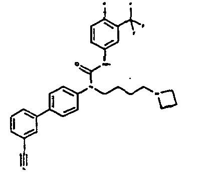 | 511.2128 | | 4 8 8 8 3 7 |

The following example illustrates the preparation of compounds using method 7.

### EXAMPLE 230

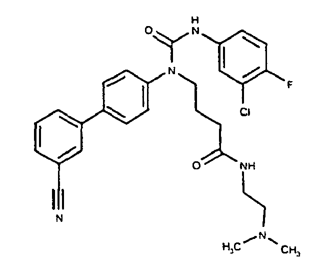

To a stirred solution of 4-bromoaniline (9.4 g, 54.82 mmol) in 250 mL toluene:ethanol:H₂O (3:1:1) was added 3-cyanophenyl boronic acid (16.11 g, 109.65 mmol), Pd(PPh₃)₄ (6.3 g, 5.48 mmol) and Na₂CO₃ (35 g, 330 mmol). The mixture was degassed with N₂, then heated to 100 °C for 24 h. The reaction mixture was concentrated then diluted with EtOAc, washed with H₂O, dried over MgSO₄, filtered, concentrated and chromatographed to yield 4.78 g (45%) of biaryl aniline.

A solution of biaryl aniline (2.50 g, 12.87 mmol) in CH₃CN (25 mL) was treated with potassium carbonate (2.67 g, 14.2 mmol) and ethyl 4-bromobutyrate (2.0 mL, 14.2 mmol) and heated to 80 °C. After 16 h, potassium carbonate (2.67 g, 14.2 mmol) and ethyl 4-bromobutyrate (2.0 mL, 14.2 mmol) were added. After 16 h further, the reaction mixture was cooled to rt, diluted with H₂O and extracted with EtOAc (2x). The organic extracts were dried over MgSO₄, filtered, concentrated and chromatographed to afford 2.10 g (<53%) of an inseparable mixture of monoalkylated along with dialkylated aniline as an oil.

A solution of impure aniline (700 mg, <2.27 mmol) in dichloroethane (20 mL) was treated with diisopropylethylamine (870 µL, 5.00 mmol) and 3-chloro, 4-fluorophenyl isocyanate (566 µL, 4.50 mmol) and heated to 80 °C. After 36 h, the reaction mixture was diluted with saturated aqueous NaHCO₃ and extracted with CH₂Cl₂ (3x). The organic extracts were dried over MgSO₄, filtered, concentrated and chromatographed to yield 740 mg (68%) of urea as a solid.

A solution of ester (740 mg, 1.54 mmol) in EtOH/THF (2:1, 12 mL) was treated with a solution of sodium hydroxide (1.0 N, 7 mL). After 1h, the reaction mixture was concentrated, diluted with 1 N sodium hydroxide and extracted with diethyl ether (2x). The aqueous phase was acidified to pH = 2 with 6 N HCl, and extracted with EtOAc (2x). The combined ethyl acetate layers were washed with water, dried over MgSO₄, filtered and concentrated to give 680 mg (97%) acid as a solid.

A solution of acid (100 mg, 0.221 mmol) in CH₂Cl₂ (2 mL) was treated with triethylamine (350 µL, 0.553 mmol), BOP reagent (117 mg, 0.265 mmol) and *N, N* - dimethylethylenediamine (36 µL, 0.33 mmol). After 24 h, the reaction mixture was diluted with saturated aqueous NH₄Cl and extracted with EtOAc (2x). The combined organic extracts were washed with saturated aqueous NaHCO₃, brine, dried over MgSO₄, filtered, concentrated and chromatographed to yield 89.7 mg (71%) of amide.

¹H NMR (300 MHz, CDCl₃) δ 7.86 (s, 1 H), 7.82 - 7.81 (m, 1 H), 7.68 - 7.58 (m, 5 H), 7.45 (d, *J* = 8.4 Hz, 2 H), 7.25 (m, 1 H), 7.02 (t, *J* = 8.7 Hz, 1 H), 6.62 (s, 1 H), 3.85 (t, *J* = 6.9 Hz, 2 H), 3.41 (q, *J* = 6.0 Hz, 2 H), 2.62 - 2.60 (m, 2 H), 2.34 (s, 6 H), 1.93 - 1.88 (m, 2 H)
HRMS (M+H⁺): 522.2065

### EXAMPLES 231-236

Employing preparative procedures similar to those described in Example 230, the following compounds shown below in Table VII were prepared.

**Example VII**

| Ex. | Structure/Name | HRMS | 300MHZ-¹H NMR CDCl₃, |
|---|---|---|---|
| 231 | 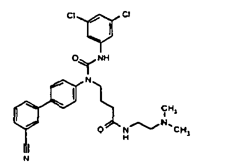 | 538.1766 | |
| 232 | 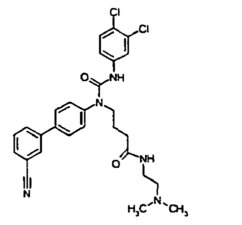 | 538.1784 | |
| 233 | 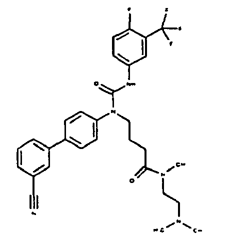 | 570.2429 | |
| 234 | 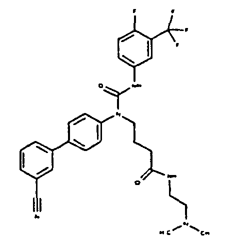 | 556.2328 | |
| 235 | 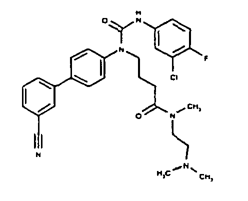 | 536.2339 | |
| 236 | 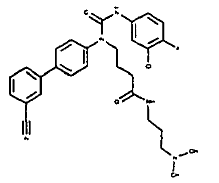 | 536.2339 | |

The following example illustrates the preparation of the compound using method 8.

### EXAMPLE 237

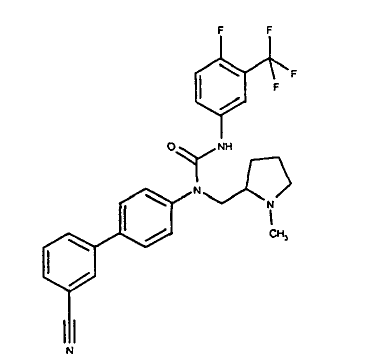

To a stirred solution of 4-bromoaniline (9.4 g, 54.82 mmol) in 250 mL toluene:ethanol:H₂O (3:1:1) was added 3-cyanophenyl boronic acid (16.11 g, 109.65 mmol), Pd(PPh₃)₄ (6.3 g, 5.48 mmol) and Na₂CO₃ (35 g, 330 mmol). The mixture was degassed with N₂, then heated to 100 °C for 24 h. The reaction mixture was concentrated then diluted with EtOAc, washed with H₂O, dried over MgSO₄, filtered, concentrated and chromatographed to yield 4.78 g (45%) of biaryl aniline.

To a stirred solution of biaryl aniline (0.53 g, 2.73 mmol) and N-(tert-butoxycarbonyl)-L-prolinal (0.54 mL, 2.86 mmol) in methylene chloride (4mL) was added titanium isopropoxide (0.98 mL, 3.28 mmol) and stirred and rt for 24 h. The reaction mixture was cooled to 0°C and sodium cyanoborohydride (0.51 g, 8.19 mmol) was added in methanol (2 mL) drop-wise. The reaction was stirred 0°C for 20 mins and at rt for 5 h. The reaction mixture was diluted with EtOAc:water, 3:1, (12 mL) then filtered through Celite. The resulting filtrate was then washed with brine, dried over Na₂SO₄, concentrated and chromatographed to yield 0.656 g (64%) of amine.

To a stirred solution of amine (0.655 g, 1.737 mmol) and diisopropylethyl amine (1.51 mL, 8.687 mmol) in dichloroethane (3 mL) was added 4-fluoro-3-trifluoromethylphenyl isocyanate (1.24 mL, 8.687 mmol) and stirred at 50°C for 24 h. The reaction mixture was cooled to rt then diluted with methylene chloride, washed with H₂O, aqueous NaOH, dried over Na₂SO₄, concentrated and chromatographed to yield 1.00 g (99%) of urea.

To a stirred solution of urea (1.00 g, 1.73 mmol) in methylene chloride (20 mL) was added trifluoroacetic acid (2.6 mL, 34.74 mmol) and stirred under N₂ at rt for 3 h. The reaction mixture was concentrated, then diluted with methylene chloride and washed with aqueous NaOH, dried and concentrated to yield 0.83 g (100%) of secondary amine.

To a stirred solution of amine (0.071 g, 0.147 mmol) in dichloroethane (1 mL) was added a 37% aqueous solution of formaldehyde (0.033 mL, 0.442 mmol), acetic acid (0.017 mL, 0.294 mmol), and sodium triacetoxyborohydride (0.063 g, 0.294 mmol) and stirred at rt for 24 h. The reaction mixture was quenched with aqueous NaOH and extracted with methylene chloride. The combined extracts were dried over Na₂SO₄, concentrated and chromatographed to yield 0.033 g (45%) amine.

¹H NMR (300 MHz, CDCl₃) δ 7.85-7.79 (m, 2H), 7.69-7.47 (m, 6H), 7.39 (d, J = 9.3 Hz, 2H), 7.10 (t, J = 10.6 Hz, 1H), 3.97-3.93 (m, 2H), 3.28-3.27 (m, 1H), 2.87 (m, 1H), 2.60 (s, 3H), 2.57-2.51 (m, 1H), 1.98-1.87 (m, 3H), 1.82-1.75 (m, 1H)
HRMS (M+H⁺): 497.1974

### EXAMPLES 238-246

Employing preparative procedures similar to those described in Example 237, compounds were prepared whose structures are set forth in Table VIII as follows:

**Table VIII**

| Ex. | Structure/Name | HRMS | 300MHZ-¹H NMR CDCl₃, |
|---|---|---|---|
| 238 | 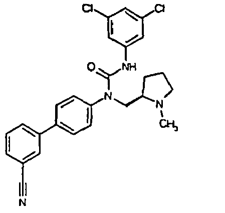 | 479.1409 | |
| 239 | 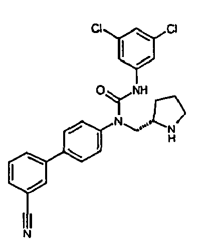 | 465.1246 | |
| 240 | 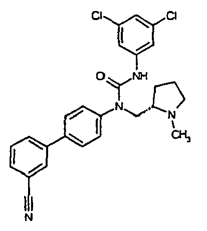 | 479.1409 | |
| 241 | 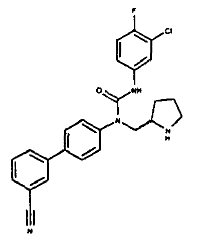 | 449.1552 | |
| 242 | 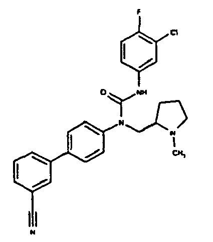 | 463.1704 | |
| 243 | 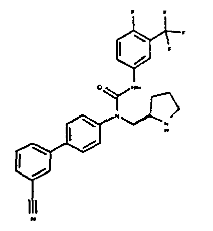 | 483.1800 | 7.85-7.79 (m, 2H), 7.70-7.51 (m, 7H), 7.44 (d, *J* = 9.3 Hz, 2H), 7.09 (t, *J* = 10.6 Hz, 1H), 4.00-3.91 (m, 1H), 3.75-3.65 (m, 2H), 3.22-2.98 (m, 3H), 1.99-1.92 (m, 2H), 1.80-1.76 (m, 1H), 1.55-1.51 (m, 1H) |
| 244 | 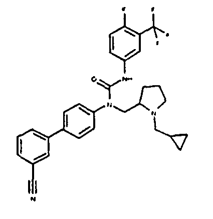 | 537.2287 | |
| 245 | 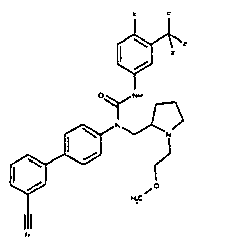 | 541.2239 | |
| 246 | 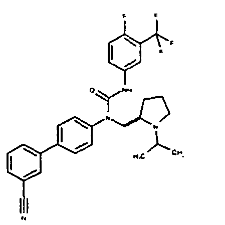 | 525.2278 | |

## Claims

1. A compound of the formula: 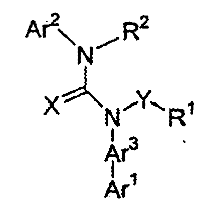 or a pharmaceutically acceptable addition salt and/or hydrate thereof, or where applicable, a geometric or optical isomer or racemic mixture thereof;
wherein
Ar¹ is an aryl or heteroaryl group,
Ar² is an aryl, heteroaryl or aralkyl group,
Ar³ is an arylene or heteroarylene group or Ar¹ and Ar³ together form a fluorene, substituted fluorene or fluorenone group with the proviso that Ar³ must be arylene;
said Ar¹, Ar² and Ar³ groups possessing 0 to 3 substituents independently selected from the group consisting of -(C₁-C₆)alkyl, -(C₃-C₇)cycloalkyl, halo, -CN, -(C₁-C₆)alkoxy, -CF₃, -OCF₃, -CONH₂, -CONH(C₁-C₆)alkyl, -CON(C₁-C₆)alkyl (C₁-C₆)alkyl, - NH₂, -NH C(O)(C₁-C₆)alkyl, -NHSO₂(C₁-C₆)alkyl, -S(C₁-C₆)alkyl, -SO(C₁-C₆)alkyl, - SO₂(C₁-C₆)alkyl, methytenedioxy and NO₂;
X is O, S or N-CN;
Y is a single bond or a -(C₁-C₄)alkylene- group;
R¹ is thiazole, aryl or heteroaryl; or 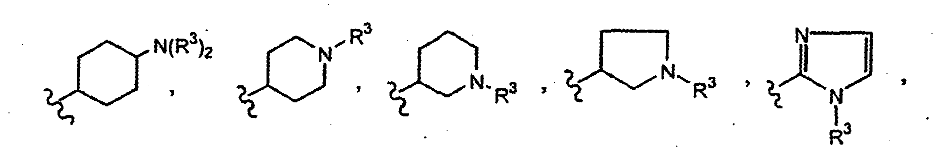 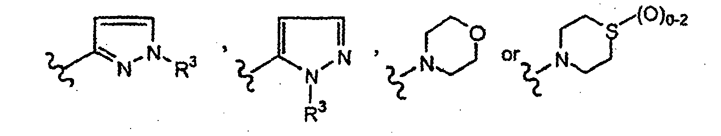 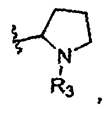 or 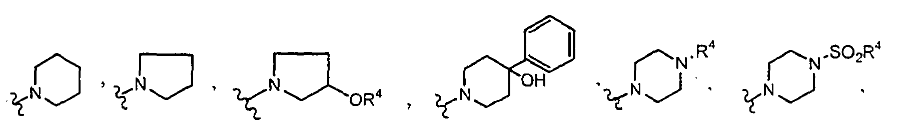 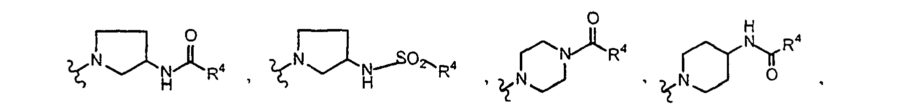 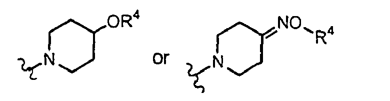 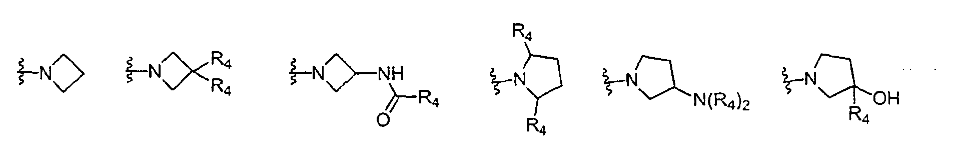 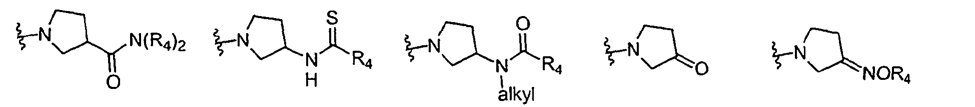 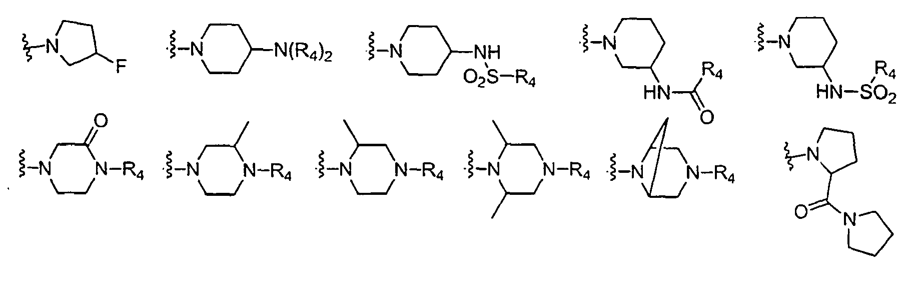 ,or
R¹ is -N(R⁵)₂, -NHC(O)(C₂-C₃)alkylene N(R⁵)₂; -C(O)NH(C₂-C₃)alkylene N(R⁵)₂: C(O)N(Me)(C₂-C₃)alkyleneN(R⁵)_{2,} -C(OH)(C₁-C₂)alkyleneN(R⁵)₂, -N(Me)(C₂-C₃)alkyleneN(R⁵)_{2,} -NH(C₂-C₃)alkyleneC(O)R⁵, -N(Me)(C₂-C₃)alkyleneN(Me)SO₂(R⁵) or -N(Me)(C₂-C₃)alkyleneC(O)N(R⁵)₂;
R² is H or -(C₁-C₆)alkyl.
R³ is independently H, or nonsubstituted or halosubstituted -(C₁-C₆)alkyl, -(C₃-C₇)cycloalkyl, -(C₃-C₇)cycloalkyl(C₁-C₆)alkyl, -(C₁-C₆)alkoxy, -(C₁-C₆)alkoxy (C₁-C₆)alkylene, aryl, -aralkyl or -heteroaralkyl; or
R⁴ is H, nonsubstituted or halosubstituted -(C₁-C₆)alkyl, -NH(C₁-C₆)alkyl, -NHaryl, aryl; or alkoxy or hydroxy substituted alkyl, and
R⁵ is independently H, or nonsubstituted or halosubstituted -(C₁-C₆)alkyl, -(C₃-C₇)cycloalkyl, -(C₃-C₇)cycloalkyl(C₁-C₆)alkyl, aryl, -aralkyl, -heteroaralkyl, -(C₁-C₆)alkoxy or (C₁-C₆)alkylene(C₁-C₆)alkoxy.

2. A compound as defined in Claim 1;
or a pharmaceutically acceptable addition salt and or hydrate thereof, or where applicable, a geometric or optical isomer or racemic mixture thereof;
wherein
Ar¹ and Ar² are independently phenyl or pyridyl,
Ar³ is 1, 4-arylene,
R¹ is 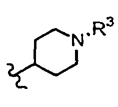 in which R³ is -(C₁-C₆)alkyl, -(C₃-C₇)cycloalkylmethyl, (C₁-C₆)alkoxy- or (C₁-C₆)alkoxy(C₁-C₆)alkylene-,
R² is H,
X is O; and
Y is a single bond or -(C₁-C₃)alkylene.

3. A compound as defined in Claim 1
Or a pharmaceutically acceptable addition salt and/or hydrate thereof, or where applicable, a geometric or optical isomer or racemic mixture thereof;
wherein
Ar¹ and Ar² are independently phenyl or pyridyl,
Ar³ is 1,4-arylene,
R¹ is -N(R⁵)₂ or -C(O)NH(C₂-C₃)alkylene N(R⁵)₂ in which each R⁵ is independently H, -(C₁-C₆)alkyl, -ar(C₁-C₆)alkyl, heteroaryl, heteroarylalkyl, halo-substituted -(C₁-C₆)alkyl, -(C₃-C₇)cycloalkyl,
X is O; and
Y is -(C₂-C₃)alkylene.

4. A compound as defined in Claim 1
Or a pharmaceutically acceptable addition salt and/or hydrate thereof, or where applicable, a geometric or optical isomer or racemic mixture thereof;
wherein
Ar¹ and Ar² are independently phenyl or pyridyl,
Ar³ is 1,4-arylene,
R¹ is selected from 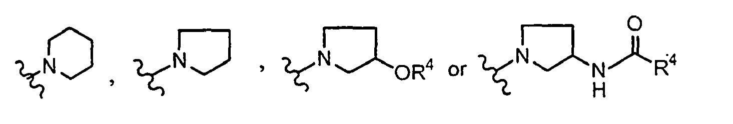
X is O; and
Y is -(C₂-C₃)alkylene.

5. A compound as defined in Claim 2
or a pharmaceutically acceptable addition salt and/or hydrate thereof, or where applicable, a geometric or optical isomer or racemic mixture thereof;
wherein
Ar¹ is 3-substituted phenyl or pyridyl,
Ar² is halo-substituted or CF₃-substituted phenyl or pyridyl and
R³ is methyl, ethyl, propyl, -CH₂CH₂CF₃, cyclopentyl, cyclopropylmethyl or 3-methoxyethyl.

6. A compound as defined in Claim 5 wherein the 3-substituent on the phenyl or pyridyl is - CN, -OCF₃ or chloro.

7. A compound as defined in Claim 3 wherein Ar¹ is 3-substituted phenyl or pyridyl, Ar² is halo-substituted or CF₃-substituted phenyl or pyridyl and R⁵ is methyl, ethyl, propyl, -CH₂CH₂CF₃, cyclopentyl, cyclopropylmethyl or 3-methoxyethyl.

8. A compound as defined in Claim 7 wherein the 3- substituent on the phenyl or pyridyl is -CN, -OCF₃ or chloro.

9. A compound as defined in Claim 4 wherein Ar¹ is 3-substituted phenyl or pyridyl, Ar² is halo-substituted or CF₃-substituted phenyl or pyridyl and R⁵ is methyl, ethyl, propyl, -CH₂CH₂CF₃, cyclopentyl, cyclopropylmethyl or 3-methoxyethyl.

10. A compound as defined in Claim 9 wherein the 3- substituent on the phenyl or pyridyl is -CN, -OCF₃ or chloro.

11. A compound as defined in Claim 1 selected from the group consisting of

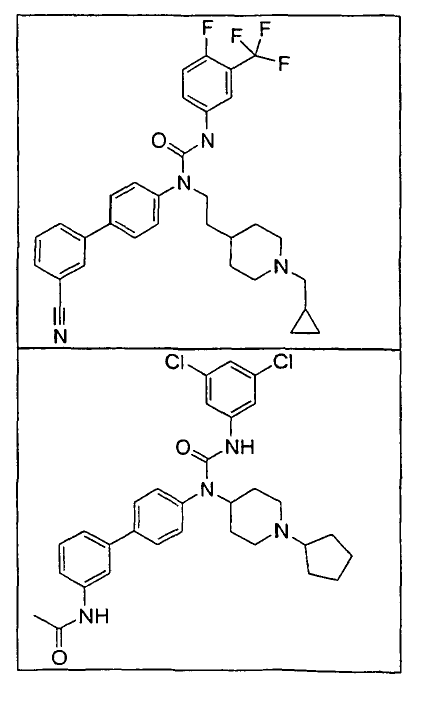

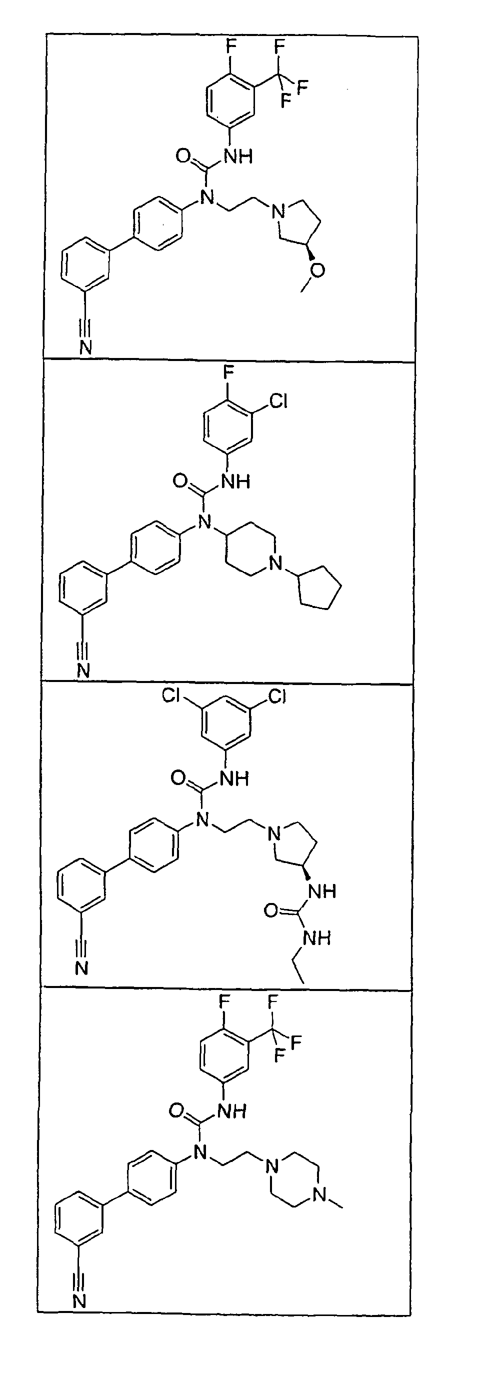

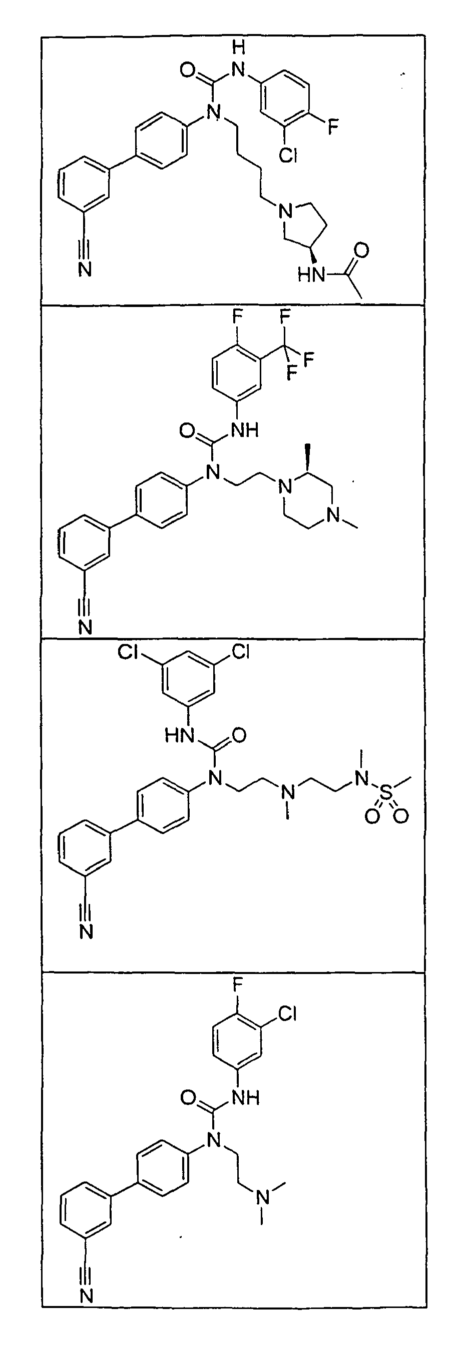

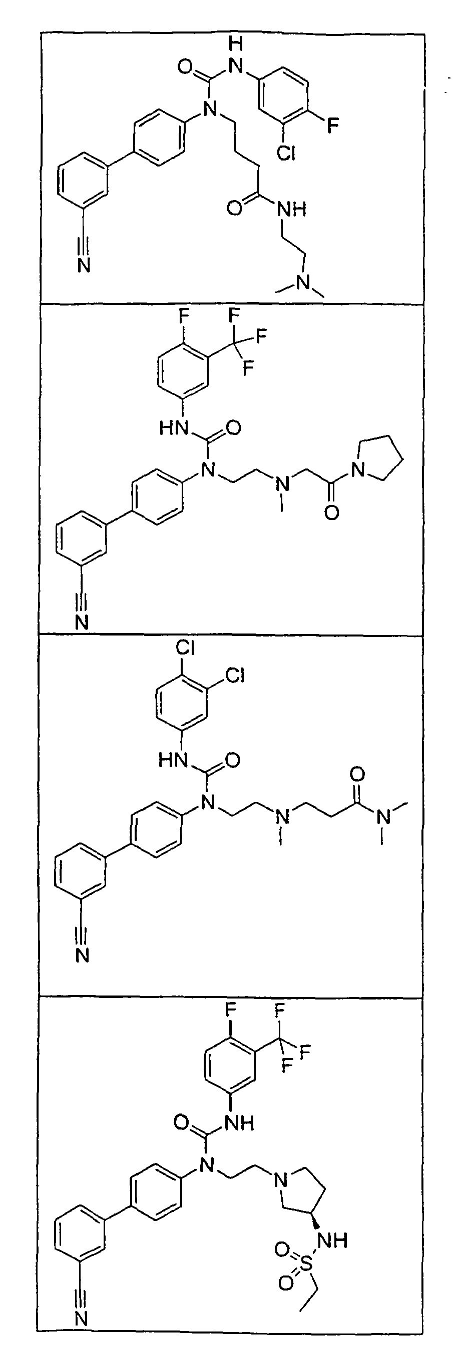

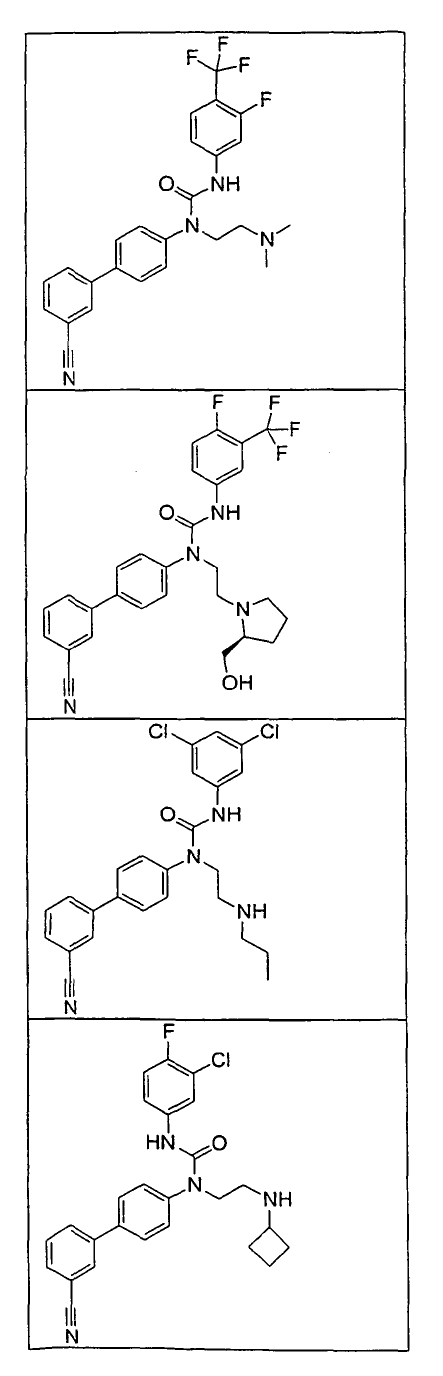

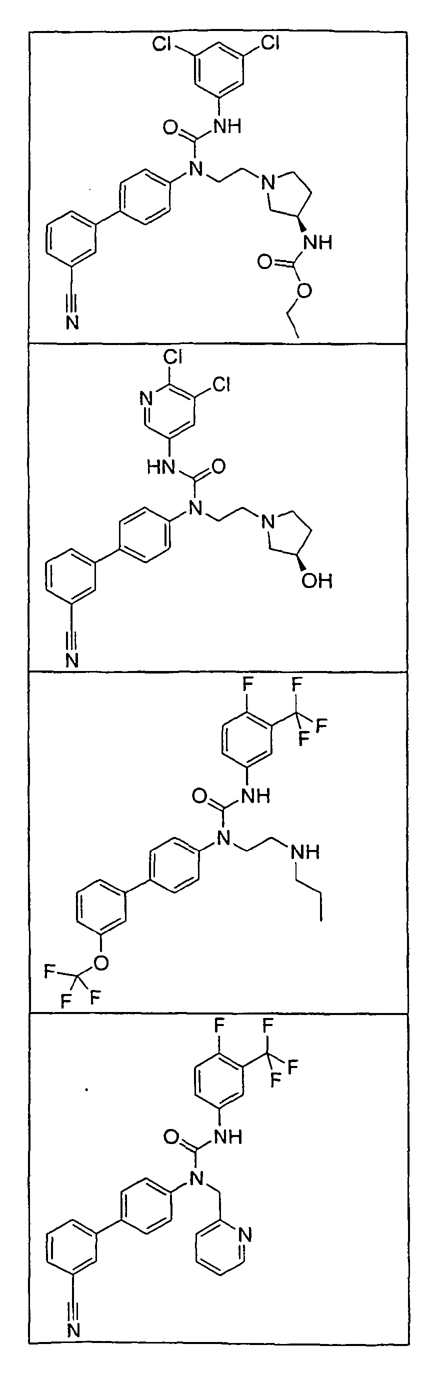

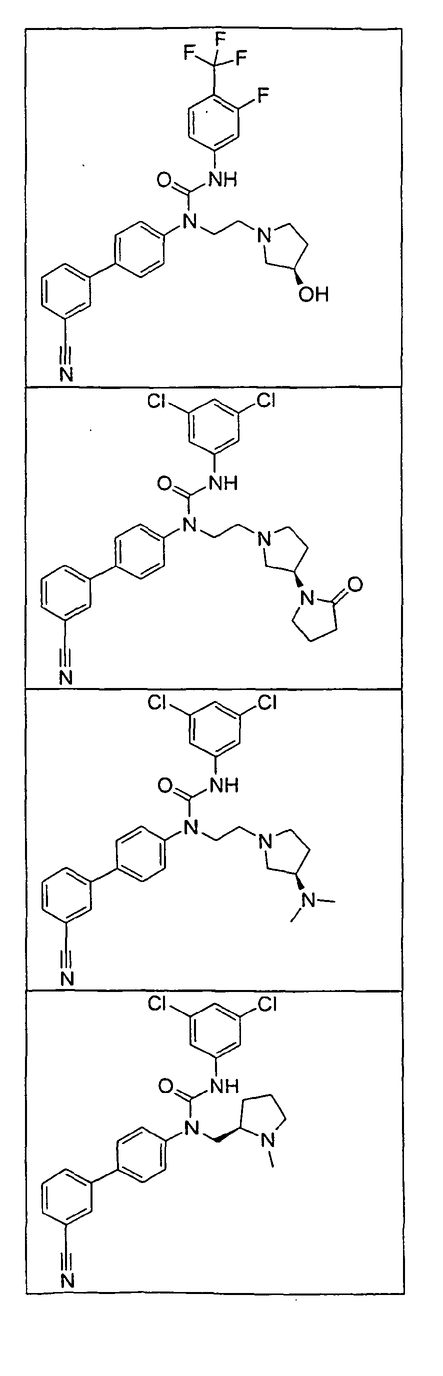

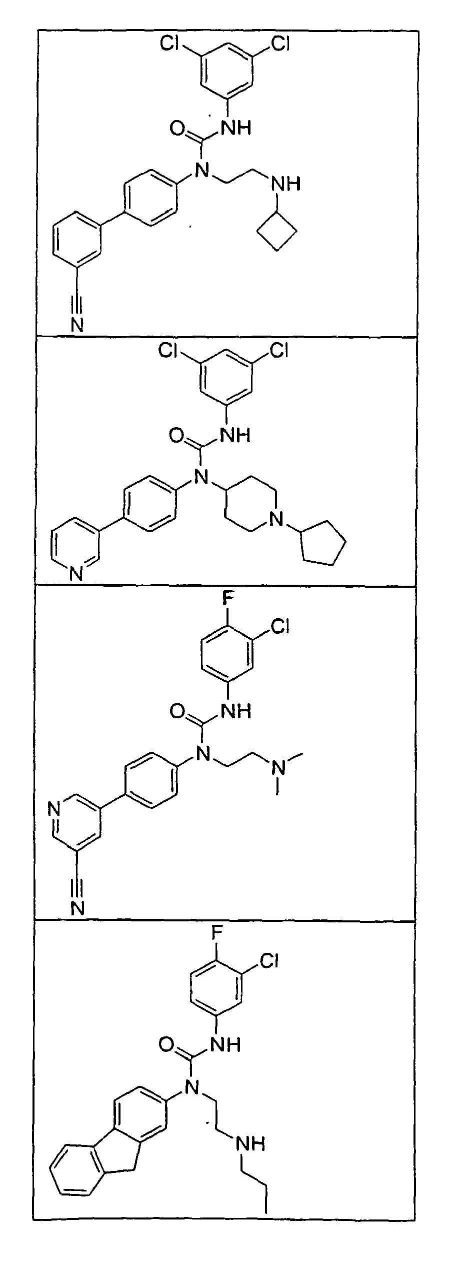

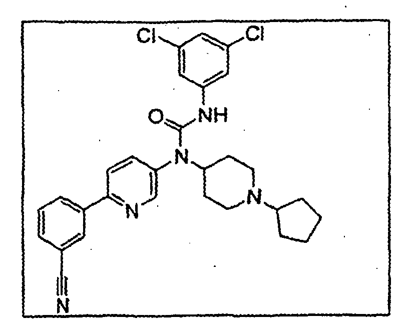

12. A pharmaceutical composition comprising a therapeutically effective amount of a compound of claim 1 in combination with a pharmaceutically acceptable carrier.

13. Use of a compound of claim 1 for the manufacture of a medicament for treating a metabolic disorder, eating disorder or diabetes.

14. A pharmaceutical composition which comprises an effective amount of a compound as defined in claim 1 and a pharmaceutically acceptable carrier thereof.

15. Use of a compound of claim 1 or a pro-drug thereof or a pharmaceutically acceptable salt of said compound or of said pro-drug, for the manufacture of a medicament for treating an eating disorder.

16. The use of claim 15 wherein said eating disorder is hyperphagia.

17. The use of claim 13 wherein said metabolic disorder is obesity.

18. Use of a compound of claim 1 or a pro-drug thereof or a pharmaceutically acceptable salt of said compound or of said pro-drug, for the manufacture of a medicament for treating a disorder associated with obesity.

19. The use of claim 18 wherein said disorder associated with obesity is type II diabetes, insulin resistance, hyperlipidemia or hypertension.

20. A pharmaceutical composition which comprises a therapeutically effective amount of a composition comprising
a first compound, said first compound being a compound of claim 1, a pro-drug thereof, or a pharmaceutically acceptable salt of said compound or of said pro-drug;
a second compound, said second compound being an antiobesity and/or anorectic agent such as a β₃ agonist, a thryomimetic agent, an anorectic agent or an NPY antagonist; and
a pharmaceutically acceptable carrier thereof.

21. Use of a first compound, said first compound being a compound of claim 1, a pro-drug thereof, or a pharmaceutically acceptable salt of said compound or of said pro-drug, for the manufacture of a medicament for treating an eating disorder, wherein the treatment comprises the administation of the medicament and a second compound, said second compound being an antiobesity and/or anorectic agent such as a β₃ agonist, a thryomimetic agent, an anorectic agent or an NPY antagonist.

22. A pharmaceutical composition which comprises a therapeutically effective amount of a composition comprising
a first compound, said first compound being a compound of claim 1, a pro-drug thereof, or a pharmaceutically acceptable salt of said compound or of said pro-drug;
a second compound, said second compound being an aldose reductase inhibitor, a glycogen phosphorylase inhibitor, a sorbitol dehydrogenase inhibitor, a protein tyrosine phosphatase 1B inhibitor, a dipeptidyl protease inhibitor, insulin (including orally bioavailable insulin preparations), an insulin mimetic, metformin, acarbose, a PPAR-gamma ligand such as troglitazone, rosaglitazone, pioglitazone, or GW-1929, a sulfonylurea, glipazide, glyburide, or chlorpropamide; and a pharmaceutically acceptable carrier therefor.

23. A pharmaceutical composition made by combining the compound as defined in claim 1 and a pharmaceutically acceptable carrier therefor.

24. A process for making a pharmaceutical composition comprising combining a compound as defined in claim 1 and a pharmaceutically acceptable carrier.

25. A compound as defined in any one of claims 1 to 11 for use as a medicament.

## Patentansprüche

1. Verbindung gemäss der Formel: 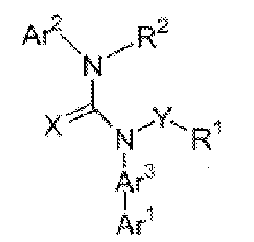 oder ein pharmazeutisch verträgliches Additionssalz und/oder Hydrat davon, oder wo anwendbar, eine geometrische oder optische Isomer oder razemische Mischung davon;
wobei
Ar¹ eine Aryl- oder Heteroaryl-Gruppe ist,
Ar² eine Aryl-, Heteroaryl oder Aralkyl-Gruppe ist,
Ar³ eine Arylen- oder Heteroarylen-Gruppe ist oder Ar¹ und Ar³ zusammen eine Fluoren-, substituierte Fluoren- oder Fluorenon-Gruppe ausbilden, mit dem Proviso, dass Ar³ Arylen sein muss;
besagte Ar¹, Ar² und Ar³-Gruppen 0 bis 3 Substituenten besitzen, unabhängig ausgewählt aus der Gruppe bestehend aus -(C₁-C₆) Alkyl, -(C₃-C₇) Cycloalkyl, Halo, -CN, -(C₁-C₆) Alkoxy, -CF₃, - OCF₃, -CONH₂, -CONH(C₁-C₆) Alkyl, -CON(C₁-C₆) Alkyl (C₁-C₆) alkyl, NH₂, -NH C(O) (C₁-C₆) Alkyl, -NHSO₂(C₁-C₆) Aryl, -S(C₁-C₆) Aryl, -SO(C₁-C₆) Alkyl, -SO₂(C₁-C₆) Allyl, Methylendioxy und NO₂;
X O, S oder N-CN ist;
Y eine Einfachbindung oder eine -(C₁-C₄) Alkylen-Gruppe ist;
R¹ Thiazol, Aryl oder Heteroaryl ist; oder 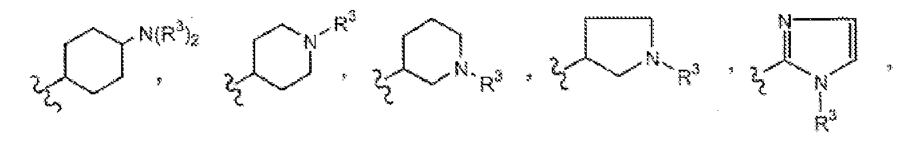 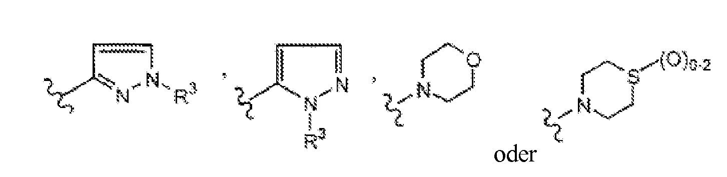 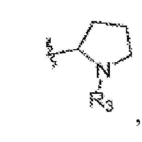 oder 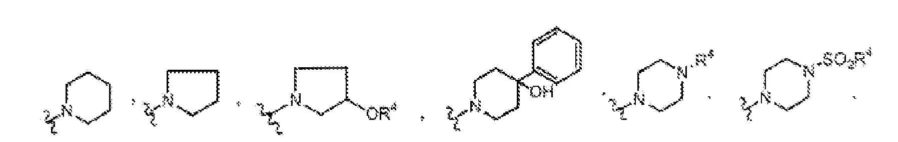 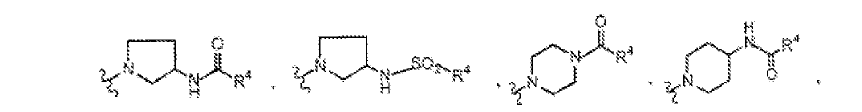 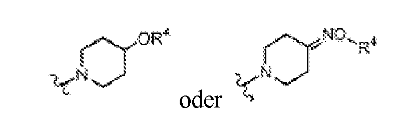 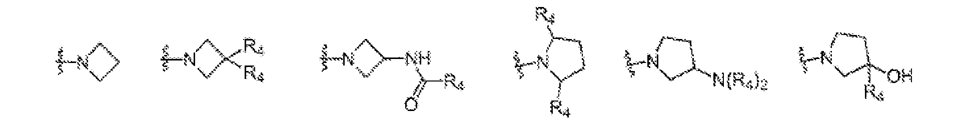 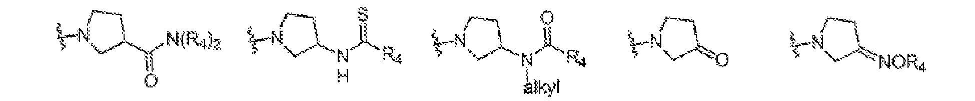 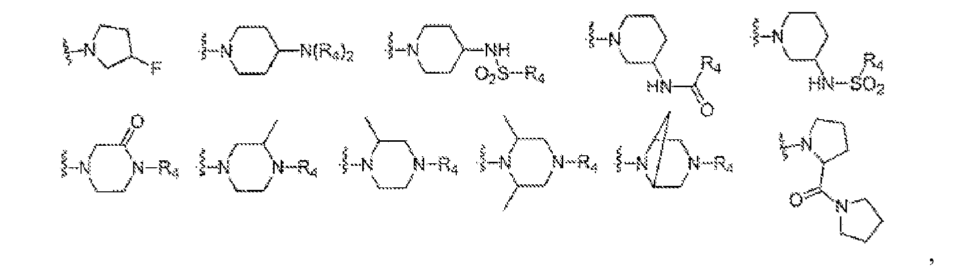
oder
R¹ -N(R⁵)₂, -NHC(O)(C₂-C₃) Alkyle N(R⁵)₂; -C(O)(C₁-C₆)NH(C₂-C₃) Alkyle N(R⁵)₂ ist; C(O)N(Me) (C₂-C₃) Alkylen N(R⁵)₂, -C(OH)(C₁-C₂) Alkylen N(R⁵)₂, -N(Me)(C₂-C₃) Alkylen N(R⁵)₂, -NH(C₂-C₃) Alkylen C(O)R⁵, -N(Me)(C₂-C₃) Alkylen N(Me)SO₂ (R⁵) oder -N(Me)(C₂-C₃) Alkylen C(O)N(R⁵)₂ ist;
R² H oder -(C₁-C₆) Alkyl ist;
R3 unabhängig H, oder nicht-substituiertes oder halosubstituiertes -(C₁-C₆) Alkyl, -(C₃-C₇) Cycloalkyl, -(C₃-C₇) Cycloalkyl (C₁-C₆) alkyl, -(C₁-C₆) Alkoxy, -(C₁-C₆) Alkoxy (C₁-C₆) alkylen, Aryl, -Aralkyl oder -Heteroaralkyl ist; oder
R⁴ H, nicht-substituiertes oder halosubstituiertes -(C₁-C₆) Alkyl, -NH (C₁-C₆) Alkyl, -NH Aryl, Aryl; oder Alkoxy- oder Hydroxy-substituiertes Alkyl ist, und
R⁵ unabhängig H, oder nicht-substituiertes oder halosubstituiertes -(C₁-C₆) Alkyl, -(C₃-C₇) Cycloalkyl, -(C₃-C₇) Cycloalkyl (C₁-C₆) alkyl, Aryl, -Aralkyl, -Heteroalkyl, -(C₁-C₆) Alkoxy oder (C₁-C₆) Alkylen (C₁-C₆) alkoxy ist.

2. Verbindung gemäss Anspruch 1; oder ein pharmazeutisch verträgliches Additionssalz und/oder Hydrat davon, oder wo anwendbar, eine geometrische oder optische Isomer oder razemische Mischung davon;
wobei
Ar¹ und Ar² unabhängig Phenyl oder Pyridyl sind,
Ar³ 1,4-Arylen ist,
R¹ 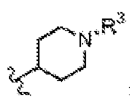 ist, wobei R³ -(C₁-C₆) Alkyl, -(C₃-C₇) Cycloalkylmethyl, (C₁-C₆) Alkoxy- oder (C₁-C₆) Alkoxy (C₁-C₆) alkylen- ist,
R² H ist,
X O ist; und
Y eine Einfachbindung oder -(C₁-C₃) Alkylen ist.

3. Verbindung gemäss Anspruch 1
oder ein pharmazeutisch verträgliches Additionssalz und/oder Hydrat davon, oder wo anwendbar, eine geometrische oder optische Isomer oder razemische Mischung davon;
wobei
Ar¹ und Ar² unabhängig Phenyl oder Pyridyl sind,
Ar³ 1,4-Arylen ist,
R1 -N(R⁵)2 oder -C(O)NH(C₂-C₃) Alkylen N(R⁵)₂ ist, wobei jedes R⁵ unabhängig H, -(C₁-C₆) Alkyl, -Ar(C₁-C₆)alkyl, Heteroaryl, Heteroarylalkyl, Halo-substituiertes -(C₁-C₆) Alkyl, -(C₃-C₇) Cycloalkyl ist,
X O ist; und
Y -(C₂-C₃) Alkylen ist.

4. Verbindung gemäss Anspruch 1
oder ein pharmazeutisch verträgliches Additionssalz und/oder Hydrat davon, oder wo anwendbar, eine geometrische oder optische Isomer oder razemische Mischung davon;
wobei
Ar¹ und Ar² unabhängig Phenyl oder Pyridyl sind,
Ar³ 1,4-Arylen ist,
R¹ ausgewählt ist aus 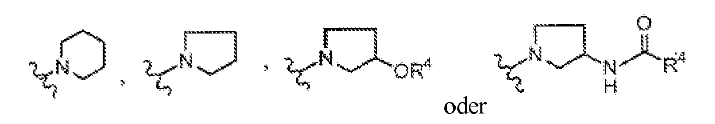
X O ist; und
Y -(C₂-C₃) Alkylen ist.

5. Verbindung gemäss Anspruch 2
oder ein pharmazeutisch verträgliches Additionssalz und/oder Hydrat davon, oder wo anwendbar, eine geometrische oder optische Isomer oder razemische Mischung davon;
wobei
Ar¹ 3-substituiertes Phenyl oder Pyridyl ist,
Ar² Halo-substituiertes oder CF₃-substituiertes Phenyl oder Pyridyl ist, und
R³ Methyl, Ethyl, Propyl, -CH₂CH₂CF3, Cyclopentyl, Cyclopropylmethyl oder 3-Methoxyethyl ist.

6. Verbindung gemäss Anspruch 5, wobei der 3-Substituent auf dem Phenyl oder Pyridyl -CN, -OCF₃ oder Chlor ist.

7. Verbindung gemäss Anspruch 3, wobei Ar¹ 3-substituiertes Phenyl oder Pyridyl ist, Ar² Halo-substituiertes oder CF₃-substituiertes Phenyl oder Pyridyl ist und R⁵ Methyl, Ethyl, Propyl, - CH₂CH₂CF₃, Cyclopentyl, Cyclopropylmethyl oder 3-Methoxyethyl ist.

8. Verbindung gemäss Anspruch 7, wobei der 3-Substituent auf dem Phenyl oder Pyridyl-CN, -OCF₃ oder Chlor ist.

9. Verbindung gemäss Anspruch 4, wobei Ar¹ 3-substituiertes Phenyl oder Pyridyl ist, Ar² Halo-substituiertes oder CF₃-substituiertes Phenyl oder Pyridyl ist und R⁵ Methyl, Ethyl, Propyl, - CH₂CH₂CF₃, Cyclopentyl, Cyclopropylmethyl oder 3-Methoxyethyl ist.

10. Verbindung gemäss Anspruch 9, wobei der 3-Substituent auf dem Phenyl oder Pyridyl-CN, -OCF₃ oder Chlor ist.

11. Verbindung gemäss Anspruch 1, ausgewählt aus der Gruppe bestehend aus

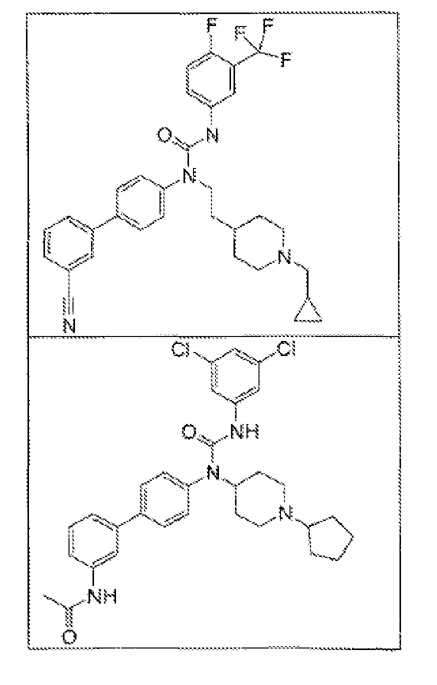

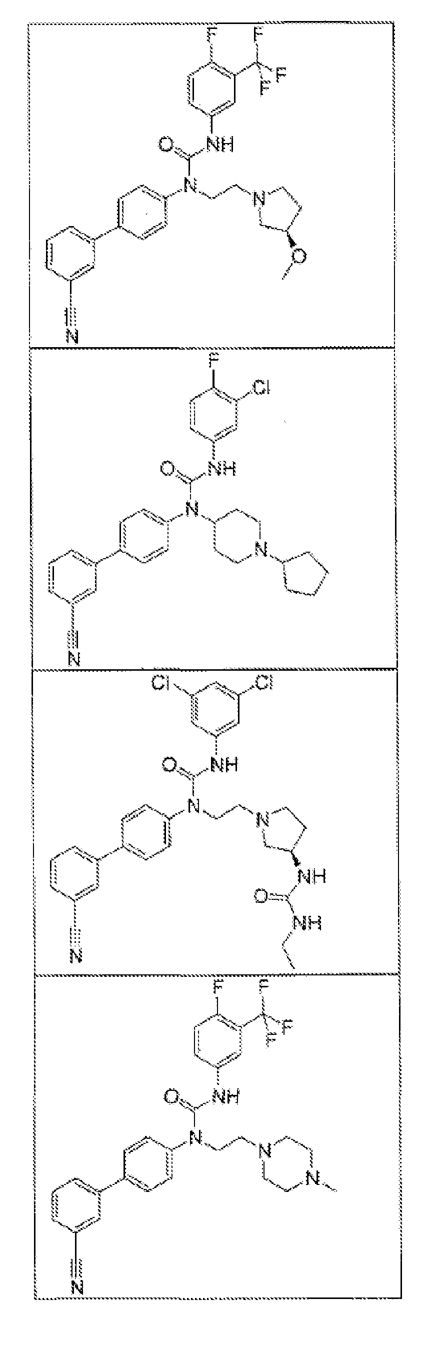

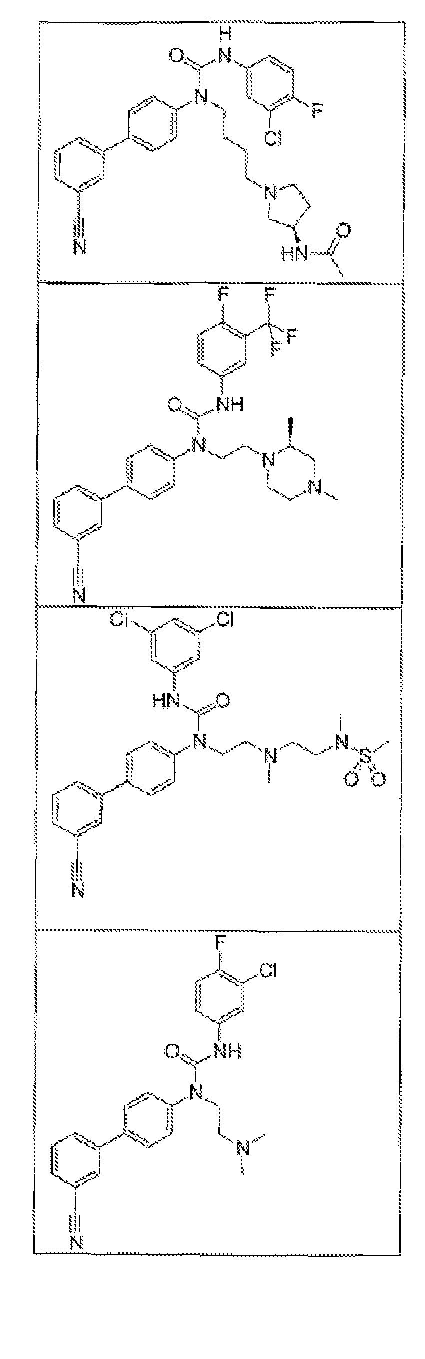

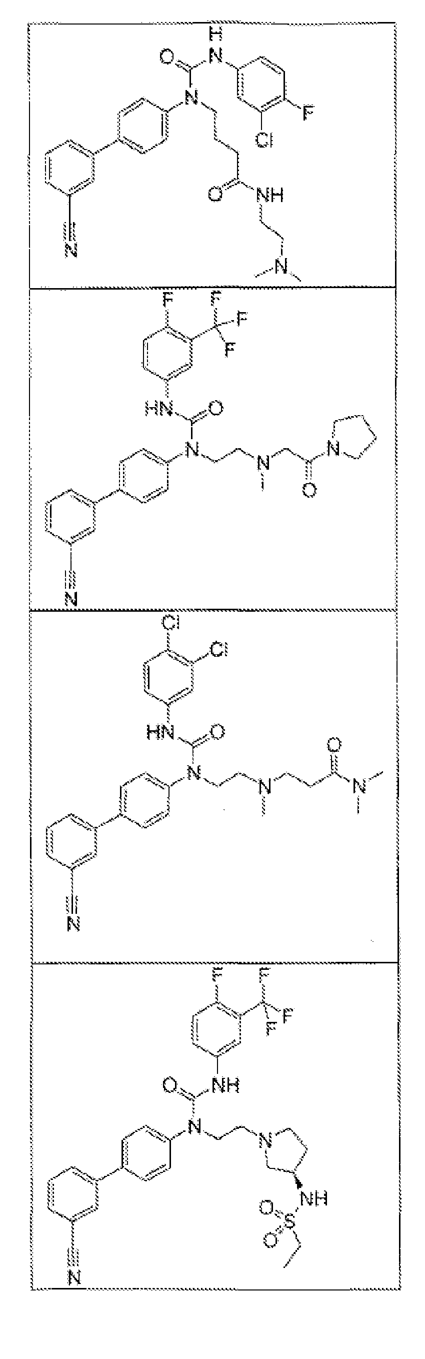

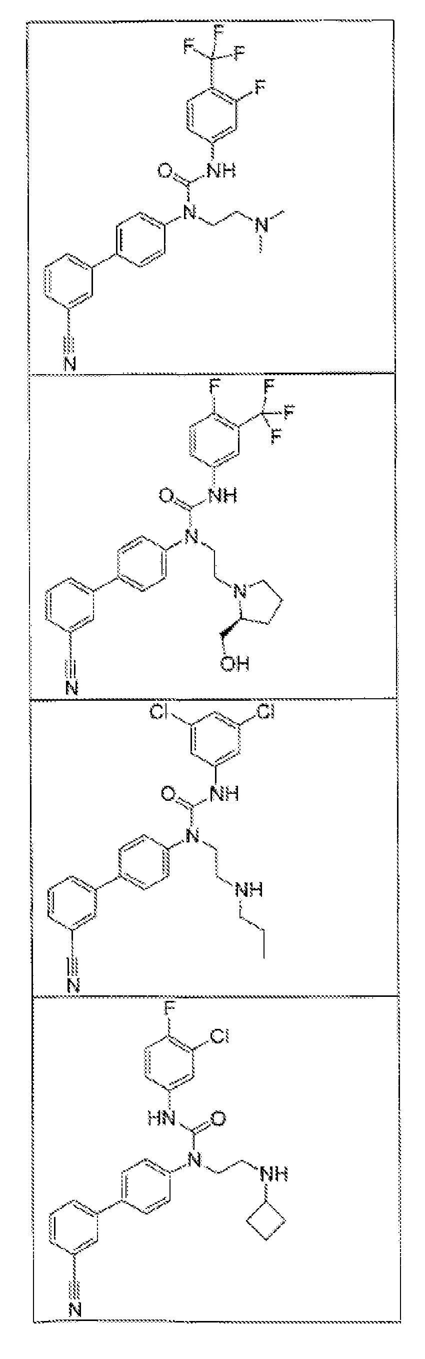

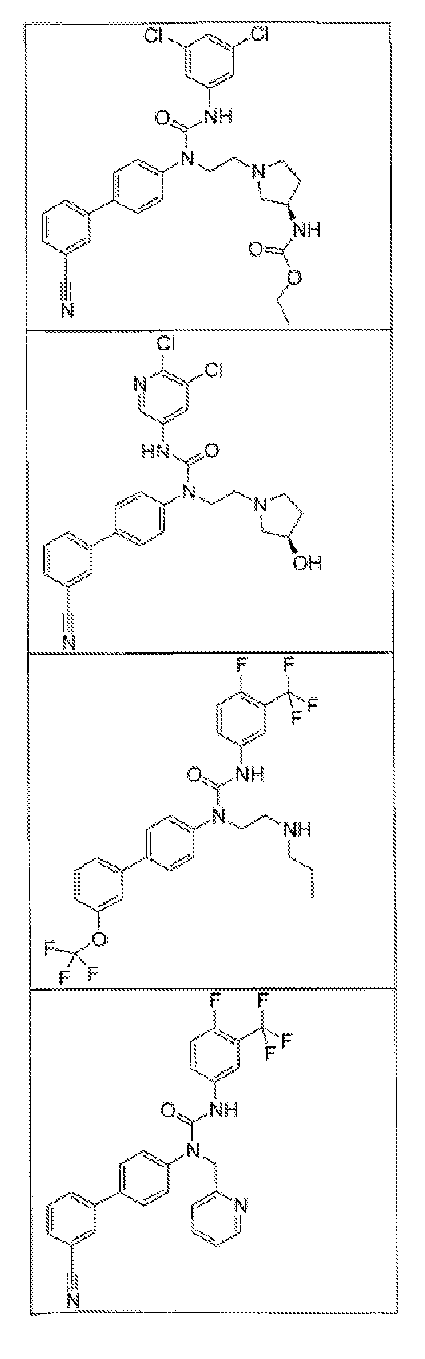

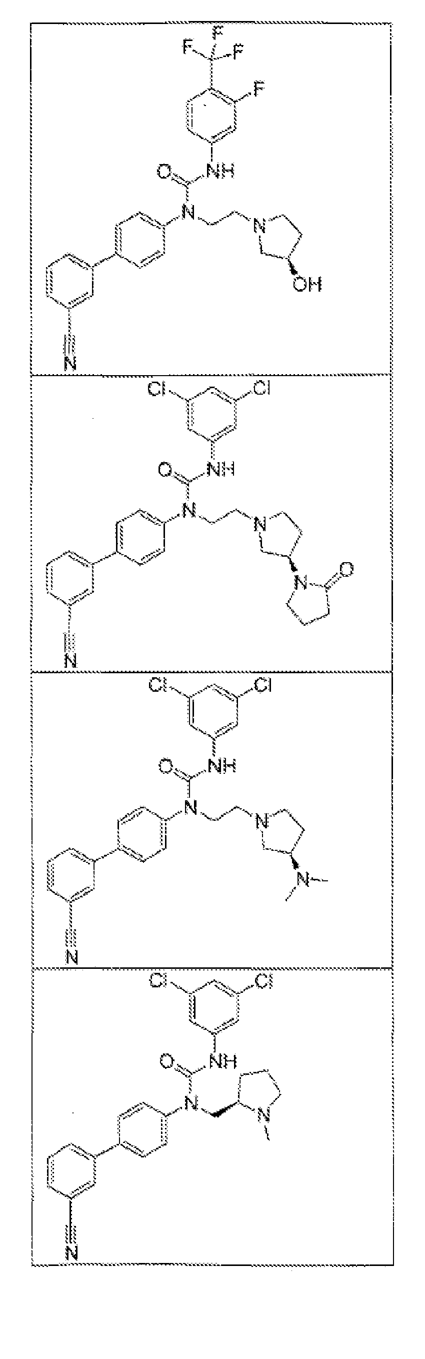

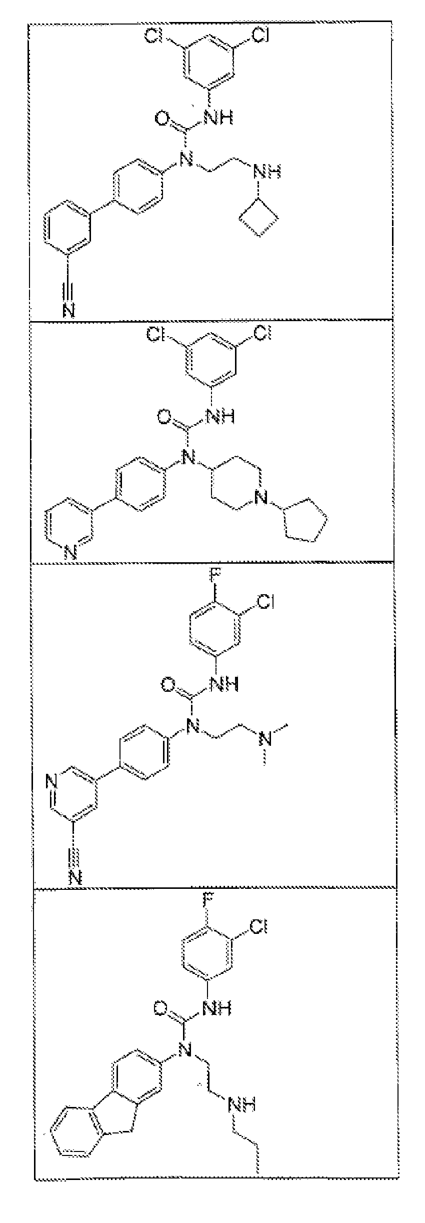

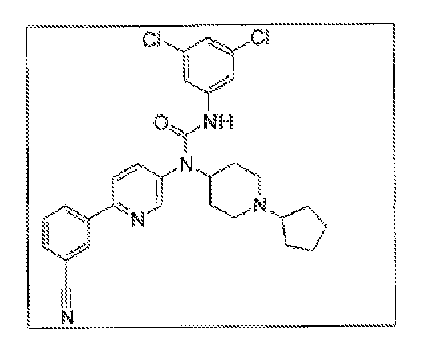

12. Pharmazeutische Zusammensetzung umfassend eine therapeutisch wirksame Menge einer Verbindung gemäss Anspruch 1 in Kombination mit einem pharmazeutisch verträglichen Träger.

13. Verwendung einer Verbindung gemäss Anspruch 1 für die Herstellung eines Medikamentes für die Behandlung einer metabolischen Störung, einer Essstörung oder Diabetes.

14. Pharmazeutische Zusammensetzung, die eine wirksame Menge einer Verbindung wie in Anspruch 1 definiert und einen pharmazeutisch verträglichen Träger davon umfasst.

15. Verwendung einer Verbindung gemäss Anspruch 1 oder einer Prodrug davon oder eines pharmazeutisch verträglichen Salzes der besagten Verbindung oder der besagten Prodrug für die Herstellung eines Medikamentes für die Behandlung einer Essstörung.

16. Verwendung gemäss Anspruch 15, wobei besagte Essstörung Hyperphagie ist.

17. Verwendung gemäss Anspruch 13, wobei besagte metabolische Störung Adipositas ist.

18. Verwendung einer Verbindung gemäss Anspruch 1 oder einer Prodrug davon oder eines pharmazeutisch verträglichen Salzes der besagten Verbindung oder der besagten Prodrug für die Herstellung eines Medikamentes für die Behandlung einer Störung, die Adipositas zugeordnet ist.

19. Verwendung gemäss Anspruch 18, wobei besagte Störung, die Adipositas zugeordnet ist, Typ II Diabetes, Insulinresistenz, Hyperlipidemie oder Bluthochdruck ist.

20. Pharmazeutische Zusammensetzung, die eine therapeutisch wirksame Menge einer Zusammensetzung umfasst, umfassend
eine erste Verbindung, wobei die besagte erste Verbindung eine Verbindung gemäss Anspruch 1 ist, ein Prodrug davon, oder ein pharmazeutisch verträgliches Salz der besagten Verbindung oder der besagten Prodrug;
eine zweite Verbindung, wobei die besagte zweite Verbindung ein Antiadipositas- und/oder Anorexiemittel wie ein β₃ Agonist, ein thryomimetisches Agens, ein Anorexiemittel oder ein NPY Antagonist ist; und ein pharmazeutisch verträglicher Träger davon.

21. Verwendung einer ersten Verbindung, wobei die besagte erste Verbindung eine Verbindung gemäss Anspruch 1, ein Prodrug davon, oder ein pharmazeutisch verträgliches Salz der besagten Verbindung oder der besagten Prodrug ist; für die Herstellung eines Medikamentes für die Behandlung einer Essstörung, wobei die Behandlung die Verabreichung des Medikamentes umfasst, und einer zweiten Verbindung, wobei die besagte zweite Verbindung ein Antiadipositas- und/oder Anorexiemittel ist wie ein β₃ Agonist, ein thryomimetisches Agens, ein Anorexiemittel oder ein NPY Antagonist.

22. Pharmazeutische Zusammensetzung , die eine therapeutisch wirksame Menge einer Zusammensetzung umfasst, umfassend
eine erste Verbindung, wobei die besagte erste Verbindung eine Verbindung gemäss Anspruch 1, ein Prodrug davon, oder ein pharmazeutisch verträgliches Salz der besagten Verbindung oder der besagten Prodrug ist;
eine zweite Verbindung, wobei die besagte zweite Verbindung ein Aldose Reduktase-Inhibitor, ein Glykogenphosphorylase-Inhibitor, ein Sorbitoldehydrogenase--Inhibitor, ein Protein Tyrosin Phosphatase 1B Inhibitor, ein Dipeptidylprotease-Inhibitor, Insulin (einschliessend oral bioverfügbare Insulinpräparate), Insulinmimetika, Metformin, Acarbose, ein PPAR-gamma Ligand wie Troglitazon, Rosaglitazon, Pioglitazon, oder GW-1929, ein Sulfonylharnstoff, Glipazid, Glyburid, oder Chlorpropamid; und ein pharmazeutisch verträglicher Träger dafür.

23. Pharmazeutische Zusammensetzung hergestellt durch Kombinieren der Verbindung wie in Anspruch definiert 1 und von einem pharmazeutisch verträglichen Träger dafür.

24. Verfahren zur Herstellung einer pharmazeutischen Zusammensetzung, umfassend das Kombinieren einer Verbindung wie in Anspruch definiert 1 und eines pharmazeutisch verträglichen Trägers.

25. Verbindung wie in einem der Ansprüche 1 bis 11 definiert für die Verwendung als Medikament.

## Revendications

1. Un composé présentant la formule : 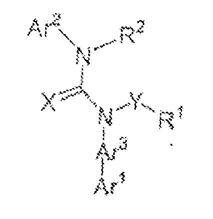 ou un hydrate ou un sel d'addition, acceptable du point de vue pharmaceutique, en dérivant, ou lorsque cela peut s'appliquer, un isomère géométrique ou optique ou un mélange racémique en dérivant, formule dans laquelle
Ar¹ est un radical aryl ou hétéroaryl,
Ar² est un radical aryl, hétéroaryl ou aralkyl,
Ar³ est un radical arylène ou hétéroarylène,
Ar¹ et Ar³ forment ensemble un groupe fluorène, fluorène substitué ou fluorénone, à la condition que Ar³ soit un radical arylène
ou
les radicaux Ar¹, Ar² et Ar³ comportent de 0 à 3 substituants choisis, indépendamment les uns des autres, dans le groupe des radicaux suivants : -(C₁-C₆)-alkyl, -(C₃-C₇)-cycloalkyl, halo, -CN, -(C₁-C₈)-alkoxy, -CF₃, -OCF₃, -CONH₂, - CONH-[(C₁-C₆)-alkyl], -CON(C₁-C₆)-alkyl-(C₁-C₆)-alkyl, NH₂, NHC(O)(C₁-C₆)-alkyl, -NHSO₂[C₁-C₆]-alkyl, S(C₁-C₆)-alkyl, -SO(C₁-C₆)-alkyl, -SO₂(C₁-C₆)-alkyl, méthylènedioxy et NO₂ ;
X est O, S ou N-CN ;
Y est une simple liaison ou un radical -(C₁-C₄)-alkylène ;
R¹ est un groupe thiazole, aryl ou hétéroaryl ou un groupe 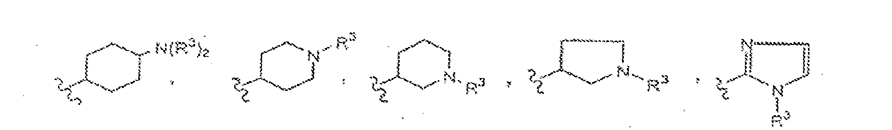 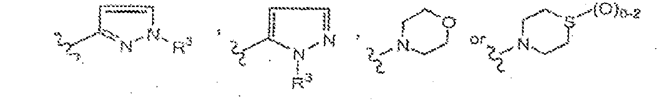 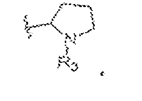 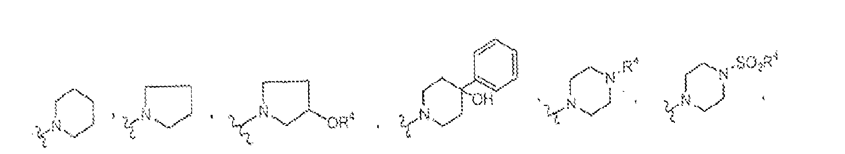 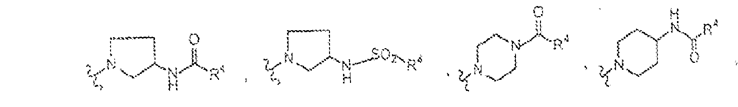 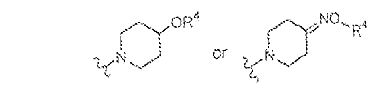 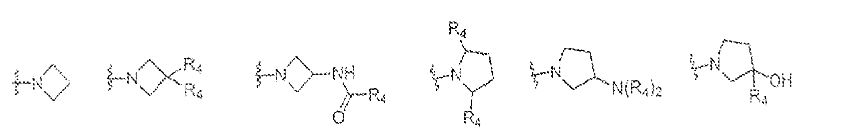 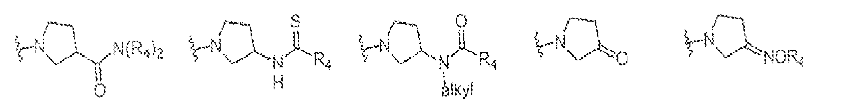 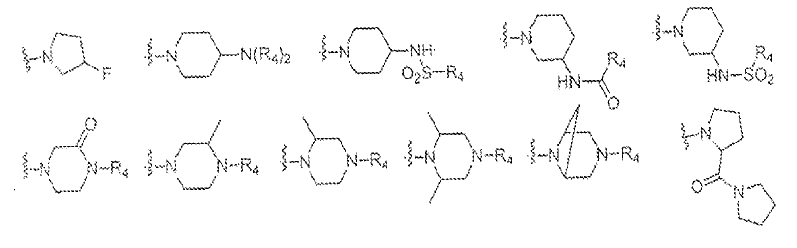
R¹ est un groupe -N(R⁵)₂, -NHC(O)(C₂-C₃)-alkylène-N(R⁵)₂, - C(O)NH(C₂-C₃)-alkylène-N(R⁵)₂, C(O)N(Me)(C₂-C₃)-alkylène-N(R⁵)₂, - C(OH)(C₁-C₂)-alkylèneN(R⁵)₂, -N(Me)(C₂-C₃)-alkylène-N(R⁵)₂, -NH(C₂-C₃)-akylène-C(O)R⁵, -N(Me)(C₂-C₃)-akylène-N(Me)SO₂(R⁵) ou -N(Me)(C₂-C₃)-alkylène-C(O)N(R⁵)₂,
R² est un atome d'hydrogène ou un radical -(C₁-C₆)-alkyl.
R³ est, indépendamment, un atome d'hydrogène ou un radical, non substitué ou halosubstitué, -(C₁-C₆)-alkyl, -(C₃-C₇)-cycloalkyl, -(C₃-C₇)-cycloalkyl-(C₁-C₆)-alkyl, -(C₁-C₆)-alkoxy, -(C₁-C₆)-alkoxy (C₁-Ce)-alkylène, aryl, -aralkyl ou -hétéroaralkyl, ou
R⁴ est un atome d'hydrogène non substitué ou halosubstitué, un radical -(C₁-C₆)-alkyl, -NH(C₁-C₆)-alkyl, -NHaryl, aryl, ou alkoxy ou alkyl à substituant hydroxy, et,
R⁵ est, indépendamment, un atome d'hydrogène ou un radical, non substitué
ou halosubstitué, -(C₁-C₆)-alkyl, -(C₃-C₇)-cycloalkyl, -(C₃-C₇ )-cycloalkyl(C₁-C₆)-alkyl, aryl, -aralkyl, -hétéroaralkyl, -(C₁-C₆)-alkoxy ou (C₁-C₆)-alkylène(C_{1,}-C₆)-alkoxy.

2. Un composé tel que défini dans la revendication 1 ; ou un hydrate ou un sel d'addition, acceptable du point de vue pharmaceutique, en dérivant, ou, lorsque cela peut s'appliquer, un isomère géométrique ou optique ou un mélange racémique en dérivant, dans la formule duquel :
Ar¹ et Ar² are indépendamment phényl ou pyridyl,
Ar³ est 1, 4-arylène
- R¹ est 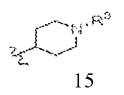 où R³ est un radical -(C₁-C₆)-alkyl, -(C₃-C₇)- cyclo-alkylméthyl, (C₁-C₆)-alkoxy- ou (C₁-C₆)-alkoxy-(C,-C₆)-alkylène-,
- R² est un atome d'hydrogène
- X est O; et
- Y est une simple liaison ou un radical -(C₁-C₃)-alkylène.

3. Un composé tel que défini dans la revendication 1 ou un hydrate ou un sel d'addition, acceptable du point de vue pharmaceutique, en dérivant, ou lorsque cela peut s'appliquer, un isomère géométrique ou optique ou un mélange racémique en dérivant, dans la formule duquel :
Ar¹ et Ar² sont, indépendamment, des radicaux phényl ou pyridyl.
Ar³ est un radical 1,4-arylène,
R¹ est un groupe -N(R⁵)₂ ou -C(O)NH(C₂-C₃)-alkylène-N(R⁵)₂ dans lequel chacun des substituants R⁵ est, indépendamment l'un de l'autre, un atome d'hydrogène ou un radical -(C₁-C₆)-alkyl, -aryl-(C₁-C₆)-alkyl, hétéroaryl, hétéroarylalkyl, -(C₁-C₆)-alkyl halosubstitué, -(C₃-C₇)-cycloalkyl,
X est O ; et
Y est un radical-(C₂-C₃)-alkylène.

4. Un composé tel que défini dans la revendication 1 ou un hydrate ou un sel d'addition, acceptable du point de vue pharmaceutique, en dérivant, ou lorsque cela peut s'appliquer, un isomère géométrique ou optique ou un mélange racémique en dérivant, dans la formule duquel :
Ar¹ et Ar² sont, indépendamment l'un de l'autre, des radicaux phényl ou pyridyl.
Ar³ est un radical 1,4-arylène,
R¹ est choisi parmi 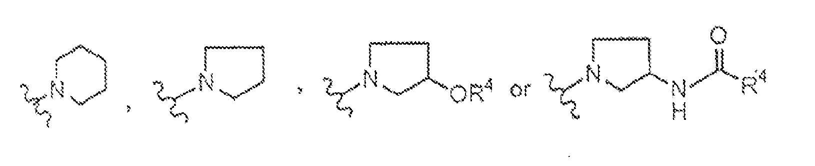
- X est O; et
- Y est un radical -(C₂-C₃) alkylène.

5. Un composé tel que défini dans la revendication 2; ou un hydrate ou un sel d'addition, acceptable du point de vue pharmaceutique, en dérivant, ou lorsque cela peut s'appliquer, un isomère géométrique ou optique ou un mélange racémique en dérivant, dans la formule duquel :
Ar¹ est un radical pyridyl ou phényl substitué en position 3,
- Ar² est radical pyridyl ou phényl halo-substitué ou substitué par un groupe CF₃-, et
- R³ est un radical méthyl, éthyl, propyl, -CH₂CH₂CF₃, cyclopentyl, cyclopropylméthyl ou 3-méthoxyéthyl.

6. Un composé tel que défini dans la revendication 5, dans la formule duquel le substituant en position 3 sur le radical phényl ou pyridyl est un groupe - CN, -OCF₃ ou chloro.

7. Un composé tel que défini dans la revendication 3, dans la formule duquel Ar¹ est un radical phényl ou pyridyl substitué en position 3, Ar² est halo-substitué ou est un radical phényl ou pyridyl halosubstitué ou substitué par un un groupe CF₃- et R⁵ est un radical méthyl, éthyl, propyl, -CH₂CH₂CF₃, cyclopentyl, cyclopropylméthyl ou 3-méthoxyéthyl.

8. Un composé tel que défini dans la revendication 7, dans la formule duquel le substituant en position 3 sur le radical phényl ou pyridyl est un groupe -CN, -OCF₃ ou chloro.

9. Un composé tel que défini dans la revendication 4, dans la formule duquel Ar¹ est un radical phényl ou pyridyl substitué en position 3, Ar² est un radical phényl ou pyridyl halo-substitué ou substitué par un un groupe CF₃- et R⁵ est un radical méthyl, éthyl, propyl, -CH₂CH₂CF₃, cyclopentyl, cyclopropylméthyl ou 3-méthoxyéthyl.

10. Un composé tel que défini dans la revendication 9, dans la formule duquel le substituant en position 3 sur le radical phényl ou pyridyl est un groupe -CN, -OCF₃ ou chloro.

11. Un composé tel que défini dans la revendication 1, choisi dans le groupe comprenant :

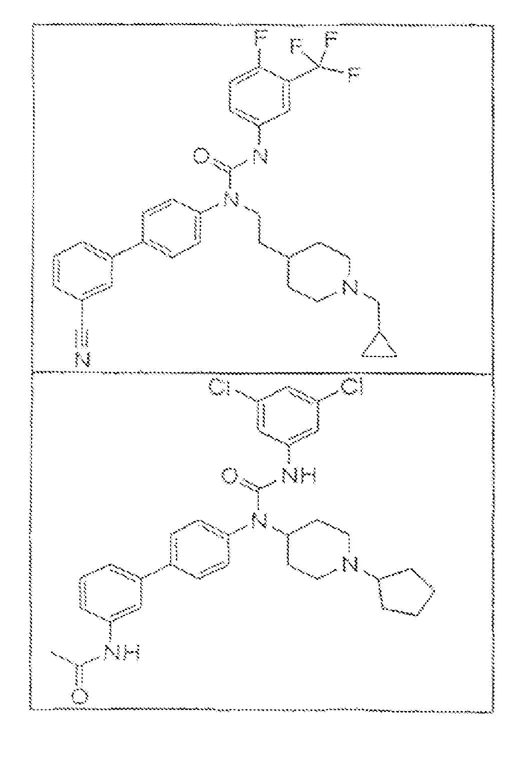

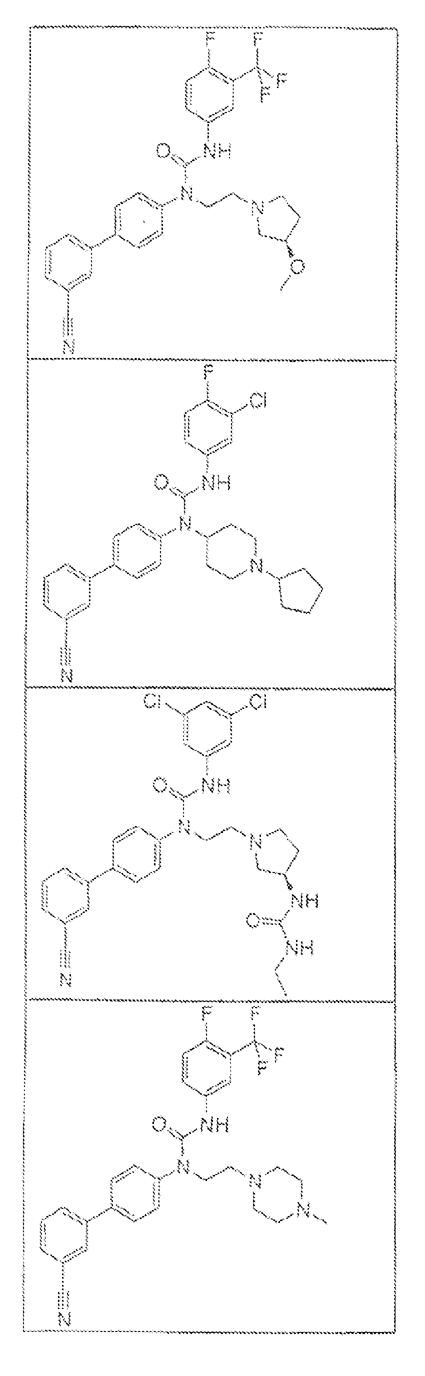

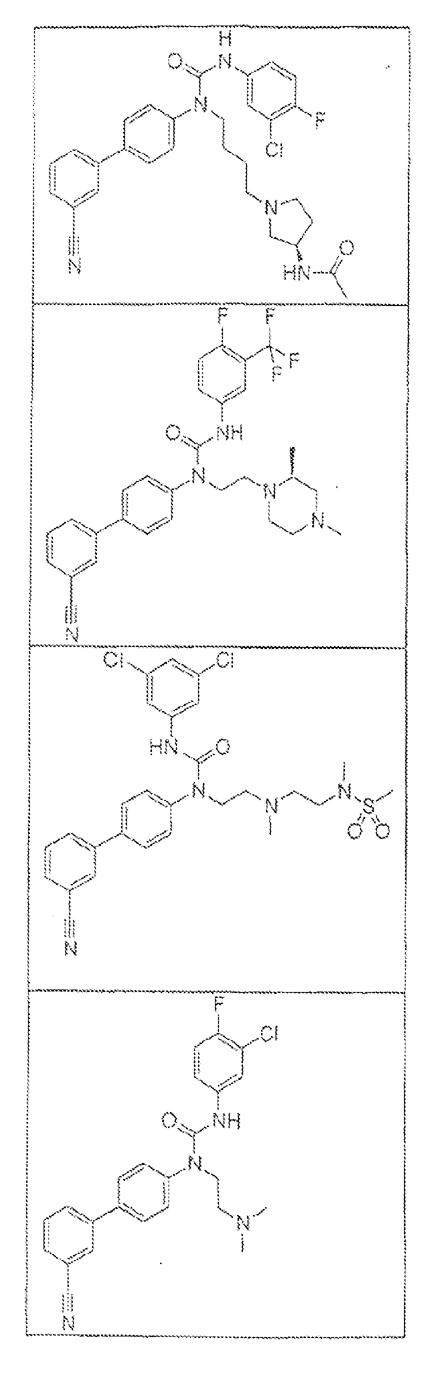

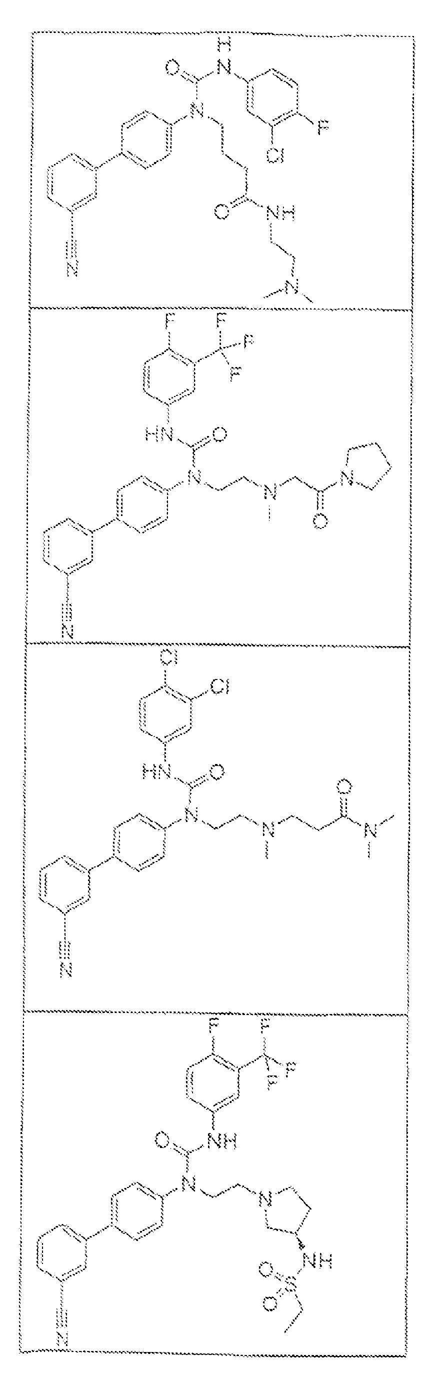

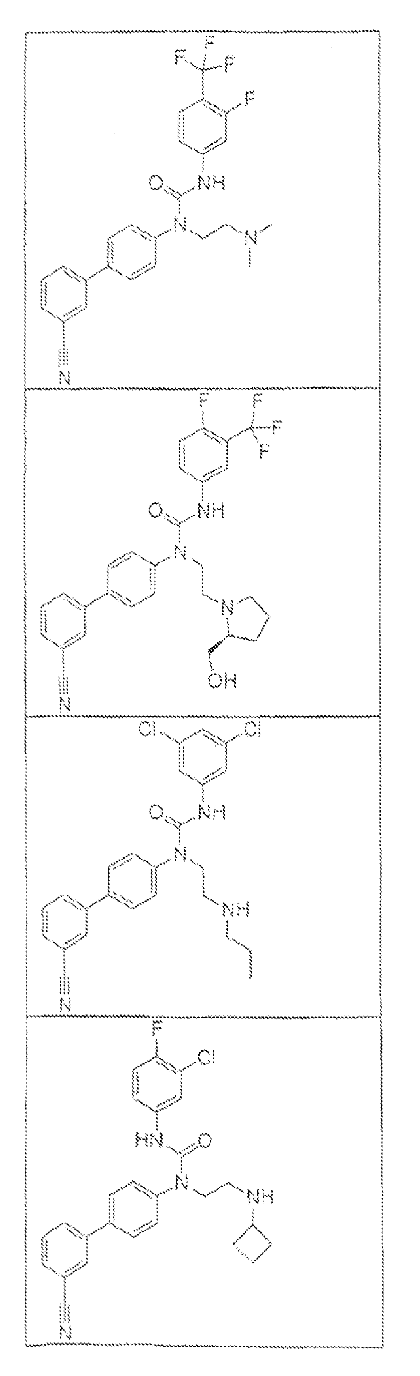

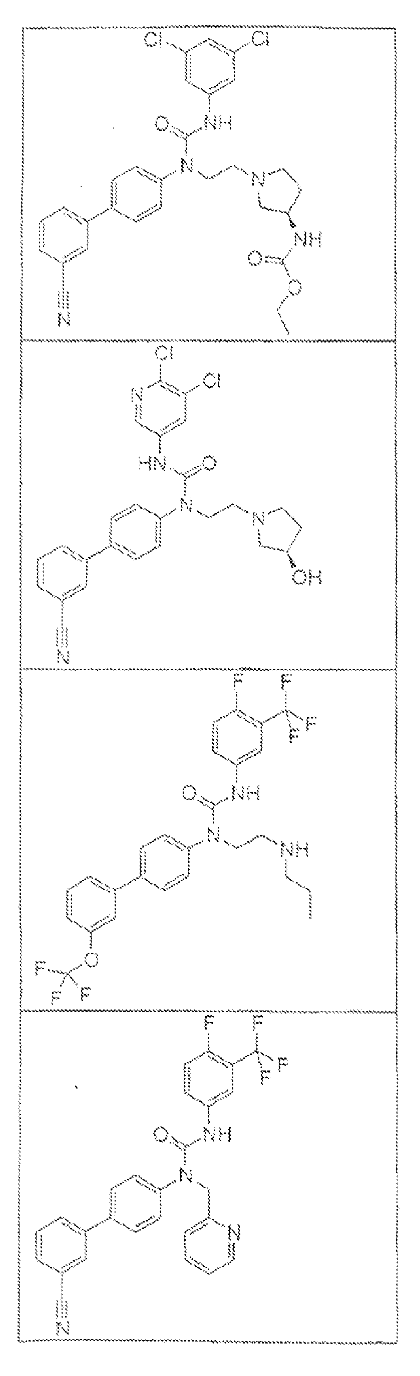

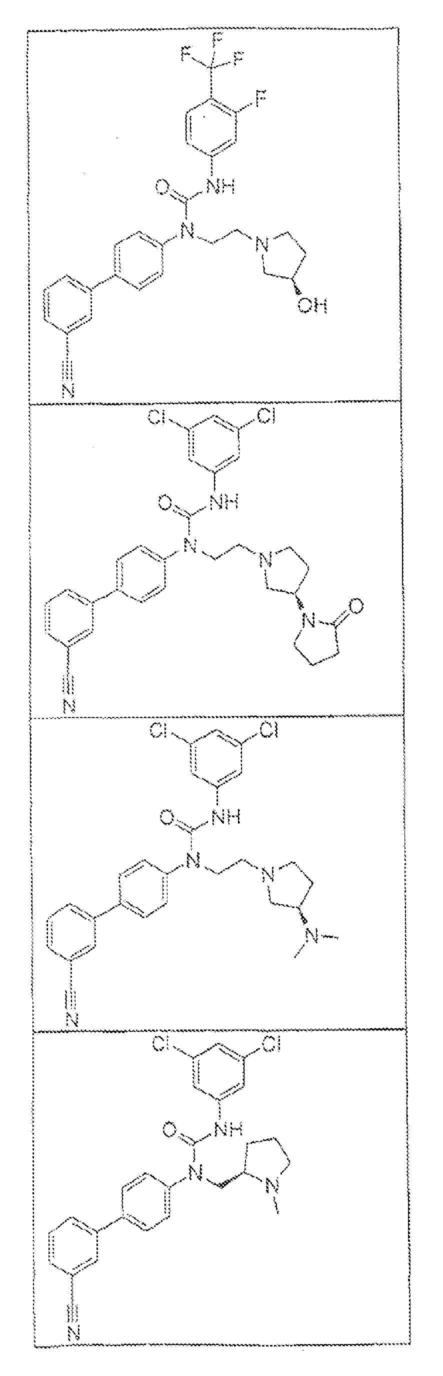

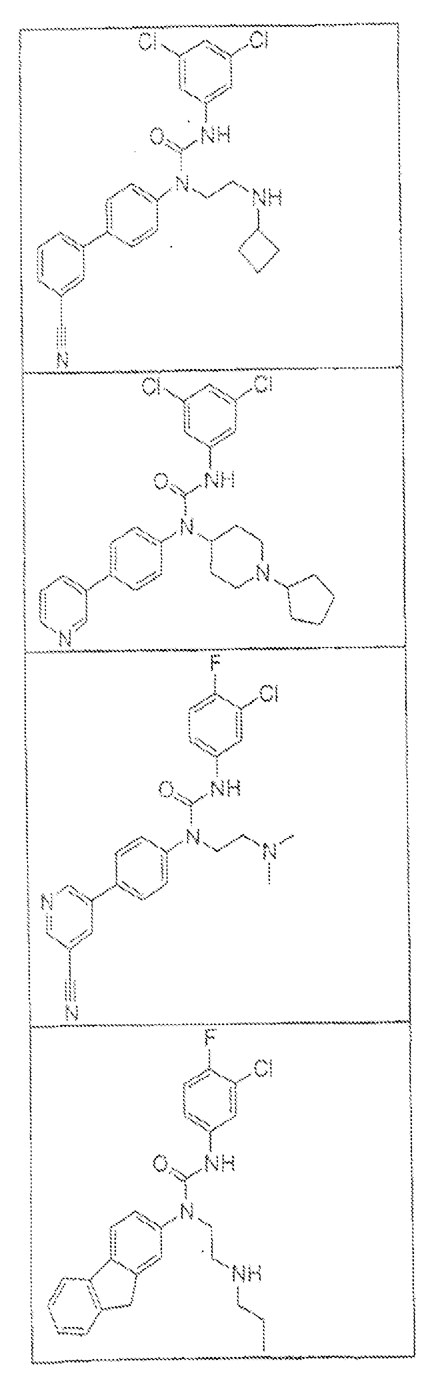

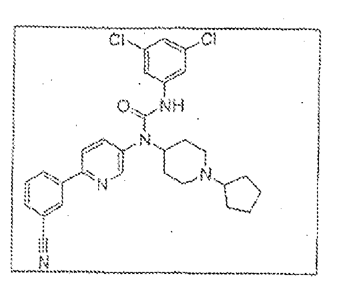

12. Une composition pharmaceutique comprenant une quantité efficace du point de vue thérapeutique d'un composé selon la revendication 1 en association avec un support acceptable du point de vue pharmaceutique.

13. Utilisation d'un composé selon la revendication 1, pour la fabrication d'un médicament pour traiter les désordres métaboliques, les désordres alimentaires ou les diabètes.

14. Une composition pharmaceutique qui comprend une quantité efficace d'un composé tel que défini dans la revendication 1 et un support acceptable du point de vue pharmaceutique.

15. Utilisation d'un composé selon la revendication 1, ou d'un de ses pro-médicaments ou un sel acceptable du point de vue pharmaceutique dudit composé ou dudit pro-médicaments pour la fabrication d'un médicament pour traiter les désordres alimentaires.

16. Utilisation selon la revendication 15, dans laquelle ledit désordre alimentaire est l'hyperphagie.

17. Utilisation selon la revendication 13, dans laquelle le désordre métabolique est l'obésité.

18. Utilisation d'un composé selon la revendication 1 ou d'un de ses pro-médicaments ou d'un sel acceptable du point de vue pharmaceutique dudit composé ou de ses pro-médicaments, pour la fabrication d'un médicament pour traiter un désordre associé à l'obésité.

19. Utilisation selon la revendication 18 dans laquelle ledit désordre associé à l'obésité est un diabète de type II, la résistance à l'insuline, l'hyper-lipidémie ou l'hypertension.

20. Une composition pharmaceutique qui comprend une quantité efficace du point de vue thérapeutique d'une composition comprenant :
- un premier composé, ce premier composé étant un composé selon la revendication 1, un de ses pro-médicaments ou un sel acceptable du point de vue pharmaceutique dudit composé ou desdits pro-médicaments;
- un second composé, ledit second composé étant un agent antiobésité et/ou anorectique, tel que un β₃ agoniste, un agent thryomimétique, un agent anorectique
ou un antagoniste NPY ; et
- un support acceptable du point de vue pharmaceutique.

21. Utilisation d'un premier composé, ce premier composé étant un composé selon la revendication 1, un de ses pro-médicaments, ou un sel acceptable du point de vue pharmaceutique dudit composé desdits pro-médicaments pour la fabrication d'un médicament pour traiter un désordre alimentaire dans laquelle le traitement comprend l'administration du médicament; et d'un second composé, ledit second composé étant un agent antiobésité et/ou anorectique, tel que un β₃ agoniste, un agent thryomimétique, un agent anorectique ou un antagoniste NPY.

22. Une composition pharmaceutique qui comprend une quantité efficace du point de vue thérapeutique d'une composition comprenant :
- un premier composé, ce premier composé étant un composé selon la revendication 1, un de ses pro-médicaments ou un sel acceptable du point de vue pharmaceutique dudit composé ou desdits pro-médicaments;
- un second composé, ce second composé étant un inhibiteur d'aldose réductase, un inhibiteur de glycogène phosphorylase, un inhibiteur de sorbitol déhydrogénase, un inhibiteur de protéine de tyrosine phosphatase 1B, un inhibiteur de dipeptidyl protéase, l'insuline (incluant une préparation d'insuline biodisponible oralement), une mimétique insuline, la metformine, l'acarbose, un ligand PPAR-gamma tel que troglitazone, rosaglitazone, pioglitazone ou GW-1929, une sulfonylurée, la glypazide, la glyburide, ou la chlorpropamide; et un support acceptable du point de vue pharmaceutique.

23. Une composition pharmaceutique obtenue par combinaison du composé tel que défini dans la revendication 1 et d'un support acceptable du point de vue pharmaceutique.

24. Un procédé de fabrication d'une composition pharmaceutique comprenant la combinaison du composé tel que défini dans la revendication 1 et d'un support acceptable du point de vue pharmaceutique.

25. Un composé tel que défini dans une quelconque des revendications 1 à 11 destiné à être utilisé comme médicament.
